(19) Europäisches Patentamt
European Patent Office
Office européen des brevets

(11) **EP 2 462 222 B1**

(12) **EUROPEAN PATENT SPECIFICATION**

(45) Date of publication and mention
of the grant of the patent:
**24.05.2017 Bulletin 2017/21**

(21) Application number: **10740816.3**

(22) Date of filing: **03.08.2010**

(51) Int Cl.:
**C12N 5/00** *(2006.01)* **C12Q 1/68** *(2006.01)*

(86) International application number:
**PCT/US2010/044194**

(87) International publication number:
**WO 2011/017288 (10.02.2011 Gazette 2011/06)**

(54) **IMPROVED PLANTS, MICROBES, AND ORGANISMS**

VERBESSERTE PFLANZEN, MIKROBEN UND ORGANISMEN

PLANTES, MICROBES ET ORGANISMES ENRICHIS

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **05.08.2009 US 231604 P**

(43) Date of publication of application:
**13.06.2012 Bulletin 2012/24**

(73) Proprietor: **Chromocell Corporation
North Brunswick, NJ 08902 (US)**

(72) Inventor: **SHEKDAR, Kambiz
New York,
NY 10010 (US)**

(74) Representative: **O'Connell, Maura et al
FRKelly
27 Clyde Road
Dublin D04 F838 (IE)**

(56) References cited:
**WO-A1-2008/066909    WO-A2-2005/079462**

- **LI YONGSHENG ET AL: "Molecular beacons: An optimal multifunctional biological probe" BIOCHEMICAL AND BIOPHYSICAL RESEARCH COMMUNICATIONS, vol. 373, no. 4, September 2008 (2008-09), pages 457-461, XP002600712 ISSN: 0006-291X**
- **CHRISTENSEN N M ET AL: "Advances in imaging RNA in plants" TRENDS IN PLANT SCIENCE, ELSEVIER SCIENCE, OXFORD, GB, vol. 15, no. 4, 1 April 2010 (2010-04-01), pages 196-203, XP026997587 ISSN: 1360-1385 [retrieved on 2010-02-10]**
- **KARRER E E ET AL: "IN SITU ISOLATION OF MRNA FROM INDIVIDUAL PLANT CELLS: CREATION OF CELL-SPECIFIC CDNA LIBRARIES" PROCEEDINGS OF THE NATIONAL ACADEMY OF SCIENCES OF THE UNITED STATES (PNAS), NATIONAL ACADEMY OF SCIENCE, US LNKD-DOI:10.1073/PNAS.92.9.3814, vol. 92, 1 April 1995 (1995-04-01), pages 3814-3818, XP002912944 ISSN: 0027-8424**
- **ZHANG LU ET AL: "Enhanced fluorescence imaging in chlorophyll-suppressed tobacco tissues using virus-induced gene silencing of the phytoene desaturase gene", BIOTECHNIQUES, vol. 48, no. 2, February 2010 (2010-02), pages 125-129, ISSN: 0736-6205(print)**

**Description**

**1. INTRODUCTION**

The present disclosure relates to methods for identification, isolation, and enrichment of plant cells, plants, microbial cells, and organisms comprising desired genetic profiles and to plant cells, plants, microbial cells, and organisms resulting from these methods. In certain aspects, organisms obtained by the methods disclosed herein are not genetically engineered organisms.

**2. BACKGROUND**

Organisms including plants, animals, and microbes share many conserved genes, yet have wide diversity due to genetic variability and evolution. The genome of organisms are able to mutate and to produce alterations in cellular structures and functions that are selected for as the organisms adapt to changing environments. As a result, genes that are desirable or confer a survival advantage are passed on to later generations and are selected for, while others are not passed on to later generations, or remain intact but are not selected for. However, the process of adaptation during evolution is a slow process. There are tremendous benefits, for industries that use plants, animals, or microbes as raw materials, in being able identify, isolate, and enrich certain strains or variants of organisms possessing improved characteristics that exist in the natural heterogeneous population of organisms.

2.1 **Plants**

The recent increase in food prices demonstrates the limitations of traditional agriculture. There is a need for improved plants that can be grown under sub-optimal conditions, such as too little water, too much water, too high salinity in the soil, presence of plant diseases and pests *et al.* Traditionally, adapted plants were selected for over long periods of time. Recent advance of genetic engineering allowed more rapid generation of adapted plants but brought with it a concern about the ecological impact of plants whose genomes were modified by introduction of foreign recombinant DNA into their genomes.

Modulation of certain stages or steps of plant development and/or growth can be beneficial for generating more desirable plants. Plant development is partially dependent on the plant's response to a variety of environmental signals. For example, photosynthesis is a process comprising a series of complex reactions that uses light energy to generate carbohydrates from $CO_2$, usually with the consumption of $H_2O$ and evolution of $O_2$ (see, *e.g.,* Lodish et al., Molecular Cell Biology, W.H. Freeman and Company, New York, NY, 2000). Also, the development of root systems is, in part, a response to the availability and distribution of moisture and nutrients within the soil.

Nitrate is a key required nutrient for the synthesis of amino acids, nucleotides and vitamins and is commonly considered to be the most limiting for normal plant growth *(*Vitousek et al., 2004, Biogeochemistry 13). Plants are keenly sensitive to nitrogen levels in the soil and, atypically of animal development, adopt their body plan to cope with their environment *(*Lopez-Bucio et al., 2003, Curr Opin Plant Biol 6, 280-7); Malamy et al., 2005, Plant Cell Environ 28, 67-77); Walch-Liu et al., 2006, Ann Bot (Lond) 97, 875-81).

2.2 **Microorganisms**

Microorganisms, such as bacteria, fungi, archae, protozoa, and algae and others, have an important role in industry and food production. Similarly to plants, microorganisms can adapt to the conditions under which they are used in industrial and food production processes. While traditional methods had to rely on long selection processes, genetic engineering by introduction of foreign genetic DNA into the genomes of the microorganisms raises ecological concerns.

**3. SUMMARY**

Provided herein are methods to expedite the selection of plants, animals, and microorganisms that possess one or more desired traits, *e.g.,* a trait that is optimized for particular environmental conditions. The present disclosure provides methods to improve organisms including plants, microorganisms, and animals, by identifying and selecting plants, plant cells, eukaryotic cells, animals, microbial cells, or microorganisms with gene expression profiles that correlated with a desirable trait or phenotype, for instance improved growth properties and/or tolerance to adverse environmental conditions and/or enrichment or reduction of certain biological products or metabolites produced. The present methods may be applied to these organisms to identify and isolate cells comprising desired gene expression profiles for production of improved strains or variants without genetic engineering. The present methods circumvent the need to introduce genetic elements into the organism thereby minimizing the risk of integration events with unintended risks and consequences for human health or to the environment that may be caused by genetically modified organisms (GMOs).

The present methods take advantage of the naturally occurring high degree of genetic diversity that exists in organisms, and efficiently identify, select, and enrich for plants, plant cells, eukaryotic cells, animals, microbial cells, or microorganisms possessing desired gene expression profiles conferring improved properties. The present methods can identify, select, and enrich for cells or organisms with improved properties from a pool of genetically diverse cells or organisms, respectively, faster and more efficiently than conventional methods. In particular aspects, the cells or organisms have not been genetically modified. In particular aspects, the present methods allow for the generation of novel homogeneous populations of cells or organisms possessing improved properties.

In specific aspects of the invention defined by the claims appended to this description, the present methods allow for identification, selection and enrichment of agricultural staples, which include, but are not limited to, wheat, tobacco, tea, coffee, cocoa, corn, soybean, sugar cane, and rice. In particular embodiments, the methods described herein allow for identification, selection and enrichment of crops that are resistant to or that have improved growth in conditions of drought, flood, cold, hot, low or lack of light, extended periods of darkness, nutrient deprivation, or poor soil quality including sandy, rocky acidic or basic soil. In particular embodiments, the methods described herein allow for identification, selection and/or enrichment of crops that grow faster, crops that generate more seed, crops that are resistant to pests and microorganisms, or crops with improved taste or consistency for use as foods.

In certain aspects of the present invention defined by the claims appended to this description, crops used as a source of biologics or metabolites could be identified, selected, and/or optimized for yield, cost of goods, or proportion or purification of desired metabolite versus contaminant. For example, the present methods may identify, isolate, and/or enrich crops with increased production of biologics for use as therapeutics, crops with increased sugar yield as an energy source, crops with increased yield of desired or health-promoting components, or crops (*e.g.*, tobacco) with decreased levels of undesired components. In certain embodiments, a protoplast has been genetically modified (*e.g.*, recombinantly expresses one or more transgene).

Though not part of the claimed invention, the present methods also can identify, isolate, and/or enrich for improved microbes, *e.g.*, microbes used in foods or used to produce metabolites (*e.g.*, antibiotics) or to catalyze reactions (including enzymes used in commercial applications). Such improve microbes may possess increased yield of the desired product (functional foods, antibiotics), faster growth, optimal temperature tolerance and adoption to simplified growth conditions, improved properties for use in fermentation (*e.g.*, cheese, yogurt, bread, beer...), or improved catalysis or production of components (enzymes) for catalysis of commercial applications. In certain aspects, a microbial cell or microorganism has been genetically modified (*e.g.*, recombinantly expresses one or more transgene).

The present methods allow for the identification, isolation, and/or enrichment of plants, plant cells, eukaryotic cells, animals, microbial cells, or microorganisms comprising desired genetic profiles. In certain embodiments, such methods do not involve the introduction of external genetic material into the cell or organism. The method relies on the use of fluorogenic oligonucleotide probes targeted to specific genetic sequences (*i.e.,* nucleic acid sequences) of interest to detect the gene expression profile of millions of individual cells (see, *e.g.*, International Application No. PCT/US2005/005080 published as WO 2005/079462). The fluorogenic oligonucleotide probes undergo a conformational fluorogenic change upon binding to target sequences present in cells, resulting in a fluorescent signal that is correlated with the level of expression, which can be increased or decreased. In specific embodiments, fluorogenic oligonucleotide probes comprise a fluorophore and a quencher positioned in such a way that the fluorescent signal from the fluorophore is quenched when the fluorogenic oligonucleotide probes adopt a certain confirmation in the absences of target sequences; in the presence of target sequences the fluorogenic oligonucleotide probes adopt a different confirmation that does not allow the quencher to quench the fluorescent signal of the fluorophore or that reduces the degree to which the fluorescent signal of the fluorophore is quenched. Cell sorting methods are then used to isolate the cells for growth and propagation. For more detail on the use of fluorogenic oligonucleotide probes to detect nucleic acid molecules such as transcripts, see, *e.g.,* U.S. Patent No. 6,692,965 by Shekdar et al. issued February 17, 2004, and International Application No. PCT/US2005/005080 published as WO 2005/079462. Multiple differentially labeled fluorogenic oligonucleotide probes may be used to simultaneously assess the expression levels (*e.g.*, transcript expression levels) of multiple genes of interest in cells. Because the use of these fluorogenic oligonucleotide probes is a traceless process in that the desired cells that are isolated are not engineered and the probes used in the process are degraded, the methods are applicable to obtain improved organisms including crops and microorganisms without the need for genetic engineering. However, fluorogenic oligonucleotide probes may also be used to identify and isolate cells that express high levels of introduced sequences. In certain embodiments, the methods described herein can be combined with traditional genetic engineering of plant cells, eukaryotic cells, or microbial cells.

In certain aspects, the methods described herein comprise selecting naturally occurring cells. In specific embodiments, the methods described herein comprise selecting cells with naturally occurring variants or mutations in one or more genes. In specific embodiments, the methods described herein comprise selecting cells with naturally occurring variants or mutations in promoter regions of genes. In certain other embodiments, the present methods comprise selecting cells that underwent prior treatments. Such prior treatments may be exposure to sunlight or ultraviolet (UV) light, mutagens such as ethyl methane sulfonate (EMS), and chemical agents. In specific embodiments, such prior treatments may include exposure to undesirable growth conditions, *e.g.*, low oxygen or low nutrients conditions, or toxic conditions.

In certain aspects, the gene expression profile of an individual cell may differ from the gene expression profile that results in the whole organism produced from the cell. To accommodate these situations, a plurality of cells is selected with a plurality of different expression profiles, wherein each of the expression profile includes the desired aspects of the expression profile, *i.e.,* up- or downregulation of certain specific gene expressions. The plurality of cells is then used to generate whole organisms. Existing methods such as RT-PCR or assessment of the intended properties can then be used to select those variant organisms which resulted in the intended gene expression or improved desired properties.

The present invention relates to a method for identifying a plant cell expressing an RNA of interest, wherein said method comprises:

(a) introducing into a plant cell a fluorogenic oligonucleotide probe capable of detecting the RNA of interest, wherein the fluorogenic oligonucleotide is transferred into the plant cell by using polyethylene glycol (PEG); and

(b) determining whether the RNA of interest is present in the plant cell, and wherein the plant cell is a protoplast. In some embodiments, the method further comprises the step of quantifying the level of the RNA of interest. The level of the RNA of interest may be compared with the level of the RNA of interest in a reference population, *e.g.*, a population representative of a population that exists naturally. In specific embodiments, the RNA of interest is an mRNA the encodes for a protein or polypeptide of interest. In other embodiments, the RNA of interest is not a coding RNA or is not translated.

The present invention also relates to a method for identifying a plant cell with a desired RNA expression profile, wherein the method comprises:

(a) introducing into a plant cell a plurality of fluorogenic oligonucleotide probes each capable of detecting an RNA of interest, wherein the plurality of fluorogenic oligonucleotides are transferred into the plant cell by using polyethylene glycol (PEG); and

(b) quantifying the RNA levels detected by the plurality of fluorogenic oligonucleotides, and wherein the plant cell is a protoplast. In specific embodiments, such method further comprises the step of comparing the quantified RNA levels of the plant cell with the RNA levels in a reference cell.

In certain embodiments, the methods of the invention described herein further comprise treating the protoplast with UV light, natural light, or a chemical agent, for example, prior to introducing into the protoplasts fluorogenic oligonucleotide probes. In particular embodiments, the plurality of fluorogenic oligonucleotide probes comprises at least 2, 3, 4, 5, 6, 7, 8, 9, 10, 15, 20, 25, 30, 35, 40, 45, 50, 60, 70, 80, 90, 100, 125, 150, 175, 200, 250, 300, 350, 500, 600, 700, 800, 900, or at least 1000 fluorogenic oligonucleotide probes. In specific embodiments, the RNA of interest is encoded by a gene selected from the group consisting of genes listed in Table 2. In some embodiments, the RNA of interest is translated. In other embodiments, the RNA of interest is not translated. In other embodiments, the protoplast has been derived from seedling. In particular embodiments, the seedling has been treated with UV light, natural light, or a chemical agent. In some embodiments, the protoplast is a cell of a plant selected from the group consisting of oak, maple, pine, spruce, tobacco, tea, coffee, cocoa, corn, soybean, sugar cane, seaweed, cactus, palm tree, herb, weeds, grass, and rice. Non-limiting examples of herbs include, basil, oregano, thyme, or rosemary. In specific embodiments, the protoplasts used in the methods described herein do not express a recombinant gene or have not been genetically engineered. In certain embodiments, the protoplasts used in the methods described herein express a recombinant gene or have not been genetically engineered.

The present invention also provides for a method for generating an improved plant, wherein the method comprises:

(a) identifying a plant cell using the methods of Claim 1 or 2 of the claims appended to this description; and

(b) generating an improved plant from the plant cell identified in step (a), wherein the plant cell is a protoplast. In specific embodiments, the improved plant is a clone of a plant cell identified by the methods described herein.

In specific aspects, the present disclosure provides for an isolated plant cell identified by the methods described herein. In specific aspects, the isolated plant cell or improved plant does not express a recombinant gene or has not been genetically engineered. In specific aspects, the isolated plant cell or improved plant expresses a recombinant gene or has been genetically engineered.

In some aspects, the present disclosure relates to a population of plants, wherein each plant is a clone of a cell identified by the methods described herein.

In certain aspects, the present disclosure relates to a product derived from an improved plant wherein the plant is a clone of a plant cell identified by the methods described herein.

In particular aspects, the present disclosure relates to a seed of a plant that is a clone of a plant cell identified by the methods described herein. In some aspects, the seed has covering. In other aspects, the seed does not have covering.

In various aspects, the present disclosure relates a method for identifying a microorganism, microbial cell or a eukaryotic cell expressing at least one RNA of interest, wherein said method comprises:

(a) introducing into a microorganism, microbial cell or a eukaryotic cell a fluorogenic oligonucleotide probe capable of detecting the RNA of interest; and
(b) determining whether the RNA of interest is present in the plant cell. In some aspects, the method further comprises the step of quantifying the level of the RNA of interest.

In other aspects, the present disclosure relates to a method for identifying a microorganism, microbial cell, or a eukaryotic cell with a desired RNA expression profile, wherein the method comprises:

(a) introducing into a microorganism, a microbial cell, or a eukaryotic cell a plurality of fluorogenic oligonucleotide probes each capable of detecting an RNA of interest;
(b) quantifying the RNA levels detected by the plurality of fluorogenic oligonucleotides probes. In specific aspects, such method further comprises the step of comparing the quantified RNA levels of the microorganism, microbial cell, or eukaryotic cell with the RNA levels in a reference microorganism, microbial cell, or eukaryotic cell, respectively.

In certain aspects, methods described herein further comprises treating the microorganism, microbial cell, or a eukaryotic cell with UV light, natural light, or a chemical agent, for example, prior to introducing into the microorganism, microbial cell, or eukaryotic cell fluorogenic oligonucleotide probes. In particular aspects, the plurality of fluorogenic oligonucleotide probes comprises at least 2, 3, 4, 5, 6, 7, 8, 9, 10, 15, 20, 25, 30, 35, 40, 45, 50, 60, 70, 80, 90, 100, 125, 150, 175, 200, 250, 300, 350, 500, 600, 700, 800, 900, or at least 1000 fluorogenic oligonucleotide probes. In some aspects, the RNA of interest is translated. In other aspects, the RNA of interest is not translated. In specific aspects, the RNA of interest is encoded by a gene selected from the group consisting of genes listed in Tables 3, 4, and 5. In specific aspects, the microorganism, microbial cell, or eukaryotic cell expresses one or more recombinant RNA of interest.

In particular aspects, the present disclosure provides for a method for generating a non-human animal, wherein the method comprises:

(a) identifying a non-human eukaryotic cell using the methods described herein; and
(b) generating a non-human animal from the non-human eukaryotic cell identified in step (a). In specific aspects, the step of generating a non-human animal from the non-human eukaryotic cell comprises reprogramming the cell to an induced pluripotent stem (iPS) cell, and generating a non-human animal from the iPS cell. In certain aspects, the present disclosure relates to improved non-human animals generated by such methods. In some aspects, the present disclosure relates to a population of improved non-human animals, wherein each improved non-human animal is a clone of a improved non-human animal generated by the methods described herein. In specific aspects, the non-human eukaryotic cell expresses one or more RNA of interest or a desired gene expression profile.

In certain aspects, the present disclosure relates to a product derived from an improved non-human animal wherein the non-human animal is a clone of a non-human eukaryotic cell identified by the methods described herein.

In particular aspects, the present disclosure provides for a method for generating tissue or an organ of an animal, wherein the method comprises:

(a) identifying a eukaryotic cell using the methods described herein; and
(b) generating tissue or an organ of an animal from the eukaryotic cell identified in step (a). In specific aspects, the eukaryotic cell expresses one or more RNA of interest or a desired gene expression profile. In specific aspects, the eukaryotic cell is a human cell. In other aspects, the eukaryotic cell is a non-human cell. In particular aspects, the tissue or organ generated is human tissue or a human organ. In particular aspects, the tissue or organ generated is non-human tissue or a human organ.

In specific aspects, the present disclosure provides for an isolated microorganism or isolated eukaryotic cell identified by the methods described herein. In particular aspects, microorganisms that express one or more genes of interest may be, but are not limited to, bacteria, protozoa, fungi, or algae. In specific aspects, the eukaryotic cell is a cell of a mouse, rat, monkey, dog, cat, pig, sheep, goat, horse, chicken, donkey, frog, worm, insect (*e.g.,* fly), or cow.

In some aspects, the present disclosure relates to a population of microorganisms, wherein each microorganism is a clone of a microorganism identified by the methods described herein. In some aspects, the present disclosure relates to a population of eukaryotic cells, wherein each eukaryotic cell is a clone of a eukaryotic cell identified by the methods described herein.

In certain aspects, the present disclosure relates to a product derived from tissues or organs generated by the methods described herein.

In certain aspects, the present disclosure relates to a computer-implemented method for identifying a trait associated with a plant or microorganism, wherein the method comprises:

(a) receiving a first RNA expression profile comprising measured amounts of a plurality of different cellular constituents in a first cell of said plant or microorganism;

(b) comparing said first RNA expression profile to a plurality of landmark RNA expression profiles stored in a database to determine a measure of similarity between said first RNA expression profile and each said landmark RNA expression profile in said plurality of landmark RNA expression profiles, wherein each said landmark RNA expression profile comprises measured amounts of a plurality of different cellular constituents in a second cell of a second plant or microorganism that is associated with at least one respective known trait;

(c) determining one or more landmark RNA expression profiles most similar to said first RNA expression profile based on the measures of similarity determined in step (b); and

(d) identifying the at least one respective known trait associated with the second plant or microorganism corresponding to the respective one or more landmark RNA expression profiles determined to be most similar to said first RNA expression profile in step (c) as a trait associated with said plant or microorganism;

wherein steps (a), (b), (c), and (d) are implemented on a suitably programmed computer.

In specific aspects of such method said one or more landmark RNA expression profiles are most similar to said first RNA expression profile if said measures of similarity are above a predetermined threshold.

In certain aspects, the present disclosure relates to computer-implemented method for identifying a trait associated with a plant or microorganism, wherein the method comprises:

(a) receiving a first RNA expression profile comprising measured amounts of a plurality of different cellular constituents in a first cell of said plant or microorganism;

(b) clustering a plurality of RNA expression profiles, which plurality comprises said first RNA expression profile and a plurality of landmark RNA expression profiles, wherein each said landmark RNA expression profile comprises measured amounts of a plurality of different cellular constituents in a second cell of a second plant or microorganism that is associated with at least one respective known trait;

(c) identifying one or more landmark RNA expression profiles in said plurality of landmark RNA expression profiles that cluster with the first RNA expression profile; and

(d) characterizing said plant or organism as being associated with said at least one respective known trait associated with the second plant or microorganism corresponding to the respective one or more landmark RNA expression profiles identified as clustered with said first RNA expression profile in step (c);

wherein steps (a), (b), (c), and (d) are implemented on a suitably programmed computer.

In certain aspects, the present disclosure relates to a computer-implemented method of classifying a plant or microorganism as having a trait using a classifier, wherein the method comprises:

(a) processing, using said classifier, a first RNA expression profile comprising measured amounts of a plurality of different cellular constituents in a first cell of said plant or microorganism, to classify said plant or microorganism as to a known trait, wherein said classifier is trained according to a method comprising:

training said classifier for classifying a plant or microorganism as to a known trait using a plurality of landmark RNA expression profiles stored in a database, wherein each said landmark RNA expression profile comprises measured amounts of a plurality of different cellular constituents in a second cell of a second plant or microorganism that is associated with at least one respective known trait;

wherein step (a) is implemented on a suitably programmed computer.

In certain aspects, the present disclosure relates to a computer-implemented method of classifying a plant or microorganism as having a trait using a classifier, wherein the method comprises:

(a) training a classifier for classifying a plant or microorganism as to a known trait using a plurality of landmark RNA expression profiles stored in a database, wherein each said landmark RNA expression profile comprises measured amounts of a plurality of different cellular constituents in a second cell of a second plant or microorganism that is associated with at least one respective known trait; and

(b) processing, using said classifier, a first RNA expression profile comprising measured amounts of a plurality of different cellular constituents in a first cell of said plant or microorganism, to classify said plant or microorganism as to a known trait;

wherein steps (a) and (b) are implemented on a suitably programmed computer.

In particular aspects, the present disclosure provides for high throughput screens to identify organisms (*e.g.,* plants, animals, and microorganisms) and cells (*e.g.,* plant cells, eukaryotic cells, and microbial cells) described herein. In certain aspects, the present disclosure allows for fast and efficient screening of hundreds, thousands, tens of thousands, hundreds of thousands, or millions of cells.

## 4. BRIEF DESCRIPTION OF FIGURES

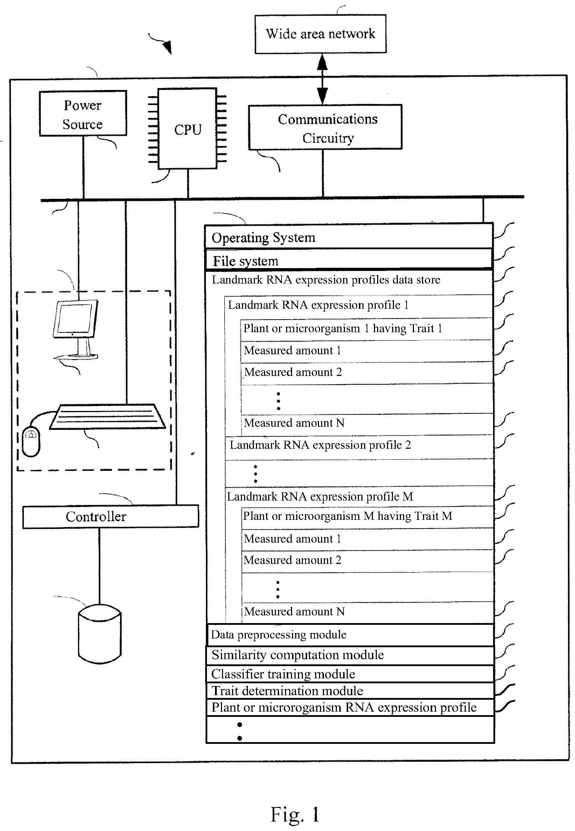

Figure 1. Program modules for computer program product to analyze and compare gene expression profiles.

## 5. DETAILED DESCRIPTION

Provided herein are methods to expedite the selection of organisms such as plants, animals, or microorganisms that possess one or more desired traits, *e.g.*, organisms that are optimized for particular environmental conditions. The present disclosure provides methods to improve organisms including plants, animals, and microorganisms by identifying and selecting plants, plant cells and microorganisms with gene expression profiles that correlate with improved growth properties and/or tolerance adverse environmental conditions and/or to enrich or reduce certain biologic products or metabolites that they produce. The present methods may be applied to identify and isolate cells (*e.g.,* plant cells, eukaryotic cells, or microbial cells) comprising desired gene expression profiles for production of improved strains or variants without genetic engineering. In certain aspects, present methods may be applied to identify and isolate cells (*e.g.,* plant cells, eukaryotic cells, or microbial cells) comprising desired gene expression profiles for production of improved strains or variants that have been genetically modified.

In certain aspects, cells with gene expression profiles that correlate with desired traits or with the absence of undesired traits are identified, selected, and cultivated. In certain aspects, the presence of a desired trait, or the absence of an undesired trait, correlates with the upregulation of a gene. Upregulation of the gene can be tested using fluorogenic oligonucleotide probes or molecular beacons that are capable of detecting expression of the gene (e.g., detecting transcripts of the gene). In certain aspects, gene expression is upregulated in a test cell if it is expressed at at least 10%, 25%, 50%, 75%, 100%, 250%, 500%, 750%, 1000%, 5000%, 10000%, 50000%, or at least 100000% higher levels than the gene is expressed in a reference cell or reference cell population. In certain aspects, gene expression is upregulated in a test cell if it is expressed at at least 0.2 fold, 0.5 fold, 1 fold, 2 fold, 3 fold, 4 fold, 5 fold, 6 fold, 7 fold, 8 fold, 9 fold, 10 fold, 100 fold, 500 fold, or 1,000 fold, or at least 10,000 fold higher levels than the gene is expressed in a reference cell or reference cell population. A reference cell can be a cell of the same organism, a cell of the same cell type, a cell that is derived from the same clonal cell, a cell that is derived from the same organism (*e.g.*, plant, animal or microorganism), a cell that is derived from the same part of an organism (*e.g.*, plant, animal or microorganism), a cell that possess the same function, as the test cell. A reference cell population can be the unselected starting population of cells. In certain aspects, the unselected starting population of cells comprises a heterogeneous population of cells. In specific aspects, an average value (*e.g.*, an average value of the expression level of a gene) calculated for the reference cell population is used as a reference to determine whether gene expression in a selected cell is unregulated or downregulated. In other words, gene expression of a selected cell is considered to be upregulated or downregulated relative to the average value calculated for the reference population.

In certain aspects, the presence of a desired trait, or the absence of an undesired trait, correlates with the downregulation of a gene. Downregulation of the gene can be tested using fluorogenic oligonucleotide probes or molecular beacons that are capable of detecting expression of the gene. In certain aspects, gene expression is downregulated in a test cell if it is expressed at at least 5%, 10%, 15%, 20%, 25%, 30%, 35%, 40%, 45%, 50%, 60%, 70%, 75%, 80%, 90%, 95%, 99%, or 100% lower levels than the gene is expressed in a reference cell or reference cell population. A reference cell can be a cell of the same organism, a cell of the same cell type, a cell that is derived from the same clonal cell, a cell that is derived from the same organism (*e.g.*, plant, animal or microorganism), a cell that is derived from the same part of an organism (*e.g.*, plant, animal or microorganism), or a cell that possess the same function, as the test cell. A reference cell population can be the unselected starting population of cells. In certain aspects, the unselected starting population of cells comprises a heterogeneous population of cells. In specific aspects, an average value (*e.g.*, an average value of the expression level of a gene) calculated for the reference cell population is used as a reference to determine whether gene expression in a selected cell is unregulated or downregulated. In other words, gene expression of a selected cell is considered to be upregulated or downregulated relative to the average value calculated for the reference population.

In the sections below, illustrative genes for use with the methods of the disclosure are listed. The skilled artisan will understand that, depending on the specific family or species or variety of the organism, these genes can also include homo logs of the specific gene listed. In certain aspects, a homolog of a gene encodes a protein whose amino acid sequence is at least 50%, 60%, 70%, 80%, 90%, 95%, 98%, 99%, or at least 99.5% identical to the amino acid sequence encoded by the gene. In certain aspects, mutants (*e.g.*, stop codons, frame shifts, nucleotide variants), different splice forms, and orthologs of the gene can also be used with the methods of the disclosure. In certain aspects, a gene that encodes a protein in the same pathway of one of the genes listed in the sections below can also be used with the methods of the disclosure. In certain, more specific aspects, the gene product can act upstream or downstream of the gene product of one of the genes listed in the sections below.

Described in more detail in the sections below are methods relating to plants, microorganisms, and non-human animals, and cells *(e.g.,* plant cells, microbial cells, or eukaryotic cells).

## 5.1 **Terminology**

As used herein, "transgenic plant" includes reference to a plant which comprises within its genome a heterologous polynucleotide. Generally, the heterologous polynucleotide is stably integrated within the genome such that the polynucleotide is passed on to successive generations. The heterologous polynucleotide may be integrated into the genome alone or as part of a recombinant expression cassette. "Transgenic" is used herein to include any cell, cell line, callus, tissue, plant part or plant, the genotype of which has been altered by the presence of heterologous nucleic acid including those transgenics initially so altered as well as those created by sexual crosses or asexual propagation from the initial transgenic. The term "transgenic" as used herein does not encompass the alteration of the genome (chromosomal or extra-chromosomal) by conventional plant breeding methods or by naturally occurring events such as random cross-fertilization, non-recombinant viral infection, non-recombinant bacterial transformation, non-recombinant transposition, or spontaneous mutation.

## 5.2 **Plants**

The present methods take advantage of the naturally occurring high degree of genetic diversity that exists in organisms, and efficiently identify, select, and enrich for plants and plant cells possessing desired gene expression profiles conferring improved properties. The present methods can identify, select, and/or enrich for plants with improved properties from a pool of genetically diverse plants or plant cells, *e.g.,* such as plants and plant cells that exist naturally. In particular aspects, the present methods allow for the generation of novel homogeneous populations possessing improved properties. In certain embodiments, the present methods include a step to increase genetic variability. Increasing genetic variability may be achieved using any one of various methods known to one skilled in the art. In specific embodiments, increasing genetic variability may be achieved as described herein in section 5.6 below.

In particular embodiments, the methods of the invention described herein allow for identification, selection and enrichment of crops that are resistant to or that have improved growth in conditions of drought, flood, cold, hot, low or lack of light, extended periods of darkness, nutrient deprivation, or poor soil quality including sandy, rocky acidic or basic soil. In particular embodiments, the methods of the invention described herein allow for identification, selection and enrichment of crops that grow faster, crops that generate more seed, crops that are resistant to pests and/or microorganisms, or crops with improved taste or consistency for use as foods.

In specific embodiments, the methods of the invention described herein provide for identification, isolation and/or enrichment of plants cells or plants that express one or more RNAs of interest. Such RNAs of interest may be any RNA that confers a benefit to the plant.

Non-limiting examples of RNAs of interest include messenger RNAs (mRNAs) that encode proteins; antisense RNA; small interfering RNA (siRNA); microRNA (miRNA); structural RNAs; cellular RNAs (*e.g.*, ribosomal RNAs, tRNAs, hnRNA, and snRNA); random RNAs; RNAs corresponding to cDNAs or ESTs; RNAs that may be incorporated into various macromolecular complexes; RNAs that are ribozymes; linker RNA, or sequence that links one or more RNAs; or RNAs that do not have the aforementioned function or activity but which may be expressed by cells nevertheless.

In other embodiments, the methods of the invention described herein provide for identification, isolation and/or enrichment of plants cells or plants that express a desired gene expression profile. In specific embodiments, the protoplasts are derived from seedlings.

In other embodiments, the methods of the invention described herein provide for identification, isolation and/or enrichment of seedlings that express a desired gene expression profile.

Non-limiting examples of genes encoding RNAs of interest, gene profiles, and desired phenotypes are described herein in section 5.2.2.

In specific embodiments, plants or plant cells of the methods described herein do not recombinantly express a transgene or have not been genetically modified. In other embodiments, plants or plant cells of the methods described herein recombinantly express one or more transgene or have been genetically modified. In particular embodiments, transgenes may encode for RNA or polypeptides.

### 5.2.1. *Plants and plant cells*

As used herein, the term "plant" is used in its broadest sense, including, but is not limited to, any species of woody, ornamental or decorative, crop or cereal, fruit or vegetable plant, and algae (*e.g., Chlamydomonas reinhardtii*). Non-limiting examples of plants include plants from the genus *Arabidopsis* or the genus *Oryza*. Other examples include plants from the genuses *Acorus, Aegilops, Allium, Amborella, Antirrhinum, Apium, Arachis, Beta, Betula, Brassica, Capsicum, Ceratopteris, Citrus, Cryptomeria, Cycas, Descurainia, Eschscholzia, Eucalyptus, Glycine, Gossypium, Hedyotis, Helianthus, Hordeum, Ipomoea, Lactuca, Linum, Liriodendron, Lotus, Lupinus, Lycopersicon, Medicago, Mesembryanthemum, Nicotiana, Nuphar, Pennisetum, Persea, Phaseolus, Physcomitrella, Picea, Pinus, Poncirus, Populus, Prunus, Robinia, Rosa, Saccharum, Schedonorus, Secale, Sesamum, Solanum, Sorghum, Stevia, Thellungiella, Theobroma, Triphysaria, Triticum, Vitis, Zea,* or *Zinnia.*"

In certain embodiments, plants may be any plants amenable to delivery of nucleic acid molecules or transformation techniques, including gymnosperms and angiosperms, both monocotyledons and dicotyledons. Examples of monocotyledonous angiosperms include, but are not limited to, asparagus, field and sweet corn, barley, wheat, rice, sorghum, onion, pearl millet, rye and oats and other cereal grains. Examples of dicotyledonous angiosperms include, but are not limited to tomato, tobacco, cotton, rapeseed, field beans, soybeans, peppers, lettuce, peas, alfalfa, clover, cole crops or *Brassica oleracea* (*e.g.,* cabbage, broccoli, cauliflower, brussel sprouts), radish, carrot, beets, eggplant, spinach, cucumber, squash, melons, cantaloupe, sunflowers and various ornamentals.

Examples of woody species include poplar, pine, sequoia, cedar, oak, *etc.* Still other examples of plants include, but are not limited to, wheat, cauliflower, tomato, tobacco, corn, petunia, trees, *etc.* As used herein, the term "cereal crop" is used in its broadest sense. The term includes, but is not limited to, any species of grass, or grain plant (*e.g.*, barley, corn, oats, rice, wild rice, rye, wheat, millet, sorghum, triticale, *etc.*), non-grass plants (*e.g.,* buckwheat flax, legumes or soybeans, *etc.*). As used herein, the term "crop" or "crop plant" is used in its broadest sense. The term includes, but is not limited to, any species of plant or algae edible by humans or used as a feed for animals or used, or consumed by humans, or any plant or algae used in industry or commerce. As used herein, the term "plant" also refers to either a whole plant, a plant part, or organs (*e.g.,* leaves, stems, roots, *etc.*), a plant cell, or a group of plant cells, such as plant tissue, plant seeds and progeny of same. Plantlets are also included within the meaning of "plant." The class of plants which can be used in the methods described herein is generally as broad as the class of higher plants amenable to delivery of nucleic acid molecules (*e.g.*, fluorogenic oligonucleotide probes) or transformation techniques, including both monocotyledonous and dicotyledonous plants.

In specific embodiments, the present methods of the invention allow for identification, selection and enrichment of agricultural staples, which include, but are not limited to, thyme, olive, cotton, wheat, tobacco, tea, coffee, cocoa, corn, soybean, sugar cane, and rice. In certain embodiments, plants described herein may be tea plants, or grape plants *(e.g.,* grape plants comprising grapes or grape vines). In particular embodiments, plant cells described herein are cells of a tea plant or a grape plant. In other embodiments, plants may be fruit plants (*e.g.*, strawberries, blueberries, raspberries, blackberries, apples, pears, oranges, grapefruits, melons), nut plants (*e.g.,* almond, pistachio, walnuts, macadamia, peanuts), or flower plants (*e.g.*, sunflowers, roses, carnations, lilies, lilac, daffaodils, daisies, hydrangea, *etc.*).

Plants may be horticultural plants which may include lettuce, endive, and vegetable brassicas including cabbage, broccoli, and cauliflower, and carnations and geraniums. Plants may also include tobacco, cucurbits, carrot, strawberry, sunflower, tomato, pepper, chrysanthemum, poplar, eucalyptus, and pine.

In certain embodiments, plants may be medicinal plants such as cannabis or hemp plants. In particular embodiments, plant cells may be plant cells of cannabis or hemp plant. In particular embodiments, plants described herein are used for production of narcotics or other medicinal agents (*e.g.*, ginseng). For example, the plant may be poppy, coca, or opium, which may be used for medicinal purposes. Other non-limiting examples of medicinal plants include black cohosh, bloodroot, blue cohosh, cascara sagrada, devil's club, Echinacea, eyebright, ginseng, goldenseal, lomatium, Oregon grape, osha, pink lady's slipper, spikenard, stoneroot, white sage, and wild indigo.

Plants described herein may belong to a plant species, which may include, but are not limited to, corn (*Zea mays*), canola (*Brassica napus, Brassica rapa ssp.*), alfalfa (Medicago sativa), rice (*Oryza sativa*)*,* rye (*Secale cereale*)*,* sorghum (*sorghum bicolor, Sorghum vulgar*), sunflower (*Helianthus annuus*), wheat (*Triticum aestivum*), soybean (*Glycine max)*, tobacco (*Nicotiana tabacum, Nicotiana benthamiana*), potato (*Solanum tuberosum*), peanuts (*Arachis hypogaea)*, cotton (*Gossypium hirsutum*), sweet potato (*Ipomoea batatus)*, cassaya (*Manihot esculenta)*, coffee (*Coffea spp.*), coconut (*Cocos nucifera*), pineapple (*Ananas comosus)*, citrus trees (*Citrus spp.*), cocoa (*Theobroma cacao*), tea (*Camellia sinensis)*, banana (*Musa spp.)*, avocado (*Persea americana)*, fig (*Ficus casica)*, guava (*Psidium guajava*), mango (*Mangifera indica*), olive (*Olea europaea*), papaya (*Carica papaya*), cashew (*Anacardium occidentale*), macadamia (*Macadamia integrifolia*), almond (*Prunus amygdalus*), sugar beets (*Beta vulgaris*), oats, barley, *Arabidopsis* spp., vegetables, ornamentals, and conifers.

The term "plant cell" as used in the context of the methods of the invention as defined by the claims appended to this description refers to protoplasts. The term "plant cell" as used in the context of the general disclosure may also refer to gamete producing cells, and cells which regenerate into whole plants. Plant cell, as used herein, further includes, without limitation, cells obtained from or found in: seeds, suspension cultures, embryos, meristematic regions, callus tissue, leaves, roots, shoots, gametophytes, sporophytes, pollen, and microspores. Plant cells can also be understood to include modified cells, such as protoplasts, obtained from the aforementioned tissues.

Any suitable plant cell may be used in the methods described herein, *e.g.,* introduction of fluorogenic oligonucleotide probes, selection, and regeneration of plants. Optionally, germ line cells may be used in the methods described herein rather than, or in addition to, somatic cells. The term "germ line cells" refers to cells in the plant organism which can trace their eventual cell lineage to either the male or female reproductive cell of the plant. Other cells, referred to as "somatic cells" are cells which give rise to leaves, roots and vascular elements which, although important to the plant, do not directly give rise to gamete cells. Somatic cells, however, also may be used. With regard to callus and suspension cells which have somatic embryogenesis, many or most of the cells in the culture have the potential capacity to give rise to an adult plant. If the plant originates from single cells or a small number of cells from the embryogenic callus or suspension culture, the cells in the callus and suspension can therefore be referred to as germ cells. In the case of immature embryos which are prepared for treatment by the methods described herein, certain cells in the apical meristem region of the plant have been shown to produce a cell lineage which eventually gives rise to the female and male reproductive organs. With many or most species, the apical meristem is generally regarded as giving rise to the lineage that eventually will give rise to the gamete cells. An example of a non-gamete cell in an embryo would be the first leaf primordia in corn which is destined to give rise only to the first leaf and none of the reproductive structures.

Non-limiting example of crops and other taste consumables / intoxicants /stimulants and some of their uses are described in Table 1. In specific embodiments, a plant is a crop selected from those listed in Table 1. In certain embodiments, a plant is selected for an improved property for a use selected from those listed in Table 1. In particular embodiments, a plant possessed a genetic modification property selected from those listed in Table 1.

**Table 1: Crops/Taste consumables/ Intoxicants /Stimulants and Uses**

| Category | Use | Common Name | Botanical name | Family | GMO/ Modification / properties | References |
|---|---|---|---|---|---|---|
| Forage; leguminous crops | Forage for high producing cows (high level of Calcium and protein)<br><br>Medical use (homeopathy , GI tract disorders) | Alfalfa / Lucerne / Lucerne grass | *Medicago sativa* | Fabaceae | herbicide-resistant seeds Monsanto and Forage Genetics International | www.planetar k.com/dailyne wsstory.cfm/n ewsid/35154/s tory.htm |
| Bean | Food and food additive | Cocoa | *Theobroma cacao* | Sterculia ceae | | |
| Stimulant/B ean | Stimulant beverage | Coffee | *Coffea* | Rubiacea e | | |
| Fiber / Oilseed | textile fiber | Cotton / Cotton seed oil | *Gossypium vitifolium* | Malvace ae | crystal protein gene added/transferred into plant genome<br><br>gene coding for BT toxin inserted into cotton | |
| | | | | | genome<br>Pest-resistant cotton | |
| Grain/Forag e | Livestock feed grain Food<br><br>Food and other additive (*e.g.,* starch, sweeteners, corn oil, beverage and industrial alcohol, and fuel ethanol)<br>largest component of global coarse-grain trade | Corn | *Zea mays* | Poaceae | Resistant to glyphosate or glufosinate herbicides, Insect resistance<br>Vitamin-enriched | Shaista Naqvi, et al. Transgenic multivitamin corn through biofortificatio n of endosperm with three vitamins representing three distinct metabolic pathways PNAS April 27, 2009. |
| Grain | Food | Sweet Corn | *Zea mays* var. *rugosa* | Poaceae | Gene from the bacteria *Bacillus thuringiensis* added to the plant. Produces its own bioinsecticide (B.t. toxin) | |

(continued)

| Category | Use | Common Name | Botanical name | Family | GMO/ Modification / properties | References |
|---|---|---|---|---|---|---|
| Grain | flavoring and stability agent in beer bittering agent herbal medicine | Hops | *Humulus lupulus* | Cannaba ceae | | |
| Stimulant | Drug | Marijuana | *Cannabis sativa* | Cannaba ceae | | |
| Fruit | Food / culinary use Skin care | Olive Oil | *Olea europaea* | Oleaceae | | |
| | Medicinal use | | | | | |
| Stimulant | Opium (stimulant narcotic) Medicinal properties | Poppy | *Papaver rhoeas* | Papavera ceae | | |
| Vegetable/R oot and Tuber | Food | Potato | **Solanum tuberosum** | Solanace ae | Key enzyme for the synthesis of amylose switched off by inserting antisense copy of the GBSS gene *Amflora* variety produces starch composed almost exclusively of the amylopectin component of starch | http://www.ba sf.com/group/ corporate/en/i nnovations/in novative-solutions/amfl ora |
| Oilseed | Animal feed vegetable oil for human consumption biodiesel | Rapeseed | *Brassica napus* | Brassica ceae | Resistance to herbicides (glyphosate or glufosinate), High laurate canola | http://www.ge o-pie.cornell.ed u/traits/altoil. html |
| Cereal grain | Food | Rice | *Oryza sativa,* | Poaceae (Grass family) | "Golden rice": Three new genes implanted: two from daffodils and the third from a bacterium Genetically modified to contain high amounts of Vitamin A (beta-carotene) | |

EP 2 462 222 B1

| Category | Use | Common Name | Botanical name | Family | GMO/ Modification / properties | References |
|---|---|---|---|---|---|---|
| Grain legume | source of protein feed<br><br>source of vegetable oil | Soybean | *Glycine max* | Fabaceae | First commercially grown GMO<br><br>Herbicide resistant gene | |
| | | | | | taken from bacteria inserted into soybean Resistant to glyphosate or glufosinate herbicides | |
| Sugar | Food additive | Sugar cane | *Saccharum* | Poaceae | Resistance to certain pesticides, High-sucrose cane. | |
| Stimulant | Intoxicating comsumable<br><br>organic pesticide | Tobacco | *Nicotiana (tabacum)* | Solanaceae | | |
| Fruit and vegetable | Food | Tomato | *Lycopersicon esculentum* | | An antisense gene of the gene responsible for the production of PG enzyme added into plant<br><br>Variety in which the production of the enzyme polygalacturonase (PG) is suppressed, retarding fruit softening after harvesting | www.foodsaf ety.gov/~lrd/b iotechn.html<br><br>U.S. Food and Drug Administratio n Center for Food Safety and Applied Nutrition, Biotechnology of Food. FDA Backgrounder : May 18, 1994 |
| Cereal grain / Forage | Food (*e.g.,* Couscous; Tabula, Pilaf) (Semolina for pasta)<br><br>Forage | Durum Wheat (Macaroni Wheat) | *Tritium durum* | Poaceae | | |
| Cereal grain | Food | Winter Wheat (Common Wheat) | *Tritium aestivum* | Poaceae | | |

In specific embodiments plants or plant cells are purified (*e.g.*, contains less than 20%, 15%, 10%, or 5% contaminants).

### 5.2.2. *Genes and Phenotypes*

#### 5.2.2.1 *Plant Processes/Pathways*

In particular aspects, the methods described herein provide for identification, isolation, and/or enrichment of plant cells and plants that express one or more genes of interest. As used herein, genes of interest may encode for an RNA, which may or may not be translated into a polypeptide of interest. Such genes may include any gene related to a plant growth or development pathway. In certain embodiments, provided herein are methods for the selection of protoplasts that have a desired gene expression pattern or profile. A desired gene expression pattern may include certain genes being upregulated and others being downregulated, as well as particular differences or ratios of the level of expression of the genes relative to each other. In certain embodiments, the expression level of a gene of a selected protoplast is upregulated or downregulated relative to the average expression level of that gene in an unselected population. In general, certain genes that positively regulate or confer upon a plant a desired trait, are upregulated at the appropriate time. Certain genes that negatively regulate or confer upon a plant an undesired trait are downregulated at the appropriate time.

In specific embodiments, the expression level of a gene of a selected protoplast is upregulated by at least 5%, 10%, 20%, 30%, 40%, 50%, 60%, 70%, 80%, 90%, 95%, or 100% relative to the average expression level of that gene in an unselected population. In specific embodiments, the expression level of a gene of a selected protoplast is upregulated by at least 0.2 fold, 0.5 fold, 1 fold, 2 fold, 3 fold, 4 fold, 5 fold, 10 fold, 15 fold, 20 fold, 25 fold, 30 fold, 50 fold, or 100 fold relative to the average expression level of that gene in an unselected population. In specific embodiments, the expression level of a gene of a selected protoplast is downregulated by at least 5%, 10%, 20%, 30%, 40%, 50%, 60%, 70%, 80%, 90%, 95%, 99%, or 100% relative to the average expression level of that gene in an unselected population.

The skilled artisan will know which traits are desired for which plant under what circumstances. Desired traits include, but are not limited to, drought resistance, flood resistance, disease resistance, pest resistance, toxin or stress resistance, and increased activity of biosynthetic pathways.

In more specific embodiments, a plant is to be grown in an area that suffers from floods; in this case, flood resistance (*e.g.*, being able to sustain and/or grow under conditions of unusual excess of water) would be a desired property.

In other embodiments, a plant is to be grown in an area that suffers from drought; in this case, drought resistance, *e.g.*, being able to sustain and/or grow under conditions of unusually continuously dry conditions with little or now water for a long period of time, would be a desired property. For example, the drought period may be at least 3 months, 4 months, 5 months, 6 months, 9 months, 12 months, 18 months, 24 months, 30 months, 36 months, 42 months, or 48 months.

In other embodiments, a plant is to be grown in an area that has little light; in this case, the ability to sustain and/or grow under conditions with little sunlight would be a desired property. In certain embodiments, the ability to sustain and/or grow under conditions with little sunlight for at least 3 months, 4 months, 5 months, 6 months, 9 months, 12 months, 18 months, 24 months, 30 months, 36 months, 42 months, or 48 months would be desired. In some embodiments, conditions with little sunlight refers to conditions where a plant come into direct contact with sunlight on average for at most 2 hours out of a day, 1 hour out of a day, at most 30 minutes out of a day, at most 10 minutes out of a day, at most 1 minute out of a day, at most 2 hours out of a week, at most 1 hour out of a week, at most 30 minutes out of a week, at most 10 minutes out of a week, at most 2 hours out of every two weeks, at most 1 hour out of every two weeks, at most 30 minutes out of every two weeks, at most 3 hours out of a month, at most 1 hour out of a month, at most 30 minutes out of a month, at most 10 hours out of a year, at most 5 hours out of a year, or at most 1 hour out of a year.

In some embodiments, a plant is to be grown in an area with poor quality soil, *e.g.,* soil with low mineral content; in such case, the ability to sustain and/or grow in an environment with poor soil quality would be a desired property. In certain embodiments, soil with less than 45%, 40%, 35%, 30%, 25%, 20%, 15%, 10%, 5%, 2.5%, or 1% mineral content is considered to be poor quality soil.

In certain embodiments, the desired trait of a plant is conferred by increased activity of one or more biosynthetic pathways. Such biosynthetic pathways include photosynthesis, production of cellulose, nitrogen assimilation, biosynthesis of plant hormones, and biosynthesis of plant metabolites. Non-limiting examples of plant hormones include, salicylic acid, jasmonates, ethylene, abscisic acid, auxin, gibberellic acid, cytokinin, and brassinosteroids. Salicylic acid, jasmonates, and ethylene are involved in plant responses to biotic stresses.

Similarly, the skilled artisan will understand which traits are not desired under a given set of environmental conditions. Undesired traits include, but are not limited to, increased activity of biodegredation pathways.

In certain embodiments, increased or upregulated gene expression and decreased or downregulated gene expression in a protoplast is determined by comparison to the protoplast from which the protoplast was derived.

**Photosynthesis**

In specific embodiments, protoplasts or plants described herein express or have upregulated expression of one or more genes (*e.g.*, genes encoding RNA or polypeptide) that is related to the photosynthesis pathway. Non-limiting examples of gene products related to the photosynthesis pathway are listed below:

Photosystem II: psbA, photosystem II PsbA protein; psbB, photosystem II PsbB protein; psbC, photosystem II PsbC protein; psbD, photosystem II PsbD protein; psbE, photosystem II PsbE protein; psbF, photosystem II PsbF protein; psbH, photosystem II PsbH protein; psbI, photosystem II PsbI protein; psbJ, photosystem II PsbJ protein; psbK, photosystem II PsbK protein; psbL, photosystem II PsbL protein; psbM, photosystem II PsbM protein; psbO, photosystem II PsbO protein; psbP, photosystem II oxygen-evolving complex 23kDa protein; psbQ, photosystem II oxygen-evolving enhancer protein; psbR, photosystem II 10kDa protein; psbS, photosystem II 22kDa protein; psbT, photosystem II PsbT protein; psbU, photosystem II PsbU protein; psbV, photosystem c550; psbW, photosystem II PsbW protein; psbX, photosystem II PsbX protein; psbY, photosystem II PsbY protein; psbZ, photosystem II PsbZ protein; psb27, photosystem II Psb27 protein; psb28, photosystem II reaction center 13kDa protein; and psb28-2, photosystem II Psb28-2 protein.

Photosystem I: psaA, photosystem I core protein Ia; psaB, photosystem I core protein Ib; psaC, photosystem I subunit VII; psaD, photosystem I subunit II; psaE, photosystem I subunit IV; psaF, photosystem I subunit III; psaG, photosystem I subunit V; psaH, photosystem I subunit VI; psaI, photosystem I subunit VIII; psaJ, photosystem I subunit IX; psaK, photosystem I subunit X; psaL, photosystem I subunit XI; psaM, photosystem I subunit XII; psaN, photosystem I subunit PsaN; psaX, photosystem I 4.8kDa protein; Cytochrome b6/f complex: K02634 petA, apocytochrome f; petB, cytochrome b6; petC, cytochrome b6-f complex iron-sulfur subunit; petD, cytochrome b6-f complex subunit 4; petG, cytochrome b6-f complex subunit 5; petL, cytochrome b6-f complex subunit 6; petM, cytochrome b6-f complex subunit 7; petN, cytochrome b6-f complex subunit 8.

Photosynthetic electron transport genes: petE, plastocyanin; petF, ferredoxin; petH, ferredoxin--NADP+ reductase; petJ, and cytochrome c6.

F-type ATPase: ATPF1E, atpC, F-type H+-transporting ATPase subunit epsilon; ATPF1B, atpD, F-type H+-transporting ATPase subunit beta; ATPF1G, atpG, F-type H+-transporting ATPase subunit gamma; ATPF1A, atpA, F-type H+-transporting ATPase subunit alpha; ATPF1D, atpH, F-type H+-transporting ATPase subunit delta; ATPF0B, atpF, F-type H+-transporting ATPase subunit b; ATPF0C, atpE, F-type H+-transporting ATPase subunit c; ATPF0A, atpB, F-type H+-transporting ATPase subunit a; and atpI; ATP synthase protein I.

In specific embodiments, in accordance with the methods of the invention described herein, a protoplast is identified, isolated, and/or enriched for expressing (or overexpressing) one or more photosynthesis-related genes. In certain embodiments, a plant derived from a protoplast expressing (or overexpressing) one or more these photosynthesis-related genes may have one or more desirable traits, *e.g.*, ability to process sunlight into energy more efficiently, and thus ability to sustain and/or grow under conditions with low sunlight.

In certain embodiments, a gene that encodes an RNA or protein that is related to the photosynthesis pathway is downregulated. In specific embodiments, the RNA or protein is a negative regulator of photosynthesis such that downregulation of the negative regulator of photosynthesis results in upregulation of photosynthesis.

**Seed Germination**

The process of seed germination has been described, see, *e.g.,* John M. Riley, CRFG J., 1987, 19:10-12. Seed germination is regulated by several environmental factors, such as moisture, oxygen, temperature, light, and nutrients (see, *e.g.,* Seo et al., Plant Mol Biol., 2009, 69(4):463-72). The first stage of germination consists of ingesting water and an awakening or activation of the germ plasma. Protein components of cells formed as the seed develops, become inactive as it matures. After an uptake of water, the system is reactivated and protein synthesis resumes. Enzymes and hormones appear and begin to digest reserve substances in the storage tissues and to translocate the digested substances in the storage tissues to the growing points of the embryo. The sequence of the metabolic pattern than occurs during germination involves the activation of specific enzymes at the proper time and regulation of their activity.

Control is exercised by four classes of plant hormones: inhibitors such as abscissic acid which block germination; auxins which control root formation and growth; the gibberellins (e.g., gibberellic acid) which regulate protein synthesis and stem elongation; and cytokinins that control organ differentiation. Ethylene is also believed to have a control function in some plants. Sometimes the last three controls are used together to crash through dormancy in germinating difficult seed.

In specific embodiments, in accordance with the methods of the invention described herein, a protoplast is identified, isolated, and/or enriched for expressing (or overexpressing) one or more genes (*e.g.*, genes encoding RNA or polypeptide) involved in seed germination. In certain embodiments, a plant derived from a protoplast expressing (or overexpressing) one or more these genes involved in seed germination may have one or more desirable traits, *e.g.*, ability to germinate and/or grow under conditions that have undesirable moisture, oxygen, temperature, light, and/or nutrient levels.

For example, high concentrations of gibberellic acid may be effective in overcoming dormancy and causing rapid germination of seed. In certain embodiments, concentrations of about 2 ppm of gibberellic acid can cause tubers to sprout earlier.

In certain embodiments where a plant is sufficiently developed, premature flowering may be induced by gibberellic acid. Formation of male flowers may be promoted by concentrations of about 10 to 200 ppm, and female flowers by concentrations of 200 to 300 ppm. Concentrations of more than 600 ppm may suppresses initiation of both male and female flowers.

In certain embodiments when there is difficulty with fruit set because of incomplete pollination, gibberellic acid may be effectively used to increase fruit set. The resulting fruit maybe partially or entirely seedless. In other embodiments, gibberellic acid may increase the total yield in greenhouse tomato crops both as a result of increased fruit set and more rapid growth of the fruit.

In other embodiments, gibberellic acid may also increase growth, provide frost protection, and/or inhibit root formation.

In certain embodiments, a gene that encodes an RNA or protein that is related to the seed germination process is downregulated. In specific embodiments, the RNA or protein is a negative regulator of seed germination such that downregulation of the negative regulator of seed germination results in upregulation of seed germination.

## Nitrogen Assimilation Pathway

Nitrate is a key required nutrient for the synthesis of amino acids, nucleotides and vitamins and is commonly considered to be one of the most limiting for normal plant growth. Nitrates and ammonia resulting from nitrogen fixation are assimilated into the specific tissue compounds of algae and higher plants. Nitrogen-responsive master regulatory control genes include *CCAI, GLKI* and *BZIPI.*

Genes involved in the uptake and reduction of nitrate (NIA, NIR) are transcriptionally induced by nitrate. By contrast, the glutamine synthetase gene (GLN1.3) involved in assimilating inorganic N into organic form (Gln), is transcriptionally repressed by the endproducts of N-assimilation (Glu/Gln). The repression of GLN1.3 expression by the product of the glutamine synthetase (GS) enzyme reaction serves as a negative feedback loop, that shuts off further assimilation of inorganic N into Gln, when levels of Gln are abundant. As GS is an ATP dependent enzyme, this is likely to be an energy conservation mechanism. By contrast, Gln/Glu levels activate the expression of the ASN1 gene (asparagine synthetase) which serves to transfer the amide N from Gln onto Asp to make Asn and Glu as a byproduct. Asn is an inert amino acid used to store N and used for long distance N-transport *(e.g.,* to seed). The induction of ASNI by Glu/Gln is a mechanism that serves to store excess N as Asn, which is used to transport N to seed.

In specific embodiments, in accordance with the methods of the invention described herein, a protoplast is identified, isolated, and/or enriched for expressing (or overexpressing) one or more gene products (*e.g.*, RNA or polypeptide) involved in nitrogen assimilation. In certain embodiments, a plant derived from a protoplast expressing (or overexpressing) one or more gene products involved in nitrogen assimilation may have one or more desirable traits, *e.g.*, ability to sustain and/or grow under conditions with low sunlight.

In certain embodiments, a gene that encodes an RNA or protein that is related to the nitrogen assimilation pathway is downregulated. In specific embodiments, the RNA or protein is a negative regulator of nitrogen assimilation such that downregulation of the negative regulator of nitrogen assimilation results in upregulation of nitrogen assimilation.

## Stress Pathways

In specific embodiments, protoplasts or plants described herein express one or more genes of interest that is related to the oxidative stress pathway. For example, dehydration-inducible genes encoding aldehyde dehydrogenases (ALDHs) belong to a family of NAD(P)+-dependent enzymes with a broad substrate specificity that catalyze the oxidation of various toxic aldehydes to carboxylic acids. Analysis of transcript accumulation revealed that Ath-ALDH3 is induced in response to NaCl, heavy metals ($Cu^{2+}$ and $Cd^{2+}$), and chemicals that induce oxidative stress (methyl viologen (MV) and $H_2O_2$). Transgenic Arabidopsis plants overexpressing ALDH3 show improved tolerance when exposed to dehydration, NaCl, heavy metals ($Cu^{2+}$ and $Cd^{2+}$), MV, and $H_2O_2$; thus, increased activity of ALDH3 appears to constitute a detoxification mechanism that limits aldehyde accumulation and oxidative stress, thus revealing a novel pathway of detoxification in plants (see, *e.g.,* Sunkar et al., Plant J., 2003, (4):452-64). In specific embodiments, plant cells or plants overexpressing ALDH3 may confer drought-resistant properties or toxin- or oxidative stress-resistant properties. In certain embodiments, the methods described herein may select for protoplasts transcribing ALDH3. In certain embodiments, the methods described herein may select for protoplasts transcribing ALDH3 at higher levels than average levels transcribed by plants cells or plants in an naturally occurring and unselected population.

In certain embodiments, a gene that encodes an RNA or protein that is related to the stress pathway is downregulated. In specific embodiments, the RNA or protein is a negative regulator of the stress pathway such that downregulation of the negative regulator of the stress pathway results in upregulation of the stress pathway.

5.2.2.2 Other *Plant Genes/Gene Products*

In specific embodiments, plants (*e.g.*, tobacco) or plants cells expressing or overexpressing the P5 CS enzyme may show enhanced plant growth under salt and/or draught stresses.

In specific embodiments, plants (*e.g.*, tobacco or rice) or plants cells expressing or overexpressing BADH may show increased salt resistance.

In certain embodiments, plants and/or protoplasts described herein may express or have upregulated expression of one or more genes listed Table 2 below. Table 2 also describes non-limiting examples of functions of the listed genes. Thus, in a particular embodiment, a plant or protoplast expressing, or having upregulated expression of, one or more of the genes in Table 2, may possess an improved function that correlates to the function of that gene. In certain embodiments, a plant or protoplast has downregulated expression of one or more genes selected from the group listed in Table 2. In certain embodiments, at least one gene is downregulated and at least one gene is upregulated in a plant or plant cell. In certain embodiments, a negative regulator of a desired trait is downregulated. In certain embodiments, a positive regulator of an undesired trait is upregulated.

**Table 2: Plant genes and their functions**

| GENE | FUNCTION | REFERENCE |
|---|---|---|
| genes with the nucleotide binding sites and N-terminal domain with Toll/Interleukin-1 receptor homology (TIR), including the genes N, M, L6, RPP1, and RPP5 | Plant disease resistance | Meyers, 1999, The Plant Journal Vol. 3: 317-332 |
| genes with the nucleotide binding site containing monocot and dicot sequences, including the genes RPS2, RPM1, I2, Mi, Dm3, Pi-B, Xa1, RPP8, RPS5, and Prf. | Plant disease resistance | Meyers, 1999, The Plant Journal Vol. 3: 317-332 |
| stress-inducible aldehyde dehydrogenase gene: Ath-ALDH3 | Stress resistance | Sunkar, 2003, The Plant Journal Vol. 4: 452-464 |
| ALDH7 | Tolerance to drought, salinity, and oxidative stress | Rodrigues, 2006, Journal of Experimental Botany Vol. 9: 1909-1918 |
| ABA- and dehydration-inducible aldehyde dehydrogenase genes: Cp-ALDH, Ath-ALDH3, and Ath-ALDH4 | Drought resistance | Kirch, 2001, The Plant Journal Vol. 5: 555-567 |
| ZmALDH22A1 | Stress tolerance | Huang, 2008, Plant Mol Biol. Vol. 4-5: 451-463 |
| GR-RBP4 | Various stress conditions (high salinity, dehydration, cold stress) | Kwak, 2005, Journal of Experimental Botany Vol. 56: 3007-3016 |
| CuZnSOD and APX genes | Tolerance to wide range of abiotic stresses | Lee, 2007, Journal of Plant Physiol. Vol. 12: 1626-1638 |
| PgTIP1 | Salt tolerance, drought tolerance and cold acclimation ability | Peng, 2007, Planta Vol. 3: 729-740 |

(continued)

| GENE | FUNCTION | REFERENCE |
|---|---|---|
| ALDH3I1 and ALDH7B4 | Tolerance to abiotic stress, protection against lipid peroxidation and oxidative stress | Kotchoni, 2006, Plant Cell Environ. Vol. 6: 1033-1048 |
| ITN1 | Salt-stress tolerance | Sakamoto, 2008, The Plant Journal Vol. 3: 411-422 |
| codA gene from Arthrobacter globiformis | Tolerance to light-stress | Alia, 1999, Plant Mol Biol. Vol. 2: 279-288 |
| AtMYB44 | Tolerance to abiotic stress | Jung, 2008, Plant Physiology Vol. 146: 623-635 |
| OsTOP6A1 (meiotic recombination protein gene homolog) | Tolerance to abiotic stress | Jain, 2008, Plant Cell Rep. Vol. 4: 767-778 |
| Arabidopsis NDPK2 (AtNDPK2) gene | Enhanced tolerance against multiple environmental stresses | Tang, 2008, Transgenic Res. Vol. 4:705-715 |
| AtLTLI | Salt tolerance | Naranjo, 2006, Plant Cell Environ. Vol. 10: 1890-1900 |
| AtGSK 1 | NaCl tolerance | Piao, 2001, The Plant Journal Vol. 4: 305-314 |
| AtIpk2beta (inositol polyphosphate 6-/3-kinase) | Stress tolerance | Yang, 2008, Plant Mol Biol. Vol. 66: 329-343 |
| NDPKs in plants (AtNDPK2, AtMPK3, and AtMPK6) | Multiple stress tolerance | Moon, 2003, PNAS Vol. 100: 358-363 |
| Several genes in barley | Drought stress | Guo, 2009, Journal of Experimental Botany. Epub ahead of print |
| High mobility group B proteins (HMGB2, HMGB3, HMGB4 and HMGB5) | Salinity, drought or cold stress | Kwak, 2007, Plant Cell Physiol. Vol. 2: 221-231 |
| HMGB1 | Influence on growth, stress tolerance, and transcriptome | Lildballe, 2008, J Mol Biol. Vol. 1:9-12 |
| AtTSB1 | Tolerance to cadmium stress | Sanjaya, 2008. Plant Cell Environ. Vol. 8: 1074-1085 |
| DREB1A | Improving drought, salt, and freezing tolerance | Kasuga, 1999, Nature Biotechnology Vol. 17: 287-291 |
| TaCRT | Tolerance to drought | Jia, 2007, Journal of Experimental Botany Vol. 59: 739-751 |
| TsVP | Drought resistance | Li, 2008, Plant Biotechnol. Journal Vol. 2: 146-159 |
| GmbZIP132 | Tolerance to abscisic acid and salt | Liao, 2008, J Integr Plant Biol. Vol. 2: 221-230 |

(continued)

| GENE | FUNCTION | REFERENCE |
|---|---|---|
| OsWRKY 11 | Tolerance to heat and drought | Wu, 2009, Plant Cell Rep. Vol. 1: 21-30 |
| OsDREB1F | Tolerance to salt, drought, and low temperature | Wang, 2008, Plant Mol Biol. Vol. 6:589-602 |
| HVA1 | Tolerance to salt | Oraby, 2005, Crop Science Vol. 45: 2218-2227 |
| glutamine synthetase (GS) | Nitrogen assimilation | Miflin, 2002, Journal of Experimental Botany Vol. 53: 979-987 |
| glutamate dehydrogenase (GDH) | Nitrogen assimilation | Miflin, 2002, Journal of Experimental Botany Vol. 53: 979-987 |
| glutamate synthase (GOGAT) | Nitrogen assimilation | Kumar, 2009, Mol Biol Rep. Epub ahead of print |
| Alfinl, ARSK1, ATCDPK1, ATCDPK2, Atmyb2, AtP5CS, AtPLCl, cor6.6, kin1, mlipl5, MsPRP2, OsBZ8, PKABA1, rd22, rd29A (COR78), rd29B | Tolerance to salt | Winicov, 1998, Annals of Botany Vol. 82: 703-710 with references to publications on the single genes |
| Apo-Inv, ADC, BADH, betB, CDH, codA, CDPK, CAXI, EhCaBP, CaN, OsCDPK7, GST, GPX, GPD, AtGSK1, GS2, DnaK/HSP70, HKT1a, ipt, HVA1 (a LEA), mt1D, IMT1, fad7, Osmotin-like protein, proline, AhProT1, HNX1a, Alfinl, RCl3, RHL, SAMCD, AT-DBF2, SPD, SR-like putative splicing protein, DREB1A, AhDREB1, TPSP, Hal2, Hal1, Mn-SOD, AVP1 | Tolerance to salt | Flowers, 2004, Journal of Experimental Botany Vol. 55: 307-319 with references to publications on the single genes |
| SNAC1 | Drought resistance and salt tolerance | Hu, 2006, PNAS Vol. 103: 12987-12992 |
| OsMAPK5 | Disease resistance and abiotic stress tolerance | Xiong, 2003, Vol. 15: 745-759 |
| Sub1A | Tolerance to submergence | Fukao, 2008, PNAS Vol. 105: 16814-16819 |
| Ch | Resistance to cold | Kozik, 2008, J. Amer. Soc. Hort. Sci. Vol. 2: 225-227 |
| miR167 | lateral root formation in response to nitrogen | Patent application WO 08/115487 |
| CCA1, GLK1, bZIP1 | nitrogen-responsive genes/nitrogen assimilation in response to Glu | Patent application WO 08/118394 |
| NIA, NIR | uptake and reduction of nitrate | Patent application WO 08/118394 |
| glutamine synthetase gene (GLN1.3) | N-assimilation | Patent application WO 08/118394 |

(continued)

| GENE | FUNCTION | REFERENCE |
|---|---|---|
| asparagine synthetase gene (ASN 1) | makes Asn and Glu from Gln and Asp | Patent application WO 08/118394 |
| family of Glutamate receptors (GluRs): ionotropic (iGluR), metabotropic (mGluR) | | Patent application WO 96/39805 |
| STAR1 and STAR2 genes in rice | Aluminium tolerance in rice | Huang, 2009, Plant Cell. Vol. 21 (2): 655-667 |
| gene phytochrome C | Contribution to breed improvement | US Patent 7566815 |
| Ehdl (B-type response regulator in rice) | Short-day promotion of flowering, controls FT-like gene expression independently of Hdl | Doi, 2004, Genes & Development Vol. 18: 926-936 |
| GmCRY1a and GmCRY2a (cryptochromes in soybean) | Affect blue light inhibition of cell elongation | Zhang, 2008, PNAS Vol. 105 (52): 21028-21033 |
| 2B (Ppd-B1) and 2D (Ppd-D1) genes in hexaploid wheat | Mutations conferring photoperiod insensitivity | Wilhelm, 2009, Theor Appl Genet. Vol. 118 (2): 285-294Wilhwlm, 2009, Theor Appl Genet. Vol. 118 (2): 285-294 |
| Clock-gene homologs GmLCL1, GmLCL2<br><br>GmTOC1 | GmLCL1, GmLCL2: morning genes GmTOC 1: evening gene | Liu, J Plant Physiol. Vol. 166 (3): 278-289 |
| CiMFL and AtFLC | MADS FLOWERING LOCUS C-LIKE (MFL) sequence in Cichorium intybus | Locascio. 2009, New Phytol. Vol. 182 (3): 630-643 |
| TaARF (wheat adenosine diphosphate-ribosylation factor) | Ectopic overexpression increases growth rate in Arabidosis | Yao, 2009, J Integr Plant Biol. Vol. 51 (1): 35-44 |
| OsCO3 (CONSTANS-LIKE gene) | Controls flowering in rice | Kim, 2008, Planta Vol. 228 (2): 355-365 |
| TaHdl-1. TaHdl-2, and TaHdl-3 (wheat genes) | Role in photoperiodic flowering pathway | Nemoto, 2003, Plant J. Vol. 36 (1): 82-93 |
| Hd3a (rice ortholog of the Arabidopsis FT gene) | Promotes transition to flowering downstream of Hdl under short-day conditions | Kojima, 2002, Plant Cell Physiol. Vol. 43 (10): 1096-1105 |
| 190 proteins in tomato pollen | Various functions | Sheoran, 2007, Journal of Experimental Botany Vol. 58 (13): 3525-3535 (See table 1) |
| FRI and FLC (in Arabidopsis Allopolyploids) | Control flowering time | Wang, 2006, Genetics Vol. 173: 965-974 |

(continued)

| GENE | FUNCTION | REFERENCE |
|---|---|---|
| OsFY (homolog of AtFY) | Encodes a protein that can interact with OsFCA-gamma in rice | Lu, 2006, Acta Biochim Biophys Sin (Shanghai) Vol. 38 (7): 492-499 |
| Imtl in Mesembryanthemum crystallinum | Novel nyoinositol O-methyl transferase induced by osmotic stress | Vernon, 1992, EMBO J. Vol. 11 (6): 2077-2085 |

In other embodiments, a plant or protoplast described herein may express, or have upregulated expression of, one or more genes encoding an enzyme, such as one having a function or involved in a function selected from the group consisting of: Lipase/Esterase; Enantioselective hydrolysis of esters (lipids)/thioesters; Resolution of racemic mixtures; Synthesis of optically active acids or alcohols from meso-diesters; Selective syntheses; Regiospecific hydrolysis of carbohydrate esters; Selective hydrolysis of cyclic secondary alcohols; Synthesis of optically active esters, lactones, acids, alcohols; Transesterification of activated/nonactivated esters; Interesterification; Optically active lactones from hydroxyesters; Regio- and enantioselective ring opening of anhydrides; Detergents; Fat/Oil conversion; Cheese ripening; Protease; Ester/amide synthesis; Peptide synthesis; Resolution of racemic mixtures of amino acid esters; Synthesis of non-natural amino acids; Detergents/protein hydrolysis; Glycosidase/Glycosyl transferase; Sugar/polymer synthesis; Cleavage of glycosidic linkages to form mono, di- and oligosaccharides; Synthesis of complex oligosaccharides; Glycoside synthesis using UDP-galactosyl transferase; Transglycosylation of disaccharides, glycosyl fluorides, aryl galactosides; Glycosyl transfer in oligosaccharide synthesis; Diastereoselective cleavage of (-glucosylsulfoxides); Asymmetric glycosylations; Food processing; Paper processing; Phosphatase/Kinase; Synthesis/hydrolysis of phosphate esters; Regio-, enantioselective phosphorylation; Introduction of phosphate esters; Synthesize phospholipid precursors; Controlled polynucleotide synthesis; Activate biological molecule; Selective phosphate bond formation without protecting groups; Mono/Dioxygenase; Direct oxyfunctionalization of unactivated organic substrates; Hydroxylation of alkane, aromatics, steroids; Epoxidation of alkenes; Enantioselective sulphoxidation; Regio- and stereoselective Bayer-Villiger oxidations; Haloperoxidase; Oxidative addition of halide ion to nucleophilic sites; Addition of hypohalous acids to olefinic bonds; Ring cleavage of cyclopropanes; Activated aromatic substrates converted to ortho and para derivatives; diketones converted to 2-halo-derivatives; Heteroatom oxidation of sulfur and nitrogen containing substrates; Oxidation of enol acetates, alkynes and activated aromatic rings; Lignin peroxidase/Diarylpropane peroxidase; Oxidative cleavage of C--C bonds; Oxidation of benzylic alcohols to aldehydes; Hydroxylation of benzylic carbons; Phenol dimerization; Hydroxylation of double bonds to form diols; Cleavage of lignin aldehydes; Epoxide hydrolase; Synthesis of enantiomerically pure bioactive compounds; Regio- and enantioselective hydrolysis of epoxide; Aromatic and olefinic epoxidation by monooxygenases to form epoxides; Resolution of racemic epoxides; Hydrolysis of steroid epoxides; Nitrile hydratase/nitrilase; Hydrolysis of aliphatic nitrites to carboxamides; Hydrolysis of aromatic, heterocyclic, unsaturated aliphatic nitrites to corresponding acids; Hydrolysis of acrylonitrile; Production of aromatic and carboxamides, carboxylic acids (nicotinamide, picolinamide, isonicotinamide); Regioselective hydrolysis of acrylic dinitrile (-amino acids from (-hydroxynitriles)); Transaminase; Transfer of amino groups into oxo-acids; Amidase/Acylase; Hydrolysis of amides, amidines, and other C--N bonds; and Non-natural amino acid resolution and synthesis. See, *e.g.,* U.S. Patent Application Publication No. 20030215798 A1.

## Genes associated with domestication

In certain embodiments, protoplasts are identified, isolated, and/or enriched for expressing (or overexpressing) one or more gene or gene products associated with domestication (see, *e.g.,* Doebley et al. Cell Volume 127, Issue 7, 29 December 2006, Pages 1309-1321). Non-limiting examples of genes associated with domestication include: tb1 (Maize), tga1(Maize) qSH1(Rice) Rc (Rice) sh4 (Rice) fw2.2 (Tomato) Q (Wheat) c1 (Maize) r1(Maize) sh2 (Maize) su1(Maize) y1 (Maize) brix9-2-5 (Tomato) Ovate (Tomato) Rin (Tomato) Sp (Tomato) R (Pea) ehd1 (Rice) gn1 (Rice) hd1(Rice) hd6 (Rice) sd1 (Rice) waxy (Rice) rht (Wheat) vrn1 (Wheat) vrn2 (Wheat) boCal (Cauliflower) ba1 (Maize) ra1 (Maize) su1 (Maize), bt2 (Maize), ae1 (Maize), and zagl1 (Maize). In certain embodiments, a plant or plant cell has downregulated expression of one or more genes associated with domestication.

**Genes with mutant phenotypes (Arabidopsis)**

In certain embodiments, protoplasts are identified, isolated, and/or enriched for expressing (or overexpressing) one or more gene or gene products with mutant phenotypes (see, *e.g.,* Meinke et al. Plant Physiol. 2003 February; 131(2): 409-418). Non-limiting examples of genes with mutant phenotypes include: AAO 3, AAT 2, AAT 3, ABA 1, ABA 2, ABA 3, ABC 1, ABF 1, ABH 1, ABI 1, ABI 2, ABI 3, ABI 4, ABI 5, ABP 1, ACD 1, ACD 11, ACD 2, ACE, ACL 5, ACT 7*, ADG 1, ADG 2, ADH 1, ADL 1A, AG, AGB 1, AGL 24, AGL 8, AGO 1, AGR 1, AHA 4, AIM, AKT 1, ALB 3, ALE 1, ALF 5, AML 1, AMP 1, AN, ANL 2, ANT, AOP 2, AOP 3, AOS, AP 1, AP 2, AP 3, APG 3*, APG 9*, APT 1, ARA 1, ARG 1, ARS 27, ARTEMIS, AS 1, AS 2, ASB 1, ASB 3, ASK 1, ASY 1, ATR, AUX 1, AXR 1, AXR 2, AXR 3, AXR 6, BAK 1, BAL, BAN, BDL, BEL 1, BIN 2, BIN 3, BIN 5, BIO 1, BIO 2, BON 1, BOU, BP, BRI 1, BRL 1, CAD 1, CAL, CAO, CBB 3, CCA 1, CER 1, CER 2, CER 3, CER 6, CEV 1, CH 1, CH 42, CHL 1, CHL 6, CHY 1, CIA 2, CKI 1, CLA, CLCA, CLF, CLV 1, CLV 2, CLV 3, CO, COB, COI 1, COP 1, COP 10, COP 8, COP 9 , CPC, CPR 5, CRC, CRE 1, CRM 2, CRP, CSR 1, CTR 1, CUC 1, CUC 2, CYT 1, DAD 1, DAG 1, DAG 2, DDM 1, DET 1, DET 2, DET 3, DEX 1, DFL 1, DGD 1, DHDPS 2, DMC 1, DME, DND 1, DPE 1, DRL 1, DWF 1, DWF 4, DWF 5, DWF 7, EDD, EDM 1, EDR 1, EDS 1, EDS 5, EIN 2, EIN 3, ELF 3, ELF 4, ELP, EMB 1006, EMB 1011, EMB 1025, EMB 1027, EMB 1047, EMB 1067, EMB 1075, EMB 1080, EMB 1129, EMB 1138, EMB 1187, EMB 1211, EMB 1220, EMB 1265, EMB 1270, EMB 1273, EMB 1276, EMB 1290, EMB 1345, EMB 1353, EMB 1354, EMB 1374, EMB 1381, EMB 1417, EMB 1427, EMB 1441, EMB 1444, EMB 1473, EMB 1474, EMB 1507, EMB 1513, EMB 1579, EMB 1586, EMB 1611, EMB 1629, EMB 1687, EMB 1688, EMB 1692, EMB 1703, EMB 1705, EMB 1738, EMB 1745, EMB 1789, EMB 1793, EMB 1796, EMB 1860, EMB 1865, EMB 1873, EMB 1879, EMB 1899, EMB 1956, EMB 1967, EMB 1974, EMB 1997, EMB 2024, EMB 2036, EMB 2107, EMB 2171, EMB 2184, EMB 2204, EMB 2207, EMB 2219, EMB 2220, EMB 2261, EMB 2284, EMB 2289, EMB 2296, EMB 2386, EMB 2444, EMB 2453, EMB 2454, EMB 2474, EMB 2719, EMB 2726, EMB 2728, EMB 2729, EMB 2730, EMB 30, EMB 506, EMF 1, EMF 2, EMS 1, ER, ERA 1, ETR 1, ETR 2, ETT, FAB 1, FAD 2, FAD 3, FAD 5, FAD 6, FAD 7, FAR 1, FAS 1, FAS 2, FCA, FD, FDH, FEY, FFC, FHA, FHY 2, FIE, FIL, FIN 219, FIP 37*, FIS 2, FK, FKF 1, FLC, FLP, FLS 2, FLU, FPA, FPF, FRA 2, FRC 3, FRD 3, FRI, FRO 1, FRY 1, FRY 2, FS 1, FT, FUS 11, FUS 12, FUS 3, FUS 5, FUS 6, FVE, FWA, FY, GA 1, GA 2, GA 3, GA 4, GA 5, GAI, GDH 1, GI, GL 1, GL 2, GL 3, GLS 1, GPA 1, GUN 5, HBT, HCF 136, HCF 164, HFR 1, HIC, HIK, HKT1;1 HLL, HLS 1, HO 2, HOS 1, HOT*, HUA 1, HUA 2, HY 1, HY 2, HY 4, HY 5, HYD 1, HYL 1, IAR 1, IAR 3, ILR 1, IM, INO, IRE*, IRT 1, IRX 1, IRX 3, IRX 4, IXR 1, IXR 2, KAK, KAN, KCS 1, KEU, KIS, KJK, KN, KNF, KOB 1, KOM, KOR 1, KU 70, KYP, LAF 1, LAF 6, LCR, LD, LEC 1, LEC 2, LFY, LOS 1, LOS 2, LRX 1, LSD 1, LSN, LSP 1, LUG, MAA 3, MAM 1*, MEA, MGD 1, MIM, MKP 1, MMP, MNP, MOD 1*, MOM, MOR 1, MP, MPK 4, MRP 5, MS 1, MS 2, MS 5, MTO 1, MTO 2, MTO 3, MUR 1, MUR 2, MYB 4, MYB 61, NDR 1, NIA 1, NIA 2, NIM 1, NIT 1, NPH 1, NPH 3, NPH 4, NPQ 1, NRT 2, OMR 1, OPR 3, ORE 4, ORE 9, OSM 1, PAC, PAD 3, PAD 4, PAN, PAS 1, PAT 1, PAT 3, PBS 1, PBS 2, PDE 120, PDE 129, PDE 135, PDE 149, PDE 166, PDE 181, PDE 191, PDE 194, PDE 225, PDE 226, PDE 247, PDE 277, PDE 312, PDF 2, PDS 1, PED 1, PED 2, PEN 2, PFC 1, PFI, PFL, PFL 2, PGI 1, PGM, PGP, PGR 1, PHO 1, PHYB, PHYC, PI, PID, PIN 1, PIN 3, PIN 4, PKL, POC 1, POR, PPI 1, PPI 2, PPT, PRF 1, PRL, PRPL 11, PRS , PRT 1, PRZ 1, PSAE 1, PSBO, PTF 1, PXA 1, QUA 1, RAD 17*, RAD 50*, RAM 1, RAN 1, RAT 5, RCN 1, REF 8, REV, RGL 1, RHA 1, RHD 3, RHL 1, RML 1, ROT 3, RPM 1, RPP 1, RPP 13, RPP 4, RPP 8, RPS 2, RPS 4, RPS 5, RPT 2, RSH, RSW 1, RSW 3, RSY 3, RTM 1, RTM 2, SAB, SAP, SCD 1, SCR, SDD 1, SDS, SE, SEL 1, SEU, SEX 1, SGR 2, SGR 3, SGS 2, SGS 3, SHD, SHI, SHR, SHY 2, SID 2, SKO, SKU 5, SLP, SLR, SMT 1, SNG 1, SNG 2, SNI 1, SON 1, SOS 1, SOS 2, SOS 3, SOS 4, SOZ 2, SP1L, SPA 1, SPIK, SPIKE 1, SPL, SPR 1*, SPS, SPT, SPY, SQD 1, SQD 2, SQN, SRL 2, SSE, SSI 2, SSR 16, STARIK, STI, STM, SUB 1*, SUC 2, SUP, SUR 1, SUR 2, SUS 1, SUS 2, SVP, SWI 1, SYD, SYN 1, TAG 1, TASTY, TED 3, TEJ, TES, TFL 1, TFL 2, TH 1, TIR 1, TIR 3, TMM, TMT 1, TNY, TOC 1*, TOM 1, TOR*, TPS*, TRH 1, TRP 1, TRP 2, TRP 3, TRP 4, TRP 5, TSK, TSL, TSO 1, TT 1, TT 12, TT 2, TT 3, TT 4, TT 5, TT 6, TT 7, TT 8, TTG 1, TTG 2, TTN 1, TTN 3, TTN 4, TTN 5, TTN 6, TTN 7, TTN 8, TTN 9, TWN 2, TZ, UCU 2, UFO, UVH 1, UVH 3, UVR 2, UVR 3, UVR 8, VAR 1, VAR 2, VCL 1, VEP 1, VIP 4, VRN 1, VRN 2, WER 1, WUS, XPB 1, XYL 1, YDA, YUC, ZIG, ZLL, ZTL, and ZWI.

### 5.2.3. *Identification and Selection*

The methods described herein for identifying and selecting protoplasts expressing, or has upregulated expression of, at least one gene product, *e.g.*, RNA or polypeptide of interest, involve the use of fluorogenic oligonucleotide probes or molecular beacon probes. In particular embodiments, such methods comprise (a) introducing into a plant cell one or more fluorogenic oligonucleotide probes capable of detecting RNA transcripts of at least one gene of interest (*e.g.*, fluorogenic oligonucleotide probes capable of hybridizing to one or more RNA transcript of a gene of interest), wherein the fluorogenic oligonucleotide is transferred into the plant cell by using polyethylene glycol (PEG), and (b) determining whether the RNA of interest is present in the plant cell, wherein the plant cell is a protoplast. Such methods also include quantifying the level of the RNA of interest, and/or determining a gene expression profile of the plant cell. Such gene expression profile can be compared with gene expression profiles of a reference population. Plant cells containing the RNA of interest or the desired gene expression profile as compared to that of a reference population can be selected or isolated, and are used for generation of whole plants. Whole plants may be used to generate identical clones and/or homogeneous populations of plants generated from the selected plant cells.

Selected cells or plants may be tested in functional assays for validation that the cells or plants possess the function or phenotype selected for. Such functional assays are known to a skilled person in the art. For example, RNA or protein expression may be confirmed by RT-PCR, real time PCR, immunoblotting, flow cytometry, or Enzyme-linked immunosorbent assay (ELISA). Selected plants with a desired trait for sustaining and/or growing under certain conditions may be tested by exposing the selected plants to such conditions and comparing the growth with a control (*e.g.*, unselected plant). Selected plants that have phenotypes visually observable may be validated by visual inspection with the naked eye or under a microscope.

The plant sections below provide further details and description for carrying out the methods described herein.

### 5.2.3.1 *Delivery of Nucleic Acid Molecules*

Any method or delivery system may be used for the introduction, delivery and/or transfection of nucleic acids, such as fluorogenic oligonucleotide probes or transgene vectors, into plant cells and plants. Such methods are known in the art, and one skilled in the art could readily select the appropriate method that is suitable for a particular plant cell or plant species. In the methods of the invention as defined in the claims appended to this description, a fluorogenic oligonucleotide is transferred into a protoplast by using polyethylene glycol (PEG).

For introduction of fluorogenic oligonucleotide probes, a transient delivery system is preferred. For introduction of transgene vectors, in certain embodiments, a delivery system that allows for stable integration of the transgene into the plant genome may be preferred. In certain embodiments, transient delivery systems may also allow for stable integration of the transgene into the genome. In other embodiments, transient expression of the transgene may be preferred.

Transfection may be accomplished by a wide variety of means, as is known to those of ordinary skill in the art. Such methods include, but are not limited to, Agrobacterium-mediated transformation *(e.g.,* Komari et al., 1998, Curr. Opin. Plant Biol., 1:161), particle bombardment mediated transformation *(e.g.,* Finer et al., 1999, Curr. Top. Microbiol. Immunol., 240:59), protoplast electroporation *(e.g.*, Bates, 1999, Methods Mol. Biol., 111:359), viral infection *(e.g.*, Porta and Lomonossoff, 1996, Mol. Biotechnol. 5:209), microinjection, and liposome injection. Other exemplary delivery systems that can be used to facilitate uptake by a cell of a nucleic acid molecule include calcium phosphate and other chemical mediators of intracellular transport, microinjection compositions, and homologous recombination compositions (*e.g.*, for integrating a gene into a preselected location within the chromosome of the cell). Alternative methods may involve, for example, the use of liposomes, electroporation, or chemicals that increase free (or "naked") DNA uptake, transformation using viruses or pollen and the use of microprojection. Standard molecular biology techniques are common in the art (*e.g.,* Sambrook et al., 1989, Molecular Cloning: A Laboratory Manual, 2nd ed., Cold Spring Harbor Laboratory Press, New York).

For introduction of transgene vectors, one of skill in the art will be able to select an appropriate vector for introducing a nucleic acid construct in a relatively intact state. Thus, any vector which will produce a plant carrying the introduced nucleic acid construct should be sufficient. The selection of the vector, or whether to use a vector, is typically guided by the method of transformation selected.

The transformation of plants in accordance with the methods described herein may be carried out in essentially any of the various ways known to those skilled in the art of plant molecular biology. (See, for example, Methods of Enzymology, Vol. 153, 1987, Wu and Grossman, Eds., Academic Press).

Plant cells and plants can comprise one or more nucleic acid molecules or nucleic acid constructs. For producing plants or plants cells comprising transgenes, any means for producing a plant comprising the nucleotide sequence constructs described herein are encompassed by the present disclosure. For example, in certain aspects, a nucleotide sequence encoding a transgene can be used to transform a plant at the same time as the fluorogenic oligonucleotide sequences. For producing plants or plants cells comprising transgenes, viral vectors may be used to express gene products by various methods generally known in the art. Suitable plant viral vectors for expressing genes should be self-replicating, capable of systemic infection in a host, and stable. Additionally, the viruses should be capable of containing the nucleic acid sequences that are foreign to the native virus forming the vector. Transient expression systems may also be used.

### 5.2.3.1.1 **Mechanical and Chemical Means**

In some aspects, nucleic acid molecules, such as fluorogenic oligonucleotide probes, are introduced into a plant cell using mechanical or chemical means. Exemplary mechanical and chemical means are provided below. In certain aspects, nucleic acid constructs, *e.g.*, constructs comprising a transgene, may be introduced into a plant cell using mechanical or chemical means.

## Microinjection

In some aspects, nucleic acid molecules (*e.g.*, fluorogenic oligonucleotide probes or transgene vectors) can be mechanically transferred into a plant cell by microinjection using a micropipette. See, *e.g.,* WO 92/09696; WO 94/00583; EP 331083; EP 175966; Green et al., 1987, Plant Tissue and Cell Culture, Academic Press; and Crossway et al., 1986, Biotechniques 4:320-334.

## PEG

In the methods of the present invention, nucleic acid molecules (*e.g.*, fluorogenic oligonucleotide probes or transgene vectors) are transferred into a plant cell by using polyethylene glycol (PEG) which forms a precipitation complex with genetic material that is taken up by the cell.

## Electroporation

In certain aspects, Electroporation can be used to deliver nucleic acid molecules (*e.g.,* fluorogenic oligonucleotide probes or transgene vectors) to plant cells (see, *e.g.,* Fromm *et al.,* 1985, PNAS, 82:5824). "Electroporation," as used herein, is the application of electricity to a cell, such as a plant protoplast, in such a way as to cause delivery of a nucleic acid molecule into the cell without killing the cell. Typically, electroporation includes the application of one or more electrical voltage "pulses" having relatively short durations (usually less than 1 second, and often on the scale of milliseconds or microseconds) to a media containing the cells. The electrical pulses typically facilitate the non-lethal transport of extracellular nucleic acid molecules into the cells. The exact electroporation protocols (such as the number of pulses, duration of pulses, pulse waveforms, *etc.*), will depend on factors such as the cell type, the cell media, the number of cells, the substance(s) to be delivered, *etc.,* and can be determined by those of ordinary skill in the art. Electroporation is discussed in greater detail in, *e.g.,* EP 290395; WO 8706614; Riggs et al., 1986, Proc. Natl. Acad. Sci. USA 83:5602-5606; and D'Halluin et al., 1992, Plant Cell 4:1495-1505). Other forms of direct DNA uptake can also be used in the methods provided herein, such as those discussed in, *e.g.,* DE 4005152; WO 9012096; U.S. Patent No. 4,684,611; and Paszkowski et al., 1984, EMBO J. 3:2717-2722.

## Ballistic and Particle Bombardment

Another method for introducing nucleic acid molecules (*e.g.*, fluorogenic oligonucleotide probes and transgene vectors) into a plant cell is high velocity ballistic penetration by small particles with the nucleic acid molecules to be introduced contained either within the matrix of such particles, or on the surface thereof (Klein et al., 1987, Nature 327:70). Nucleic acid molecules (*e.g.*, fluorogenic oligonucleotide probes) can be introduced into a cell using particle gun ("gene gun") technology, also called microprojectile or microparticle bombardment. In this method, small, high-density particles (microprojectiles) are accelerated to high velocity in conjunction with a larger, powder-fired macroprojectile in a particle gun apparatus. The microprojectiles have sufficient momentum to penetrate cell walls and membranes, and can carry RNA or other nucleic acids into the interiors of bombarded cells. It has been demonstrated that such microprojectiles can enter cells without causing death of the cells, and that they can effectively deliver foreign genetic material into intact tissue. Bombardment transformation methods have been described, see, *e.g.*, Sanford et al., Techniques 3:3-16, 1991, and Klein et al., Bio/Techniques 10:286, 1992. Although, typically only a single introduction of a new nucleic acid molecule(s) is required, this method particularly provides for multiple introductions.

Particle or microprojectile bombardment are discussed in greater detail in, *e.g.,* the following references: U.S. Patent No. 5,100,792; EP-A-4448821 EP-A-434616; Sanford et al., U.S. Pat. No. 4,945,050; Tomes et al., 1995, "Direct DNA Transfer into Intact Plant Cells via Microprojectile Bombardment," in Plant Cell, Tissue, and Organ Culture: Fundamental Methods, ed. Gamborg and Phillips (Springer-Verlag, Berlin); and McCabe et al., 1988, Biotechnology 6:923-926.

In specific aspects, nucleic acid molecules (*e.g.*, fluorogenic oligonucleotide probes or transgene vectors) are delivered to the scutellum cells of freshly dissected immature embryos of a crop, *e.g.*, wheat. Nucleic acid molecules are co-precipitated with $CaCl_2$ and spermidine onto gold particles (Bio-Rad) that are then washed once in 100% ethanol, and then resuspended in 100% ethanol. An aliquot of the suspended gold particles are bombarded using a bio-Rad Biolistic® PDS-1000/He delivery system to the freshly isolated wheat immature embryos arranged on the center of 6 cm Petri dish in a 1 cm circle. The bombardment is performed at 1100 psi, 27 mmHg vacuum and with the embryos at a 6 cm target distance. See, *e.g.,* Dong et al., Plant Cell Rep., 2006, 25:457-465.

**Colloidal Dispersion**

In other aspects, a colloidal dispersion system may be used to facilitate delivery of a nucleic acid molecules (*e.g.*, fluorogenic oligonucleotide probes or transgene vectors) into the cell. As used herein, a "colloidal dispersion system" refers to a natural or synthetic molecule, other than those derived from bacteriological or viral sources, capable of delivering to and releasing the nucleic acid to the cell. Colloidal dispersion systems include, but are not limited to, macromolecular complexes, beads, and lipid-based systems including oil-in-water emulsions, micelles, mixed micelles, and liposomes. One example of a colloidal dispersion system is a liposome. Liposomes are artificial membrane vessels. It has been shown that large unilamellar vessels ("LUV"), which-range in size from 0.2 to 4.0 microns, can encapsulate large macromolecules within the aqueous interior and these macromolecules can be delivered to cells in a biologically active form *(e.g.,* Fraley et al., 1981, Trends Biochem. Sci., 6:77).

**Lipids**

Lipid formulations for the transfection and/or intracellular delivery of nucleic acid molecules (*e.g.*, fluorogenic oligonucleotide probes or transgene vectors) are commercially available, for instance, from QIAGEN, for example as EFFECTENE® (a non-liposomal lipid with a special DNA condensing enhancer) and SUPER-FECT® (a novel acting dendrimeric technology) as well as Gibco BRL, for example, as LIPOFECTIN® and LIPOFECTACE®, which are formed of cationic lipids such as N-[1-(2,3-dioleyloxy)-propyl]-N,N,N-trimethylammonium chloride ("DOTMA") and dimethyl dioctadecylammonium bromide ("DDAB"). Liposomes are well known in the art and have been widely described in the literature, for example, in Gregoriadis, G., 1985, Trends in Biotechnology 3:235-241; and Freeman et al., 1984, Plant Cell Physiol. 29:1353).

**Other Methods**

In addition to the above, other physical methods for the transformation of plant cells are reviewed in the following and can be used in the methods provided herein. Oard , 1991, Biotech. Adv. 9:1-11. See generally, Weissinger et al., 1988, sAnn. Rev. Genet. 22:421-477; Sanford et al., 1987, Particulate Science and Technology 5:27-37; Christou et al., 1988, Plant Physiol. 87:671-674; McCabe et al., 1988, Bio/Technology 6:923-926; Finer and McMullen, 1991, In vitro Cell Dev. Biol. 27P:175-182; Singh et al., 1998, Theor. Appl. Genet. 96:319-324; Datta et al., 1990, Biotechnology 8:736-740; Klein et al., 1988, Proc. Natl. Acad. Sci. USA 85:4305-4309; Klein et al., 1988, Biotechnology 6:559-563; Tomes, U.S. Pat. No. 5,240,855; Buising et al., U.S. Pat. Nos. 5,322,783 and 5,324,646; Klein et al., 1988, Plant Physiol. 91:440-444; Fromm et al., 1990, Biotechnology 8:833-839; Hooykaas-Van Slogteren et al., 1984, Nature (London) 311:763-764; Bytebier et al., 1987, Proc. Natl. Acad. Sci. USA 84:5345-5349; De W et al., 1985, The Experimental Manipulation of Ovule Tissues, ed. Chapman et al. (Longman, N.Y.), pp. 197-209; Kaeppler et al., 1990, Plant Cell Reports 9:415-418 and Kaeppler et al., 1992, Theor. Appl. Genet. 84:560-566; Li et al., 1993, Plant Cell Reports 12:250-255 and Christou and Ford, 1995, Annals of Botany 75:407-413; Osjoda et al., 1996, Nature Biotechnology 14:745-750.

5.2.3.1.2 **Agrobacterium**

For producing plants or plants cells comprising transgenes, *Agrobacterium* transformation is widely used by those skilled in the art to transform dicotyledonous species. There has been substantial progress towards the routine production of stable, fertile transgenic plants in almost all economically relevant monocot plants (see, *e.g.,* Toriyarna et al., 1988, Bio/Technology 6:1072-1074; Zhang et al., 1988, Plant Cell Rep. 7:379-384; Zhang et al., 1988, Theor. Appl. Genet. 76:835-840; Shimamoto et al., 1989, Nature 338:274-276; Datta et al., 1990, Bio/Technology 8: 736-740; Christou et al., 1991, Bio/Technology 9:957-962; Peng et al., 1991, International Rice Research Institute, Manila, Philippines, pp. 563-574; Cao et al., 1992, Plant Cell Rep. 11:585-591; Li et al., 1993, Plant Cell Rep. 12:250-255; Rathore et al., 1993, Plant Mol. Biol. 21:871-884; Fromm et al., 1990, Bio/Technology 8:833-839; Tomes et al., 1995, "Direct DNA Transfer into Intact Plant Cells via Microprojectile Bombardment," in Plant Cell, Tissue, and Organ Culture: Fundamental Methods, ed. Gamborg and Phillips (Springer-Verlag, Berlin); D'Halluin et al., 1992, Plant Cell 4:1495-1505; Walters et al., 1992, Plant Mol. Biol. 18:189-200; Koziel et al., 1993, Biotechnology 11: 194-200; Vasil, I. K., 1994, Plant Mol. Biol. 25:925-937; Weeks et al., 1993, Plant Physiol. 102:1077-1084; Somers et al., 1992, Bio/Technology 10: 1589-1594; WO 92/14828). In particular, *Agrobacterium* mediated transformation can be a highly efficient transformation method in monocots (Hiei et al., 1994, The Plant Journal 6:271-282). See also, Shimamoto, K., 1994, Current Opinion in Biotechnology 5:158-162; Vasil et al., 1992, Bio/Technology 10:667-674; Vain et al., 1995, Biotechnology Advances 13(4):653-671; Vasil et al., 1996, Nature Biotechnology 14:702).

The particular choice of a transformation technology will be determined by its efficiency to transform certain plant species as well as the experience and preference of the person practicing the disclosure with a particular methodology of choice. It will be apparent to the skilled person that the particular choice of a transformation system to introduce nucleic acid into plant cells is not essential to or a limitation of the disclosure, nor is the choice of technique for plant regeneration.

A transgene can be introduced into plant cells using Ti plasmids of *Agrobacterium tumefaciens* (*A. tumefaciens*), inducible plasmids of *Agrobacterium rhizogenes (A. rhizogenes)*, or plant virus vectors. For reviews of such techniques see, for example, Weissbach & Weissbach, 1988, Methods for Plant Molecular Biology, Academic Press, NY, Section VIII, pp. 421-463; and Grierson & Corey, 1988, Plant Molecular Biology, 2d Ed., Blackie, London, Ch. 7-9, and Horsch et al., 1985, Science, 227:1229.

In using an *A. tumefaciens* culture as a transformation vehicle, it may be advantageous to use a non-oncogenic strain of *Agrobacterium* as the vector carrier so that normal non-oncogenic differentiation of the transformed tissues is possible. It is also preferred that the *Agrobacterium* harbor a binary Ti plasmid system. Such a binary system comprises 1) a first Ti plasmid having a virulence region essential for the introduction of transfer DNA (T-DNA) into plants, and 2) a chimeric plasmid. The chimeric plasmid contains at least one border region of the T-DNA region of a wild-type Ti plasmid flanking the nucleic acid to be transferred. Binary Ti plasmid systems have been shown to be effective in the transformation of plant cells (De Framond, Biotechnology, 1983, 1:262; Hoekema et al., 1983, Nature, 303:179). Such a binary system is preferred because it does not require integration into the Ti plasmid of *A. tumefaciens,* which is an older methodology.

In some aspects, a disarmed Ti-plasmid vector carried by *Agrobacterium* exploits its natural gene transferability (EP-A-270355, EP-A-01 16718, Townsend et al., 1984, NAR, 12:8711, U.S. Pat. No. 5,563,055).

Methods involving the use of *Agrobacterium* in transformation according to the present disclosure include, but are not limited to: 1) co-cultivation of *Agrobacterium* with cultured isolated protoplasts; 2) transformation of plant cells or tissues with *Agrobacterium*; or 3) transformation of seeds, apices or meristems with *Agrobacterium.*

In addition, gene transfer can be accomplished by in planta transformation by *Agrobacterium,* as described by Bechtold et al., C.R. Acad. Sci. Paris, 1993, 316:1194; and Bent, A.F., Plant Physiol., 2000, 124:1540-1547. This approach is based on the vacuum infiltration of a suspension of *Agrobacterium* cells.

In certain aspects, a nucleic acid construct comprising transgene is introduced into plant cells by infecting such plant cells, an explant, a meristem or a seed, with transformed A. *tumefaciens* as described above. Under appropriate conditions known in the art, the transformed plant cells are grown to form shoots, roots, and develop further into plants.

Other methods described herein, such as microprojectile bombardment, electroporation and direct DNA uptake can be used where *Agrobacterium* is inefficient or ineffective. Alternatively, a combination of different techniques may be employed to enhance the efficiency of the transformation process, *e.g.,* bombardment with *Agrobacterium*-coated microparticles (EP-A-486234) or microprojectile bombardment to induce wounding followed by co-cultivation with *Agrobacterium* (EP-A-486233).

5.2.3.1.3 **CaMV**

In some aspects, cauliflower mosaic virus (CaMV) is used as a vector for introducing transgenes into plant cells (see, *e.g.,* U.S. Patent No. 4,407,956). CaMV viral DNA genome can be inserted into a parent bacterial plasmid creating a recombinant DNA molecule which can be propagated in bacteria. After cloning, the recombinant plasmid again can be cloned and further modified by introduction of the desired nucleic acid sequence. The modified viral portion of the recombinant plasmid can then be excised from the parent bacterial plasmid, and used to inoculate the plant cells or plants.

5.2.3.2 *Plant Generation*

Following identification and selection of desired plants cells (*e.g.,* plant cells containing transcripts of one or more genes of interest in accordance with the methods described herein or plant cells engineered to express one or more transgenes), a plant may be regenerated, *e.g.,* from single cells, callus tissue or leaf discs, as is standard in the art. Almost any plant can be entirely regenerated from cells, tissues, and organs of the plant. Available techniques are reviewed in Vasil et al., 1984, in Cell Culture and Somatic Cell Genetics of Plants, Vols. I, II, and III, Laboratory Procedures and Their Applications (Academic Press); and Weissbach and Weissbach, Methods for Plant Molecular Biology, Academic Press, 1989.

The plants obtained by regeneration from selected plant cells may then be grown, and either pollinated with the same selected strain or different strains, and the resulting hybrid having expression of the desired phenotypic characteristic identified. Two or more generations may be grown to ensure that expression of the desired phenotypic characteristic is stably maintained and inherited and then seeds harvested to ensure expression of the desired phenotypic characteristic has been achieved. In preferred embodiments, plants obtained by regeneration from selected plant cells may then be grown, and pollinated with the same selected strain.

Normally, a plant cell is regenerated to obtain a whole plant. The term "growing" or "regeneration" as used herein means growing a whole plant from a plant cell, a group of plant cells, a plant part (including seeds), or a plant piece (*e.g.,* from a protoplast, callus, or tissue part).

The regeneration of plants from either single plant protoplasts or various explants is well known in the art. See, for example, Methods for Plant Molecular Biology, A. Weissbach and H. Weissbach, eds., 1988, Academic Press, Inc., San Diego, Calf. This regeneration and growth process includes the steps of selection of transformant cells and shoots, rooting the transformant shoots and growth of the plantlets in soil. For maize cell culture and regeneration see generally, The Maize Handbook, Freeling and Walbot, Eds., 1994, Springer, New York 1994; Corn and Corn Improvement, 3rd edition, Sprague and Dudley Eds., 1988, American Society of Agronomy, Madison, Wis.

Regeneration from protoplasts varies from species to species of plants, but generally a suspension of protoplasts is first made. In certain species, embryo formation can then be induced from the protoplast suspension. The culture media will generally contain various amino acids and hormones, necessary for growth and regeneration. Examples of hormones utilized include auxins and cytokinins. Efficient regeneration will depend on the medium, on the genotype, and on the history of the culture. If these variables are controlled, regeneration is reproducible.

For example, plant regeneration from maize (Zea mays L.) protoplasts with recovery of fertile plants have been described, see, *e.g.,* Prioli et al., Bio/Technology, 7:589 - 594 (1989). Protoplasts can be isolated from embryogenic cell suspension cultures capable of regenerating fertile plants. Cell colonies and embryogenic calli can be obtained at high frequencies when protoplasts cultured either in a thin layer of liquid medium or on cellulose-nitrate filters placed on a feeder layer of maize cells. High plating efficiencies can be obtained with culture methods using either a modified KM-8p or a modified N6 culture media.

Regeneration also occurs from plant callus, explants, organs or parts. Transformation can be performed in the context of organ or plant part regeneration (see Methods in Enzymology, Vol. 118 and Klee et al., Annual Review of Plant Physiology, 38:467, 1987). Utilizing the leaf disk-transformation-regeneration method of Horsch et al., Science, 227:1229, 1985, leaf disc (*e.g.,* leaf disc of plants regenerated from plant cells selected using fluorogenic oligonucleotide probes) are cultured so that shoot regeneration can occur, *e.g.*, within 2 to 4 weeks. For introduction of transgenes, surface-sterilized leaf disks are inoculated with an *Agrobacterium tumefaciens* strain containing a transgene construct and cultured for 2 days, and the leaf disks are then transferred to selection medium. Shoot regeneration occurs within 2 to 4 weeks, and transformants are confirmed by their ability to form roots in selection medium. This method for producing transformed plants combines gene transfer, plant regeneration, and effective selection for transformants into a single process and should be applicable to plant species that can be infected by Agrobacterium and regenerated from leaf explants. Rooted plantlets are transplanted to soil as soon as possible after roots appear. The plantlets can be repotted as required, until reaching maturity.

In vegetatively propagated crops, the mature selected plants (e.g., transgenic plants or plants selected using fluorogenic oligonucleotide probes) are propagated by utilizing cuttings or tissue culture techniques to produce multiple identical plants. Selection of desirable plants is made and new varieties are obtained and propagated vegetatively for commercial use.

In seed propagated crops, mature selected plants (*e.g.*, transgenic plants or plants regenerated from plant cells selected using fluorogenic oligonucleotide probes) can be self crossed to produce a homozygous inbred plant. The resulting inbred plant produces seed with the selected or desired characteristics, *e.g.*, seed with the desired or selected expression profile or seeds containing the newly introduced foreign gene(s) (*i.e.,* transgene). These seeds can be grown to produce plants that would produce the selected phenotype.

Parts obtained from a regenerated plant, such as flowers, seeds, leaves, branches, fruit, and the like are included in the disclosure, provided that these parts comprise cells comprising the selected expression profile or the introduced transgene. Progeny and variants, and mutants of the regenerated plants are also included within the scope of the disclosure, provided that these parts comprise the selected expression profile or the introduced transgene. Selected plants expressing the gene or genes of interest can be screened for transmission of the desired expression profile by, for example, standard immunoblot and DNA detection techniques. Selected lines can also be evaluated on levels of expression of the gene or genes of interest. Expression at the RNA level can be determined initially to identify and quantitate expression-positive plants. Standard techniques for RNA analysis can be employed and include PCR amplification assays using oligonucleotide primers designed to amplify only the heterologous RNA templates and solution hybridization assays using heterologous nucleic acid-specific probes. The RNA-positive plants can then be analyzed for protein expression by Western immunoblot analysis using the appropriate reactive antibodies. In addition, in situ hybridization and immunocytochemistry according to standard protocols can be done using heterologous nucleic acid specific polynucleotide probes and antibodies, respectively, to localize sites of expression within transgenic tissue. Generally, a number of selected lines are usually screened for expression of the selected expression profile or the introduced transgene to identify and select plants with the most appropriate expression profiles or desired characteristics.

For plants expressing one or more transgenes, a preferred aspect is a transgenic plant that is homozygous for the added heterologous nucleic acid; *i.e.,* a transgenic plant that contains two added nucleic acid sequences encoding the transgene, one gene at the same locus on each chromosome of a chromosome pair. A homozygous transgenic plant can be obtained by sexually mating (selfing) a heterozygous transgenic plant that contains a single added heterologous nucleic acid, germinating some of the seed produced and analyzing the resulting plants produced for altered expression of a polynucleotide of the present disclosure relative to a control plant (*i.e.,* native, non-transgenic). Backcrossing to a parental plant and out-crossing with a non-transgenic plant are also contemplated.

Selected plant cells which are derived by the methods described herein can be cultured to regenerate a whole plant which expresses the desired expression profile or possesses the desired improved traits. Such regeneration techniques often rely on manipulation of certain phytohormones in a tissue culture growth medium. For transformation and regeneration of maize see, Gordon-Kamm et al., 1990, The Plant Cell, 2:603-618.

Plants cells transformed with a plant expression vector can be regenerated, *e.g.*, from single cells, callus tissue or leaf discs according to standard plant tissue culture techniques. It is well known in the art that various cells, tissues, and organs from almost any plant can be successfully cultured to regenerate an entire plant. Plant regeneration from cultured protoplasts is described in Evans et al., 1983, Protoplasts Isolation and Culture, Handbook of Plant Cell Culture, Macmillan Publishing Company, New York, pp.124-176; and Binding, Regeneration of Plants, Plant Protoplasts, 1985, CRC Press, Boca Raton, pp. 21-73.

### 5.2.3.3 *Cultivation*

The plants described herein may be cultivated under any suitable conditions. One skilled in the art would know how to cultivate plants under appropriate conditions. For example, one skilled in the art knows what constitute nitrogen-poor and nitrogen-rich growth conditions for the cultivation of most, if not all, important crop and ornamental plants.

For the cultivation of wheat see Alcoz et al., 1993, Agronomy Journal 85:1198-1203; Rao and Dao, 1992, J. Am. Soc. Agronomy 84:1028-1032; Howard and Lessman, 1991, Agronomy Journal 83:208-211; for the cultivation of corn see Tollenear et al., 1993, Agronomy Journal 85:251-255; Straw et al., Tennessee Farm and Home Science: Progress Report, Spring 1993, 166:20-24; Miles, S. R., 1934, J. Am. Soc. Agronomy 26:129-137; Dara et al., 1992, J. Am. Soc. Agronomy 84:1006-1010; Binford et al., 1992, Agronomy Journal 84:53-59; for the cultivation of soybean see Chen et al., 1992, Canadian Journal of Plant Science 72:1049-1056; and Wallace et al., 1990, Journal of Plant Nutrition 13:1523-1537.

For the cultivation of rice see Oritani and Yoshida, 1984, Japanese Journal of Crop Science 53:204-212; for the cultivation of linseed see Diepenbrock and Porksen, 1992, Industrial Crops and Products 1:165-173; for the cultivation of tomato see Grubinger et al., 1993, Journal of the American Society for Horticultural Science 118:212-216; Cerne, M., 1990, Acta Horticulture 277:179-182.

For the cultivation of pineapple see Magistad et al., 1932, J. Am. Soc. Agronomy 24:610-622; Asoegwu, S. N., 1988, Fertilizer Research 15:203-210; Asoegwu, S. N., 1987, Fruits 42:505-509; for the cultivation of lettuce see Richardson and Hardgrave, 1992, Journal of the Science of Food and Agriculture 59:345-349; for the cultivation of mint see Munsi, P. S., 1992, Acta Horticulturae 306:436-443.

For the cultivation of camomile see Letchamo, W., 1992, Acta Horticulturae 306:375-384; for the cultivation of tobacco see Sisson et al., 1991, Crop Science 31:1615-1620; for the cultivation of potato see Porter and Sisson, 1991, American Potato Journal, 68:493-505.

For the cultivation of brassica crops see Rahn et al., 1992, Conference "Proceedings, second congress of the European Society for Agronomy"Warwick Univ., p.424-425.

For the cultivation of banana see, *e.g.,* Hegde and Srinivas, 1991, Tropical Agriculture 68:331-334; Langenegger and Smith, 1988, Fruits 43:639-643; for the cultivation of strawberries see Human and Kotze, 1990, Communications in Soil Science and Plant Analysis 21:771-782.

For the cultivation of songhum see, *e.g.,* Mahalle and Seth, 1989, Indian Journal of Agricultural Sciences 59:395-397; for the cultivation of plantain see Anjorin and Obigbesan, 1985, Conference "International Cooperation for Effective Plantain and Banana Research" Proceedings of the third meeting. Abidjan, Ivory Coast, p. 115-117.

For the cultivation of sugar cane see Yadav, R. L., 1986, Fertiliser News 31:17-22; Yadav and Sharma, 1983, Indian Journal of Agricultural Sciences 53:38-43.

For the cultivation of sugar beet see Draycott et al., 1983, Conference "Symposium Nitrogen and Sugar Beet" International Institute for Sugar Beet Research-Brussels Belgium, p. 293-303. See also Goh and Haynes, 1986, "Nitrogen and Agronomic Practice" in Mineral Nitrogen in the Plant-Soil System, Academic Press, Inc., Orlando, Fla., p. 379-468; Engelstad, O. P., 1985, Fertilizer Technology and Use, Third Edition, Soil Science Society of America, p.633; Yadav and Sharmna, 1983, Indian Journal of Agricultural Sciences, 53:3-43.

5.2.3.4 *Products derived from improved plants*

Selected plants generated by the methods described herein can be used for producing commercial products. In specific aspects, selected plants generated by the methods described herein can be used directly in agricultural production.

Thus, the disclosure provides for products derived from the selected plants identified and/or isolated by the methods described herein. In certain aspects, the products are commercial products. Some non-limiting examples include trees, unmodified or genetically engineered trees, for *e.g.,* the production of pulp, paper, paper products or lumber; tobacco, *e.g.,* for the production of cigarettes, cigars, or chewing tobacco; crops, *e.g.,* for the production of tea, wine, fruits, vegetables and other food, including grains, *e.g.,* for the production of wheat, bread, flour, rice, corn; and canola, sunflower, *e.g.,* for the production of oils.

In certain aspects, commercial products are derived from a selected plant belonging to the species of woody, ornamental or decorative, crop or cereal, fruit or vegetable plant, and algae (*e.g., Chlamydomonas reinhardtii),* which may be used in the compositions and methods provided herein. Non-limiting examples of plants include plants from the genus *Arabidopsis* or the genus *Oryza.* Other examples include plants described herein in section 5.2.1.

In some aspects, commercial products are derived from a selected plant gymnosperms and angiosperms, both monocotyledons and dicotyledons. Examples of monocotyledonous angiosperms include, but are not limited to, asparagus, field and sweet corn, barley, wheat, rice, sorghum, onion, pearl millet, rye and oats and other cereal grains. Examples of dicotyledonous angiosperms include, but are not limited to tomato, tobacco, cotton, rapeseed, field beans, soybeans, peppers, lettuce, peas, alfalfa, clover, cole crops or Brassica oleracea (*e.g.*, cabbage, broccoli, cauliflower, brussel sprouts), radish, carrot, beets, eggplant, spinach, cucumber, squash, melons, cantaloupe, sunflowers and various ornamentals.

In certain aspects, commercial products are derived from a selected plant of the woody species, such as poplar, pine, sequoia, cedar, oak, maple *etc.*

In certain aspects, commercial products are derived from hardwood trees, such as oak, balsa, yew, eucalyptus, aspen, birch, maple, cherry blossom tree, beech, ash, holly, boxwood, teak, mahogany, ebony, lauan, and cedar. In certain aspects, commercial products are derived from softwood trees, which are coniferous, and may include hemlocks, fir, pine and spruce.

In other aspects, commercial products are derived from plants including, but are not limited to, wheat, cauliflower, tomato, tobacco, corn, petunia, trees, *etc.*

In certain aspects, commercial products are derived from selected plants that are crop plants, for example, cereals and pulses, maize, wheat, potatoes, tapioca, rice, sorghum, millet, cassaya, barley, pea, and other root, tuber, or seed crops. In one aspect, commercial products are derived from selected plants that are cereal crops, including, but are not limited to, any species of grass, or grain plant (*e.g.*, barley, corn, oats, rice, wild rice, rye, wheat, millet, sorghum, triticale, *etc.*), non-grass plants (*e.g.,* buckwheat flax, legumes or soybeans, *etc.*). In another aspect, commercial products are derived from selected plants that are grain plants that provide seeds of interest, oil-seed plants and leguminous plants. In other aspects, commercial products are derived from a selected plants that are grain seed plants, such as corn, wheat, barley, rice, sorghum, rye, *etc.* In yet other aspects, commercial products are derived from selected oil seed plants, such as cotton, soybean, safflower, sunflower, Brassica, maize, alfalfa, palm, coconut, *etc.* In certain aspects, commercial products are derived from selected plants that are oil-seed rape, sugar beet, maize, sunflower, soybean, or sorghum. In some aspects, commercial products are derived from selected plants that are leguminous plants, such as beans and peas (*e.g.*, guar, locust bean, fenugreek, soybean, garden beans, cowpea, mungbean, lima bean, fava bean, lentils, chickpea, *etc.*)

In certain aspects, commercial products are derived from a selected plants that are horticultural plant, such as lettuce, endive, and vegetable brassicas including cabbage, broccoli, and cauliflower, and carnations and geraniums; tomato, tobacco, cucurbits, carrot, strawberry, sunflower, tomato, pepper, chrysanthemum, poplar, eucalyptus, and pine.

In still other aspects, commercial products are derived from selected plants that are corn (*Zea mays*), canola (*Brassica napus, Brassica rapa ssp.*), alfalfa (Medicago sativa), rice (*Oryza sativa*), rye (*Secale cereal*), sorghum (*Sorghum bicolor, Sorghum vulgare*), sunflower (*Helianthus annuus*), wheat (*Triticum aestivum*), soybean (*Glycine max*), tobacco (*Nicotiana tabacum, Nicotiana benthamiana*)*,* potato (*Solanum tuberosum*), peanuts (*Arachis hypogaea*), cotton (*Gossypium hirsutum),* sweet potato (*Ipomoea batatus),* cassaya (*Manihot esculenta*), coffee (*Coffea spp.*), coconut (*Cocos nucifera*), pineapple (*Ananas comosus*), citrus trees (*Citrus spp.*), cocoa (*Theobroma cacao*), tea (*Camellia sinensis),* banana (*Musa spp.*), avocado (*Persea americana),* fig (*Ficus casica*), guava (*Psidium guajava*), mango (*Mangifera indica*), olive (*Olea europaea*), papaya *(Carica papaya),* cashew (*Anacardium occidentale*), macadamia (*Macadamia integrifolia*), almond (*Prunus amygdalus*), sugar beets (*Beta vulgaris*), oats, barley, *Arabidopsis* spp., vegetables, ornamentals, and conifers.

5.3 **Microorganisms**

The present methods take advantage of the naturally occurring high degree of genetic diversity that exists in organisms, and efficiently identify, select, and enrich for microorganisms possessing desired gene expression profiles conferring improved properties. The present methods can identify, select, and enrich for microorganisms with improved properties from a pool of genetically diverse microorganisms. In particular aspects, the present methods allow for the generation of novel homogeneous populations possessing improved properties. In certain aspects, the present methods include a step to increase genetic variability. Increasing genetic variability may be achieved using any one of various methods known to one skilled in the art. In specific aspects, increasing genetic variability may be achieved as described herein in section 5.6 below.

In particular aspects, the methods described herein allow for identification, selection and/or enrichment of microorganisms that are used in foods, or used to produce metabolites (*e.g.,* antibiotics), or to catalyze reactions (*e.g.,* enzymes) used in commercial applications. For example, microorganisms selected by the methods described herein may be useful for generating biofuel, or for cleaning oil spills.

In particular aspects, the methods described herein allow for identification, selection and enrichment of microorganisms that possess any one of the following improved properties:

(a) increase yield of a desired product (*e.g.*, functional foods or antibiotics;

(b) increase chemical reaction rates, such as enzymatic reaction rates, *e.g.,* for use in fermentation (*e.g.*, for making cheese, yogurt, bread, or beer);

(c) faster growth;

(d) longer life span;

(e) tolerance to a wide range of temperatures;

(f) adapt to simplified growth conditions; and

(g) improved catalysis or production of components (*e.g.*, enzymes) for catalysis of commercial application.

In specific aspects, the methods described herein provide for identification, isolation and/or enrichment of microorganisms that express one or more RNAs of interest. Such RNAs of interest may be any RNA that confers a benefit to the microorganism. Such RNA of interest may or may not be translated.

In other aspects, the methods described herein provide for identification, isolation and/or enrichment of microorganisms that express a desired gene expression profile.

In specific aspects, microorganisms or microbial cells described herein do not recombinantly express a transgene or have not been genetically modified. In other aspects, microorganisms or microbial cells described herein recombinantly express one or more transgenes or have been genetically modified. Genetically modified microorganisms or microbial cells may be generated using fluorogenic oligonucleotide probes capable of detecting microorganisms or microbial cells recombinantly expressing one or more transgene.

Non-limiting examples of genes encoding RNAs of interest, gene profiles, and desired phenotypes are described herein, *e.g.,* in Table 3, 4, or 5.

**Table 3: Microorganisms: Algae genes and their functions**

| GENE | FUNCTION | REFERENCE |
|---|---|---|
| Proteins in the chloroplast of Chlamydomonas reinhardtii | production of human therapeutic genes | Muto, 2009, BMC Biotechnology, 9:26, Epub |
| Tbal (nuclear gene of Chlamydomonas reinhardtii) | oxidoreductase required for translation of the chloroplast psbA mRNA | Somanchi, 2005, Plant J. Vol. 42 (3): 341-352 |
| PSRP-7 (S1-like protein of Chlamydomonas reinhardtii) | Component of 30S subunit | Beligni, 2004, The Plant Cell Vol. 16: 3357-3369 |
| luxCt | Luciferase transporter gene for Chlamydomonas reinhardtii chloroplast | Mayfield, 2004, Plant J. Vol. 37 (3): 449-458 |

(continued)

| GENE | FUNCTION | REFERENCE |
|---|---|---|
| Genes involved in fatty acid synthesis in microalgae | Possible feedstocks for biofuel production | Hu, 2008, Plant J. Vol. 54 (4): 621-639 |
| Bioenergy genes in microalgae | Biohydrogen and biofuel production | Beer, 2009, Curr Opin Biotechnol. Vol. 20 (3): 264-271 |
| Genes of Chlamydomonas reinhardtii | Iron-Sulfur protein assembly machineries | Godman, 2008, Genetics Vol. 179: 59-68 |
| TILs, CHLs, and ZEPs: lipocalins | Proteins in different algae and plants, possible association with stress-tolerance | Charron, 2005, Plant Physiol. Vol. 139: 2017-2028 |
| psbS gene product in Chlamydomonas reinhardtii | Correlation with energy quenching | Bonente, 2008, Photochem Photobiol. Vol. 84 (6): 1359-1370 |
| psbO (in the sea slug Elysia chlorotica) | Nuclear gene of oxygenic photosynthesis | Rumpho, 2008, PNAS Vol. 105 (46): 17867-17871 |
| LHCBM9 gene in Chlamydomonas reinhardtii | Important function under sulfur starvation and photobiological hydrogen production | Nguyen, 2008, Eukaryot Cell Vol. 7 (11): 1965-1979 |
| Subunit of ribulose bisphosphate carboxylase (rubisco) and light-harvesting complex II (LHCII) subunits | chloroplast proteins in the green alga Chlamydomonas | Uniacke, 2009, PNAS Vol. 106 (5): 1439-1444 |
| ScDSP-1 and ScDSP-2 | Death-specific protein genes in a marine diatom (Skeletonema costatum) | Chung, 2008, Appl Environ Microbiol. Vol. 74 921): 6521-6527 |
| Arabidopsis CHL27 protein | Role in photosynthesis and chloroplast development | Bang, 2008, Plant Cell Physiol. Vol. 49 (9): 1350-1363 |
| Atlg67840 | Chloroplast sensor kinase (CSK) gene product in Arabidopsis thaliana | Puthiyaveetil, 2008, PNAS Vol. 105 (29): 10061-10066 |
| Pfl1 | Pyruvate Formate-Lyase in Chlamydomonas reinhardtii | Hernschemeier, 2008, Eukaryot Cell Vol. 7 (3): 518-526 |
| 5 astaxanthin biosynthesis genes and 2 plastid terminal oxidase genes | Consumption of oxygen by astaxanthin biosynthesis | Li, 2008, J Plant Physiol. Vol. 165 (17): 1783-1797 |
| CCM1 in Chlamydomonas | Regulates carbon-concentrating mechanism | Kohinata, 2008, Plant Cell Physiol. Vol. 49 (2): 273-283 |
| AUREO, WC-la, ELI_04755, Tmden_2087, and NP0654A | LOV proteins in algae, connected with blue light | Losi, 2008, PNAS Vol. 105 (1): 7-8 |
| FCP, ASP, and HSP90 in the Antarctic diatom Chaetoceros neogracile | FCP: fucoxanthin chlorophyll a,c-binding protein ASP: ascorbate peroxidase HSP90: heat-shock protein 90 | Jung, 2007, J Microbiol Biotechnol. Vol. 17 (8): 1330-1337 |
| cbbA, cbbP, cbbE, cbbL, cbbS, cbbX, cbbG, and cbbT | Genes potentially involved in the CBB cycle in Sulfobacillus acidophilus | Caldwell, 2007, Microbiology Vol. 153: 2231-3340 |
| Algal cytochrome c6 gene | Expression in Arabidopsis enhances photosynthesis and growth | Chida, 2007, Plant Cell Physiol. Vol. 48 (7): 948-957 |

(continued)

| GENE | FUNCTION | REFERENCE |
|---|---|---|
| GapA, GapB, and CP12 | Regulation of Calvin cycle in algae and land plants | Robbens, 2007, J Mol Evol. Vol. 64 (5): 601-604 |
| rbcL, FeSOD, VTE3, cdk, and psbA (stress phase genes in Chlamydomonas reinhardtii under copper excess) | Gene markers for alarm (rbcL), hardening (FeSOD, VTE3), exhaustion (cdk, psbA) | Luis, 2006, Plant Cell Environ. Vol. 29 (11): 2043-2054 |
| TpMnSOD | Possible contribution to continued success of diatoms in the low iron regions of the oceans | Wolfe-Simon, 2006, Plant Physiol. Vol. 142: 1701-1709 |
| VHL(R)-S4 and VHL(R)-S9 | very high light VHL-resistant mutants in Chlamydomonas reinhardtii | Foerster, 2006, Proteomics Vol. 6 (15): 4309-4320 |
| RuBisCO | Phytoplankton carbon fixation gene | John, 2007, ISME J. Vol. 1 (6): 517-531 |
| PHR2 in Dunaliella salina | gene product encoding CPD photolyase | Cheng, 2007, J Photochem Photobiol B. Vol. 87 (2): 137-143 |
| p150 (plasma membrane 150-kDa proteine in Dunaliella salina | Increases with rising external salinity | Fisher, 1997, J Biol Chem. Vol. 272 (3): 1565-1570 |
| Circadian-regulated gene CONSTANS (CO) in Arabidopsis thaliana | Central role in photoperiodic control of floral transition | Serrano, 2009, Curr Biol. Vol. 19 (5): 359-368 |
| Ley-beta gene (lycopene betacylase) in Dunaliella salina | Isolation and regulation of Lcy-beta gene by abiotic stress | Ramos, 2008, Appl Microbiol Biotechnol. Vol. 79 (5): 819-828 |
| FAD-GPDH gene in Dunaliella salina | Enhanced by salt treatment, repressed by oxygen deficiency and cold stress | Yang, 2007, J Basic Microbiol. Vol. 47 (3): 266-274 |
| W80 in Chlamydomonas | Antisalt and anticadmium stress activities | Tanaka, 2007, FEMS Microbiol Lett. Vol. 271 (1): 48-52 |
| DsUGDH in Dunaliella salina | Plays part in salt tolerance mechanism | Quinghua, 2005, DNA Seq. Vol. 16 (3): 202-206 |
| cw801ea3 in Chlamydomonas sp. | New member of group 3 late embryogenesis abundant protein | Tanaka, 2004, FEMS Microbiol Lett. Vol. 236 (1): 41-45 |
| DsALDP in Dunaliella salina | Improvement of salt tolerance in E. coli expressing DsALDP fusion protein | Zhang, 2002, DNA Seq. Vol. 13 (4): 195-202 |
| PsaG (cDNA sequences of subunit V) in Dunaliella salina | Connection of light-harvesting complex I protein to photosystem reaction center | Zhang, 2002, DNA Seq. Vol. 13 (3): 173-177 |
| bbc1 of marine Chlamydomonas sp. W-80 strain | Expression in E. coli cells shows enhanced tolerance against salt-stress and freezing-stress | Tanaka, 2001, Curr Microbiol. Vol. 42 (3): 173-177 |
| LI818 gene in Chlamydomonas reinhardtii | Possible relation to CAB proteins of higher plants and green algae, but different expression | Richard, 2000, Plant Mol Biol. Vol. 42 (2): 303-316 |

(continued)

| GENE | FUNCTION | REFERENCE |
|---|---|---|
| Methyl chloride transferase (novel enzyme found in several fungi, marine algae, and halophytic plants) | functions in the control and regulation of the internal concentration of chloride ions in halophytic plant cells | Ni, 1999, PNAS Vol. 96: 3611-3615 |
| PtNOA in marine diatom Phaeodactylum tricornutum | Regulation of nitric-oxide signaling and susceptibility to diatom-derived aldehydes | Vardi, 2008, Curr Biol. Vol. 18 (12): 895-899 |
| HSF1 (heat shock factor 1) in Chlamydomonas reinhardtii | Key regulator of stress response | Schulz-Raffelt, 2007, Plant J. Vol. 52 (2): 286-295 |

**Table 4: Microorganisms: Bacteria genes and their functions**

| GENE | FUNCTION | REFERENCE |
|---|---|---|
| Lactic acid bacteria (LAB) | Food fermentation, probiotics in health-promoting products | Siezen, 2004, Curr Opin Biotechnol. Vol. 15 (2): 105-115 |
| Lactic acid bacteria (LAB) | Food production, health improvement, production of macromolecules, enzymes, and metabolites | Pfeiler, 2007, Trends Microbiol. Vol. 15 (12): 546-553 |
| Lactic acid bacteria (LAB) | Food and beverage fermentation, bulk and fine chemicals production, pharmaceuticals manufacturing | Zhu, 2009, Appl. Michrobiol Biotechn. Vol. 83 (4): 597-610 |
| Lactic acid bacteria (LAB), especially bacteriocins | Food preservation, antimicrobial functions, antifungal activity | Nes, 2004, Curr Opin Biotechnol. Vol. 15 (2): 100-104 |
| Lactic acid bacteria (LAB) | Food production, especially gut functionality | De Vos, 2004, Curr Opin Biotechnol. Vol. 15 (2): 86-93 |
| VIC, MscL, and MscS (in Gram-positive bacteria) | Channel-forming protein families | Lorca, 2004, Biochim Biophys Acta Vol. 1768 (6): 1342-1366 |
| Lactic acid bacteria (LAB), especially bacteriocins | Antimicrobial activities | De Vuyst, 2007, J Mol Microbiol Biotechnol. Vol. 13 (4): 194-199 |
| Lactic acid bacteria (LAB) | Bioprocessing roles in food and beverages | Klaenhammer, 2005, FEMS Microbiol Rev. Vol. 29 (3): 393-409 |
| Lactobacillales-specific clusters of orthologous genes (LaCOGs) (in LABs) | Food and beverage fermentation | Makarova, 2006, PNAS Vol. 103: 15611-15616 |
| Gram-positive bacteria | Regulatory Systems regarding methionine metabolism | Rodionov, 2004, Nucleic Acids Research Vol. 32 (11): 3340-3353 |
| Starter and non-starter lactic acid bacteria | Use in cultured foods | Cogan, 2007, J Dairy Science Vol. 90 (9): 4005-4021 |
| Lactic acid bacteria (LAB) | Food fermentation | Schroeter, 2009, FEMS Microbiol Lett. Vol. 292 (1): 1-6 |
| Genomic features of Lactobacillus | Biopreservation of food | Goh, 2009, Front Biosci Vol. 14: 1362-1386 |
| Strains V583 and MMH594 in Enterococcus faecalis | Food fermentation | Lepage, 2006, J Bacteriol. Vol. 188 (19): 6858-6868 Many references |

(continued)

| GENE | FUNCTION | REFERENCE |
|---|---|---|
| cylL(L), cylL(S), cylM, cylB, and cylA | Production of sheep milk cheese bryndza | Jurkovic, 2006, Lett Appl Microbiol. Vol. 42 (6): 553-559 |
| 1hv_1161, 1hv_1171, 1hv_1031, 1hv_1152, 1hv_1978, and 1hv_0028 | Dairy specific genes of Lactobacillus helveticus DPC4571 | O'Sullivan, 2009, MBC Microbiology 9:50 Epub |
| Lba_0892, 1ba_1078 | Gut specific genes of Lactobacillus helveticus DPC4571 | O'Sullivan, 2009, MBC Microbiology 9:50 Epub |
| Lba_0892, lba_1078 | Gut specific genes of Lactobacillus helveticus DPC4571 | O'Sullivan, 2009, MBC Microbiology 9:50 Epub |
| IspDF in Mesorhizobium loti | bifunctional protein that catalyzes non-consecutive steps in the methylerythritol phosphate pathway | Testa, 2006, Biochim Biophys Acta Vol. 1764 (1): 85-96 |
| gpxl, sodB, katG, acnB, gamma-TMY, dnaK2, and Hsp70 in Synechocystis sp. PCC6803 | Connection to oxidative stress | Li, 2009, Toxicon. Vol. 53 (6): 595-601 |

**Table 5: Microorganisms: Fungi genes and their functions**

| GENE | FUNCTION | REFERENCE |
|---|---|---|
| Trichoderma reesei QM9414 genes: cbh1, cbh2, egll, egl2, egl5 | Encoding cellobiohydrolases respectively endoglucanases | Ilmen, 1997, Appl Environ Microbiol Vol. 63 (3): 1298-1306 |
| creA gene in Aspergillus nidulans | Antisense expression: substantial increase in the levels of glucose-repressible enzymes | Bautista, 2000, Appl Environ Microbiol. Vol. 66 (10): 4579-4581 |
| cpcA gene in Aspergillus nidulans | Amino acid regulation gene | Hoffmann, 2001, Mol Biol Cell Vol. 12: 2846-2857 |
| Baker's-yeast (Saccharomyces cerevisiae) cells | Genes involved in stress tolerance | Shima, 2009, Biotechnol Appl Biochem. Vol. 53 (Pt3): 155-164 |
| PiD6: new cDNA from the oleaginous fungus, Pythium irregulare | Fatty acid desaturation | Hong, 2002, Plant Physiol. Vol. 129: 354-362 |
| G-protein $\alpha$-Subunit GasC | Role in Germination in Dimorphic Fungus Penicillium marneffei | Zuber, 2003, Genetics Vol. 164: 487-499 |
| FLO1, FLO5 and FLO11 in Saccharomyces cerevisiae | Flocculation genes | Govender, 2008, Appl Environ Microbiol. Vol. 74 (19): 6041-6052 |
| BTN2 in flor yeast | Involved in ethanol tolerance and biofilm formation | Espinazo-Romeu, 2008, FEMS Yeast Res. Vol. 8 (7): 1127-1136 |

In specific aspects the microorganisms or microbial cells are purified (e.g., contains less than 20%, 15%, 10%, or 5% contaminants).

Non-limiting examples of microorganisms include bacteria, fungi, archae, protozoa, and algae. In specific aspects, microorganisms are non-pathogenic. Non-pathogenic microorganisms may be used as inoculum to protect from diseases (biocontrol and biofertilizers in agriculture, probiotics), to restore contaminated sites (bioremediation) or for fermentation in food processes. Pathogenicity or virulence is the capacity of some microorganisms to cause disease. In specific aspects, microorganisms described herein are not capable to cause a disease in a plant or animal (e.g., human or live stock). In certain aspects, microorganisms described herein belong to a prokaryotic or eukaryotic microbial species from the Domains Archaea, Bacteria and Eucarya, the latter including yeast and filamentous fungi, protozoa, algae, or higher Protista. The terms "microbial cells" and "microbes" are used interchangeably with the term microorganism.

Bacteria include at least 11 distinct groups as follows: (1) Gram-positive (gram+) bacteria, of which there are two major subdivisions: (i) high G+C group (Actinomycetes, Mycobacteria, Micrococcus, others), and (ii) low G+C group (Bacillus, Clostridia, Lactobacillus, Staphylococci, Streptococci, Mycoplasmas); (2) Proteobacteria, *e.g.*, Purple photosynthetic +non-photosynthetic Gram-negative bacteria (includes most "common" Gram-negative bacteria); (3) Cyanobacteria, *e.g.*, oxygenic phototrophs; (4) Spirochetes and related species; (5) Planctomyces; (6) Bacteroides, Flavobacteria; (7) Chlamydia; (8) Green sulfur bacteria; (9) Green non-sulfur bacteria (also anaerobic phototrophs); (10) Radioresistant micrococci and relatives; and (11) Thermotoga and Thermosipho thermophiles.

Non-limiting examples of gram-negative bacteria include cocci, nonenteric rods, and enteric rods. The genera of Gram-negative bacteria include, for example, Neisseria, Spirillum, Pasteurella, Brucella, Yersinia, Francisella, Haemophilus, Bordetella, Escherichia, Salmonella, Shigella, Klebsiella, Proteus, Vibrio, Pseudomonas, Bacteroides, Acetobacter, Aerobacter, Agrobacterium, Azotobacter, Spirilla, Serratia, Vibrio, Rhizobium, Chlamydia, Rickettsia, Treponema, and Fusobacterium.

Non-limiting examples of gram positive bacteria include cocci, nonsporulating rods, and sporulating rods. The genera of gram positive bacteria include, for example, Actinomyces, Bacillus, Clostridium, Corynebacterium, Erysipelothrix, Lactobacillus, Listeria, Mycobacterium, Myxococcus, Nocardia, Staphylococcus, Streptococcus, and Streptomyces.

In certain aspects, selected microorganisms described herein can produce metabolites in quantities not available in the reference population, or in quantities higher than the average quantities produced by the reference population (or unselected starting population). A metabolite can be any substance produced by metabolism or a substance necessary for or taking part in a particular metabolic process. In certain aspects, a metabolite can be an organic compound that is a starting material (*e.g.*, glucose or pyruvate) in, an intermediate (*e.g.,* Acetyl-CoA) in, or an end product (*e.g.,* isopropanol) of metabolism. In other aspects, metabolites can be used to construct more complex molecules, or they can be broken down into simpler ones. Intermediate metabolites may be synthesized from other metabolites, perhaps used to make more complex substances, or broken down into simpler compounds, often with the release of chemical energy. End products of metabolism are the final result of the breakdown of other metabolites.

In certain aspects, selected microorganisms described herein can express one or more target enzymes involved in pathways for the production of a desirable product, *e.g.*, biofuels such as isopropanol, from using a suitable carbon substrate. Non-limiting examples of carbon substrates used by microorganisms include, 6 carbon sugars, including but not limited to glucose, lactose, sorbose, fructose, idose, galactose and mannose all in either D or L form, or a combination of 6 carbon sugars, such as glucose and fructose, and/or 6 carbon sugar acids including, but not limited to, 2-keto-L-gulonic acid, idonic acid (IA), gluconic acid (GA), 6- phosphogluconate, 2-keto-D-gluconic acid (2 KDG), 5-keto-D-gluconic acid, 2-ketogluconatephosphate, 2, 5-diketo-L-gulonic acid, 2, 3-L- diketogulonic acid, dehydroascorbic acid, erythorbic acid (EA) and D-mannonic acid. In specific aspects, microorganisms described herein are metabolically-modified microorganisms useful for producing biofuels *(e.g.,* ethanol or isopropanol), see, *e.g.,* International Patent Application Publication No. WO 2009/049274.

Appropriate culture conditions for microorganisms are known to the skilled person. Appropriate culture conditions for microorganisms may involve adjustments in medium pH, ionic strength, nutritive content, *et al.*; temperature; oxygen/CO2/nitrogen content; humidity; and/or other culture conditions.

Techniques for introducing nucleic acids into a microorganism are well-known and readily appreciated by the skilled worker. Such methods include but are not limited to transfection, viral delivery, protein or peptide mediated insertion, coprecipitation methods, lipid based delivery reagents (lipofection), cytofection, lipopolyamine delivery, dendrimer delivery reagents, electroporation or mechanical (*e.g.*, microinjection) or chemical delivery. A non-limiting example of a viral delivery system for bacteria is bacteriophage.

Selected microorganisms (including microbial cells) may be tested in functional assays for validation that the microorganisms (including microbial cells) possess the function or phenotype selected for. Such functional assays are known the a skilled person in the art. For example, RNA or protein expression may be confirmed by RT-PCR, real time PCR, immunoblotting, flow cytometry, or ELISA. Selected microorganisms with a desired trait for sustaining and/or growing under certain conditions may be tested by exposing the selected microorganisms to such conditions and comparing the growth with a control (*e.g.*, unselected microorganism or reference microorganism). Selected microorganisms that have phenotypes visually observable may be validated by visual inspection using microscopy (*e.g.*, electron microscopy).

5.4 **Animals**

The methods described herein provide for identifying and/or selection of eukaryotic cells that express one or more gene of interest. In certain aspects, the gene of interest is expressed at higher levels than other cells as a result of genetic variability. In particular aspects, the methods described here comprise (a) introducing into a eukaryotic cell one or more fluorogenic oligonucleotide probes that is capable of detecting an RNA of interest (e.g., capable of hybridizing to a target sequence of an RNA of interest); and (b) determining whether the eukaryotic cell comprises the RNA of interest. Such methods may further comprise quantifying the level of the RNA of interest. In specific aspects, the methods described herein for identifying a eukaryotic cell with a desired RNA expression profile, wherein the method comprises: (a) introducing into a eukaryotic cell a plurality of fluorogenic oligonucleotide probes each capable of detecting an RNA of interest; and (b) quantifying the RNA levels detected by the plurality of fluorogenic oligonucleotide probes. The desired gene expression profile may be determined by comparison to a reference population.

In specific aspects, eukaryotic cells identified and/or selected by the methods described herein have not been genetically engineered (e.g., do not recombinantly express one or more transgenes). In other aspects, eukaryotic cells identified and/or selected by the methods described herein have been genetically engineered (e.g., do recombinantly express one or more transgenes). In specific aspects, such cells are somatic cells or differentiated cells.

In certain aspects, the present disclosure provides for methods of generating iPS cells from eukaryotic cells identified and/or selected by the methods described herein. Such methods comprise exposing eukaryotic cells identified and/or selected by the methods described herein to conditions suitable for reprogramming a cell to an iPS cell. Conditions suitable for generating iPS cells have been described, see, e.g., Woltjen et al., Nature, 2009, 458:766-770; and Zhao et al., "iPS cells produce viable mice through tetraploid complementation," Nature, advance online publication 23 July 2009). iPS cells can be maintained in the pluripotent state by the addition of defined growth factors and/or by co-culturing the cells with irradiated fibroblasts (see, e.g., Amit et al., Semin. Reprod. Med., 2006, 24(5):298-303).

In specific aspects, methods described herein provide using iPS cells to generate a whole non-human organism, or tissue or organs. Such methods comprise exposing iPS cells described herein to conditions that are suitable for generating a whole non-human animal, or tissue or an organ.

In certain aspects a whole non-human organism or animal generated from iPS cells may be, but are not limited to, a mouse, rat, monkey, dog, cat, pig, sheep, goat, horse, chicken, donkey, frog, worm, insect (e.g., fly), or cow. In specific aspects, a non-human organism is a fish or shellfish. In other aspects, new tissue or organ generated from iPS cells may be, but are not limited to, new tissue or organ of a human, mouse, rat, monkey, dog, cat, pig, sheep, goat, horse, chicken, donkey, frog, worm, insect (e.g., fly), or cow. In other aspects, body parts generated from iPS cells may be, but are not limited to, body parts a human, mouse, rat, monkey, dog, cat, pig, sheep, goat, horse, chicken, donkey, frog, worm, insect (e.g., fly), or cow. In certain aspects, the organ may be but is not limited to breast, colon, throat, prostate, uterus, stomach, heart, brain, spinal cord, lung, liver, pancreas, kidney, eye, bladder, or skin. In certain aspects, the new tissue may be but is not limited to tissue of the muscle, breast, colon, throat, prostate, uterus, stomach, heart, brain or nervous system, spinal cord, lung, liver, pancreas, kidney, eye, bladder, or skin.

In certain aspects, iPS cells of muscle, breast, colon, throat, prostate, uterus, stomach, heart, brain or nervous system, spinal cord, lung, liver, pancreas, kidney, eye, bladder, or skin can be generated. Non-limiting examples include skin or eye cell types, especially those engineered or selected to have different hues, colors, pigmentation, e.g., for use in cosmetic cell therapy or treatment. In certain aspects, cells are first selected and then iPS cell state is induced. In other aspects, iPS cell state is first induced and cells with desired gene expression profiles (and thus desired phenotypes/traits) are then engineered or selected. Genetic variability may be induced before, during, or after induction of iPS cell state.

In specific aspects, the methods described herein for generating a non-human animal comprise the steps of:

(a) obtaining somatic or differentiated cells of a desired cell type that has been engineered to comprise one or more RNA of interest;
(b) introducing to the cell fluorogenic oligonucleotide probes capable of detecting the RNAs of interest;
(c) isolating cells comprising the RNA of interest;
(d) exposing the cells to conditions suitable to generate iPS cells; and
(e) exposing the iPS cells of step (d) to conditions suitable to generate a whole non-human organism.

Obtaining cells engineered to comprise one or more RNA of interest may be achieved by introducing one or more recombinant nucleic acid constructs encoding an RNA of interest into the cells, so that such cells may recombinantly express one or more transgenes. In certain aspects, exposing cells to a condition that increases genetic variability (e.g., conditions described herein in section 5.6, may produce a cell containing one or more RNA of interest. In other aspects, the cell comprises a desired gene expression profile. In certain aspects, the desired gene expression profile is achieved by genetic engineering or by increasing genetic variability. In specific aspects, a desired gene expression profile may be determined based on comparison with that of a reference population.

In some aspects, the methods described herein are for identifying and/or selecting cells that express an RNA of interest at a level higher than the average heterologous cell population. In specific aspects, the methods described herein are for identifying and/or selecting cells that express an RNA of interest at a level lower than the average heterologous cell population. The heterologous cell population may be a cell population of mixed cell types of different origin, or a cell population of cells of one cell type that are genetically heterologous.

In specific aspects, cells are selected based on their gene expression profile as a differentiated cell type. As a result, the same differentiated cell types in the non-human animal produced according to methods described herein would comprise the same genetic features which were selected for.

In specific aspect, the disclosure provides for improved non-human animals generated by the methods described herein. In specific aspects, the disclosure provides for commercial products derived from improved non-human animals generated by the methods described herein.

In a further aspect of the present disclosure, differentiated, adult or specialized cells generated according to the methods described herein may be used to generate stem cells. In some aspects, cells of the disclosure wherein the cell type or specification is a differentiated, adult or specialized cell may be dedifferentiated into stems cells including but not limited to multipotent stem cells, pluripotent stem cells, omnipotent stem cells, iPS cells, embryonic stem cells, cancer stem cells, and organ or tissue specific stem cells. Methods of dedifferentiation are known to those skilled in the art. See, *e.g.*, Panagiotis A. Tsonis; Stem Cells from Differentiated Cells; Molecular Interventions 4:81-83, (2004). Stem cells generated from the cells described herein may be differentiated into one or more cells of a differentiated, adult, or specialized cell type or specification. Embryonic stem cells and iPS cells generated from the cells described herein may be used to produce a whole non-human organism, e.g., a mouse. Method of producing mice using mouse embryonic stem cells are known to those skilled in the art. See, *e.g.,* Ohta et al., Biol Reprod., 79(3):486-92 (2008). Methods of producing mice using iPS cells are known to those skilled in the art. See, *e.g.,* Zhao et al., "iPS cells produce viable mice through tetraploid complementation," Nature, advance online publication 23 July 2009.

In some aspects, cells of the disclosure wherein the cell type or specification is a differentiated, adult or specialized cell may be dedifferentiated into embryonic stem cells or iPS cells, and the stem cells thus produced may be used to produce a whole non-human organism, e.g., a mouse, wherein the cells in the non-human organism of the same cell type or specification comprise the same properties for which the cells of the disclosure were selected, e.g., expression of a protein or RNA of interest.

In some aspects, cells of a specialized cell or tissue type comprising an RNA or protein or a functional or physiological form of an RNA or protein may be used to produce an embryonic stem cell or iPS cell that may be used to produce a non-human organism, *e.g.*, a mouse, wherein the cells or tissues of the non-human organism of the same type comprise the RNA or protein or the functional or physiological form of the RNA or protein. In some aspects, the non-human organism thus produced comprises the RNA or protein of a different species. In some aspects, the non-human organism is mouse and the RNA or protein is of a human origin. In some aspects, the non-human organism thus produced comprises desired gene expression profiles as compared to a reference population. In some aspects, the non-human organism thus produced may be used in testing, including preclinical testing. In some aspects, the testing or preclinical testing is used to predict the activity of test compounds in humans.

Techniques for introducing nucleic acids (*e.g.*, reporter nucleic acid constructs and recombinant nucleic acid constructs encoding CTR factors) into cells are well-known and readily appreciated by the skilled worker. The methods include but are not limited to transfection, viral delivery, protein or peptide mediated insertion, coprecipitation methods, lipid based delivery reagents (lipofection), cytofection, lipopolyamine delivery, dendrimer delivery reagents, electroporation or mechanical delivery. Examples of viral delivery systems include but are not limited to retroviruses, lentiviruses, and adenoviruses. In certain aspects, gene activation may be used for expression of one or more CTR factors in a host cell.

In certain aspects, a cell is not a human cell. In particular aspects, a cell is a cell derived from a mouse, rat, monkey, dog, cat, pig, sheep, goat, horse, chicken, frog, worm, insect *(e.g.,* fly), or cow. In certain aspects, an organism is a fish or shellfish. In some aspects, a cell is a mammalian cell, or a eukaryotic cell. In other aspects, a cell is a human cell. In some aspects, cells are primary cell. In other aspects, cells are transformed cells of a cell line.

Other non-limiting examples of cells that may be used in the methods described herein include: epidermal keratinocyte (differentiating epidermal cell), epidermal basal cell (stem cell), keratinocyte of fingernails and toenails, nail bed basal cell (stem cell), medullary hair shaft cell, cortical hair shaft cell, cuticular hair shaft cell, cuticular hair root sheath cell, hair root sheath cell of Huxley's layer, hair root sheath cell of Henle's layer, external hair root sheath cell, hair matrix cell (stem cell), surface epithelial cell of stratified squamous epithelium of cornea, tongue, oral cavity, esophagus, anal canal, distal urethra and vagina, basal cell (stem cell) of epithelia of cornea, tongue, oral cavity, esophagus, anal canal, distal urethra and vagina, urinary epithelium cell (lining urinary bladder and urinary ducts), salivary gland mucous cell (polysaccharide-rich secretion), salivary gland serous cell (glycoprotein enzyme-rich secretion), von Ebner's gland cell in tongue (washes taste buds), mammary gland cell (milk secretion), lacrimal gland cell (tear secretion), ceruminous gland cell in ear (wax secretion), eccrine sweat gland dark cell (glycoprotein secretion), eccrine sweat gland clear cell (small molecule secretion), apocrine sweat gland cell (odoriferous secretion, sex-hormone sensitive), gland of Moll cell in eyelid (specialized sweat gland), sebaceous gland cell (lipid-rich sebum secretion), bowman's gland cell in nose (washes olfactory epithelium), Brunner's gland cell in duodenum (enzymes and alkaline mucus), seminal vesicle cell (secretes seminal fluid components, including fructose for swimming sperm), prostate gland cell (secretes seminal fluid components), bulbourethral gland cell (mucus secretion), Bartholin's gland cell (vaginal lubricant secretion), gland of Littre cell (mucus secretion), uterus endometrium cell (carbohydrate secretion), isolated goblet cell of respiratory and digestive tracts (mucus secretion), stomach lining mucous cell (mucus secretion), gastric gland zymogenic cell (pepsinogen secretion), gastric gland oxyntic cell (hydrochloric acid secretion), pancreatic acinar cell (bicarbonate and digestive enzyme secretion), paneth cell of small intestine (lysozyme secretion), type II pneumocyte of lung (surfactant secretion), clara cell of lung, anterior pituitary cells, somatotropes, lactotropes, thyrotropes, gonadotropes, corticotropes, intermediate pituitary cell, secreting melanocyte-stimulating hormone, magnocellular neurosecretory cells (secreting oxytocin and/or secreting vasopressin), gut and respiratory tract cells (secreting serotonin, secreting endorphin, secreting somatostatin, secreting gastrin, secreting secretin, secreting cholecystokinin, secreting insulin, secreting glucagons, and/or secreting bombesin), thyroid gland cells, thyroid epithelial cell, parafollicular cell, parathyroid gland cells, parathyroid chief cell, oxyphil cell, adrenal gland cells, chromaffin cells, adrenal gland secreting steroid hormones (mineralcorticoids and gluco corticoids), Leydig cell of testes secreting testosterone, theca interna cell of ovarian follicle secreting estrogen, corpus luteum cell of ruptured ovarian follicle secreting progesterone (Granulosa lutein cells, and Theca lutein cells), juxtaglomerular cell (renin secretion), macula densa cell of kidney, peripolar cell of kidney, mesangial cell of kidney, hepatocyte (liver cell), white fat cell, brown fat cell, liver lipocyte, kidney glomerulus parietal cell, kidney glomerulus podocyte, kidney proximal tubule brush border cell, loop of Henle thin segment cell, kidney distal tubule cell, kidney collecting duct cell, type I pneumocyte (lining air space of lung), pancreatic duct cell (centroacinar cell), nonstriated duct cell (of sweat gland, salivary gland, mammary gland, *et al.*) such as principal cell and intercalated cell, duct cell (of seminal vesicle, prostate gland, *et al.*), intestinal brush border cell (with microvilli), exocrine gland striated duct cell, gall bladder epithelial cell, ductulus efferens nonciliated cell, epididymal principal cell, epididymal basal cell, blood vessel and lymphatic vascular endothelial fenestrated cell, blood vessel and lymphatic vascular endothelial continuous cell, blood vessel and lymphatic vascular endothelial splenic cell, synovial cell (lining joint cavities, hyaluronic acid secretion), serosal cell (lining peritoneal, pleural, and pericardial cavities), squamous cell (lining perilymphatic space of ear), squamous cell (lining endolymphatic space of ear), columnar cell of endolymphatic sac with microvilli (lining endolymphatic space of ear), columnar cell of endolymphatic sac without microvilli (lining endolymphatic space of ear), dark cell (lining endolymphatic space of ear), vestibular membrane cell (lining endolymphatic space of ear), stria vascularis basal cell (lining endolymphatic space of ear), stria vascularis marginal cell (lining endolymphatic space of ear), cell of Claudius (lining endolymphatic space of ear), cell of Boettcher (lining endolymphatic space of ear), choroid plexus cell (cerebrospinal fluid secretion), pia-arachnoid squamous cell, pigmented ciliary epithelium cell of eye, nonpigmented ciliary epithelium cell of eye, corneal endothelial cell, respiratory tract ciliated cell, oviduct ciliated cell (in female), uterine endometrial ciliated cell (in female), rete testis ciliated cell (in male), ductulus efferens ciliated cell (in male), ciliated ependymal cell of central nervous system (lining brain cavities), ameloblast epithelial cell (tooth enamel secretion), planum semilunatum epithelial cell of vestibular apparatus of ear (proteoglycan secretion), organ of Corti interdental epithelial cell (secreting tectorial membrane covering hair cells), loose connective tissue fibroblasts, corneal fibroblasts (corneal keratocytes), tendon fibroblasts, bone marrow reticular tissue fibroblasts, other nonepithelial fibroblasts, pericyte, nucleus pulposus cell of intervertebral disc, cementoblast/cementocyte (tooth root bonelike cementum secretion), ontoblast/odontocyte (tooth dentin secretion), hyaline cartilage chondrocyte, fibrocartilage chondrocyte, elastic cartilage chondrocyte, oteoblast/osteocyte, osteoprogenitor cell (stem cell of osteoblasts), hyalocyte of vitreous body of eye, stellate cell of perilymphatic space of ear, hepatic stellate cell (Ito cell), pancreatic stellate cell, skeletal muscle cells (such as Red skeletal muscle cell (slow), white skeletal muscle cell (fast), intermediate skeletal muscle cell, nuclear bag cell of muscle spindle, and nuclear chain cell of muscle spindle), satellite cell (stem cell), heart muscle cells (such as ordinary heart muscle cell, nodal heart muscle cell, and purkinje fiber cell), smooth muscle cell (various types), myoepithelial cell of iris, myoepithelial cell of exocrine glands, erythrocyte (red blood cell), megakaryocyte (platelet precursor), monocytes, connective tissue macrophage (various types), epidermal Langerhans cell, osteoclast (in bone), dendritic cell (in lymphoid tissues), microglial cell (in central nervous system), neutrophil granulocyte, eosinophil granulocyte, basophil granulocyte, mast cell, helper T cell, suppressor T cell, cytotoxic T cell, natural Killer T cell, B cell, natural killer cell, reticulocyte, stem cells and committed progenitors for the blood and immune system (various types), auditory outer hair cell of organ of Corti, basal cell of olfactory epithelium (stem cell for olfactory neurons), cold-sensitive primary sensory neurons, heat-sensitive primary sensory neurons, merkel cell of epidermis (touch sensor), olfactory receptor neuron, pain-sensitive primary sensory neurons (various types), photoreceptor cells of retina in eye (such as photoreceptor rod cells, photoreceptor blue-sensitive cone cell of eye, photoreceptor green-sensitive cone cell of eye, photoreceptor red-sensitive cone cell of eye), proprioceptive primary sensory neurons (various types), touch-sensitive primary sensory neurons (various types), type I carotid body cell (blood pH sensor), type II carotid body cell (blood pH sensor), type I hair cell of vestibular apparatus of ear (acceleration and gravity), type II hair cell of vestibular apparatus of ear (acceleration and gravity), type I taste bud cell, cholinergic neural cell (various types), adrenergic neural cell (various types), peptidergic neural cell (various types), inner pillar cell of organ of Corti, outer pillar cell of organ of Corti, inner phalangeal cell of organ of Corti, outer phalangeal cell of organ of Corti, border cell of organ of Corti, hensen cell of organ of Cortim vestibular apparatus supporting cell, type I taste bud supporting cell, olfactory epithelium supporting cell, schwann cell, satellite cell (encapsulating peripheral nerve cell bodies), enteric glial cell, astrocyte (various types), neuron cells (large variety of types, still poorly classified), oligodendrocyte, spindle neuron, anterior lens epithelial cell, crystallin-containing lens fiber cell, melanocyte, retinal pigmented epithelial cell, oogonium/oocyte, spermatid, spermatocyte, spermatogonium cell (stem cell for spermatocyte), spermatozoon, ovarian follicle cell, sertoli cell (in testis), thymus epithelial cell, and interstitial kidney cells.

In specific aspects the eukaryotic cells are purified (*e.g.*, contains less than 20%, 15%, 10%, or 5% contaminants).

In certain aspects, the present disclosure also relates to products (*e.g.*, commercial products) produced from the selected eukaryotic cells or organisms (*e.g.*, animals or fish) described herein. For example, such products may include meat or other products for human consumption. In certain aspects, such products include milk or oils derived from the selected animals described herein. In specific aspects, immunoglobulins or antibodies that may be useful as therapeutics may be derived from the selected animals described herein. In some aspects, polypeptides (*e.g.*, enzymes) may be obtained from the selected animals described herein.

Techniques for introducing nucleic acids (*e.g.*, fluorogenic oligonucleotide probes or transgene nucleic acid constructs) into cells are well-known and readily appreciated by the skilled worker. The methods include but are not limited to transfection, viral delivery, protein or peptide mediated insertion, coprecipitation methods, lipid based delivery reagents (lipofection), cytofection, lipopolyamine delivery, dendrimer delivery reagents, electroporation or mechanical or chemical delivery. Examples of vectors that may be used to introduce the nucleic acids into host cells include but are not limited to plasmids, viruses, including retroviruses, lentiviruses, adenoviruses, cosmids, baculovirus, and artificial chromosomes.

Selected eukaryotic cells or organisms (*e.g.*, animals) may be tested in functional assays for validation that the selected eukaryotic cells or organisms possess the function or phenotype selected for. Such functional assays are known the a skilled person in the art. For example, RNA or protein expression may be confirmed by RT-PCR, real time PCR, immunoblotting, flow cytometry, or ELISA. Selected eukaryotic cells or organisms with a desired trait for sustaining and/or growing under certain conditions may be tested by exposing the selected eukaryotic cells or organisms to such conditions and comparing the growth with a control (*e.g.*, unselected eukaryotic cells or organisms or reference eukaryotic cells or organisms). Selected eukaryotic cells or organisms that have phenotypes visually observable may be validated by visual inspection using microscopy or the naked eye.

## 5.5 Fluorogenic oligonucleotide probes and target sequences

The methods of the invention described herein employ the use of fluorogenic oligonucleotide probes or molecular beacons to select for plant cells comprising an RNA of interest or a desired RNA expression profile compared to that of a reference population. Fluorogenic oligonucleotide probes or molecular beacons have been described, see, *e.g.,* U.S. Patent No. 6,692,965, and International PCT Patent Application Publication No. WO 2005/079462 A2. In specific aspects, a fluorogenic oligonucleotide probe is conjugated to a fluorophore and a quencher, and can hybridize or bind to a target sequence, such as a target sequence of an RNA transcript. The fluorogenic oligonucleotide probe adopts a structure or conformation when it is not bound to or hybridized with a target sequence, and adopts a different structure or conformation when it is bound to or hybridized with a target sequence. That is, conformational change of the fluorogenic oligonucleotide probe may occur in the presence of the target sequence, where this change results in decreased efficiency for the quenching of the signal that is emitted from the fluorophore when this is excited. In specific embodiments, the fluorescent signal that is emitted from the fluorogenic oligonucleotide probe in the presence of the target sequence is higher than the fluorescent signal that is emitted from the fluorogenic oligonucleotide probe in the absence of the target sequence. In specific embodiments, the fluorescent signal is quenched when the fluorogenic oligonucleotide probe is not hybridized to the target sequence. The hybridized fluorogenic oligonucleotide probe not hybridized to a target sequence may form a stem-loop structure. In certain aspects, the quenched detection signal may be a result of this stem-loop structure.

In specific embodiments, a fluorescent signal that is emitted from the fluorogenic oligonucleotide probe in the presence of a target sequence is at least about 5%, 10%, 20%, 30%, 40%, 50%, 60%, 70%, 80%, 90%, or 100% higher than the fluorescent signal that is emitted from the fluorogenic oligonucleotide probe in the absence of the target sequence. In specific embodiments, the fluorescent signal that is emitted from the fluorogenic oligonucleotide probe in the presence of the target sequence is at least about 1 fold, 1.5 fold, 2 fold, 3 fold, 4 fold, 5 fold, 10 fold, 15 fold, 20 fold, 50 fold, 100 fold, 500 fold, or 1,000 fold higher than the fluorescent signal that is emitted from the fluorogenic oligonucleotide probe in the absence of the target sequence.

A variety of RNA sequences, any of which may be used as target sequences, e.g., a fragment of an RNA transcript encoded by a gene of interest, or an untranslated region ("UTR") of a gene of interest. A target sequence may be a part of a 3' UTR of an RNA. In certain embodiments, the target sequence may be a part of a 5' UTR of an RNA.

In specific embodiments, a target sequence does not have to be translated for detection by the fluorogenic oligonucleotide. A target sequence may be a fragment of an RNA transcript region that is translated. In other embodiments, a target sequence may be a fragment of an RNA transcript region that is not translated. The target sequences may comprise multiple target sequences that are the same or different, wherein one fluorogenic oligonucleotide probe hybridizes to each target sequence.

In particular embodiments, a target sequence is a fragment of an RNA transcript that is expressed in a cell-type specific manner. In other embodiments, a target sequence is a fragment of an RNA transcript, of which expression is induced by an environmental signal, *e.g.*, presence or absence of nitrogen or sunlight.

In some embodiments, a target sequence may be an RNA having a secondary structure. The structure may be a three-arm junction structure.

In specific embodiments, a target sequence comprises a region of an RNA of interest, wherein the region comprises one or more mutations. In such case, the fluorogenic oligonucleotide probe may detect an RNA of interest that has a mutation, and may not detect an RNA that does not have the mutation. An RNA of interest with such a mutation may confer a desirable trait that is not observed with a wild-type RNA. In specific embodiments, fluorogenic oligonucleotide probes are capable of detecting transcript of a gene naturally expressed (e.g., endogenous gene). In certain embodiments, fluorogenic oligonucleotide probes are capable of detecting transcript of a transgene, e.g., transcript of a recombinant nucleic acid construct encoding a transgene. Such fluorogenic oligonucleotide probes are useful for selecting cells that contain or recombinantly express one or more transgenes.

In particular embodiments, a target sequence or fluorogenic oligonucleotide probe has a GC-content of about 30%-70%. In specific embodiments, a target sequence is at least about 30%, 35%, 40%, 45%, 50%, 55%, 60%, 65%, 70%, or 75% GC-rich. In other embodiments, a target sequence is at most about 30%, 35%, 40%, 45%, 50%, 55%, 60%, 65%, 70%, or 75% GC-rich.

In a specific embodiment, a target sequence or fluorogenic oligonucleotide probe is about 5 to 1,000 nucleotides, about 5 to 750 nucleotides, about 5 to 500 nucleotides, about 5 to 250 nucleotides, about 5 to 200 nucleotides, about 5 to 150 nucleotides, about 5 to 100 nucleotides, about 5 to 100 nucleotides, about 5 to 75 nucleotides, about 5 to 500 nucleotides, about 10 to 100 nucleotides, about 10 to 75 nucleotides, about 10 to 50 nucleotides, about 10 to 30 nucleotides, about 5 to 20 nucleotides, about 20 to 100 nucleotides, about 20 to 75 nucleotides, or about 30 to 100 nucleotides, in length, or any length in between. In a specific embodiment, a target sequence or fluorogenic oligonucleotide probe is about 5, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 nucleotides in length.

In certain embodiments, a target sequence or fluorogenic oligonucleotide probe is at most 10 nucleotides, 15 nucleotides, 20 nucleotides, 25 nucleotides, 30 nucleotides, 35 nucleotides, 40 nucleotides, 45 nucleotides, 50 nucleotides, 55 nucleotides, 60 nucleotides, 65 nucleotides, 70 nucleotides, 75 nucleotides, 80 nucleotides, 85 nucleotides, 90 nucleotides, 95 nucleotides, or 100 nucleotides in length. In a specific embodiment, a target sequence or fluorogenic oligonucleotide probe is less than 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 nucleotides in length.

In some embodiments, a target sequence does not comprise a transcription termination sequence. In eukaryotic cells, purified RNA Polymerase III terminate transcription after polymerizing a series of U residues. The transcription termination sequence may comprise a series of U residues such as UUU, UUUU, UUUUU, UUUUUU, UUUUUUU, UUUUUUUU, or UUUUUUUUU. In certain embodiments a RNA Polymerase III transcription termination sequence may comprise an RNA sequence comprising 10 or more U residues, consecutively or nonconsecutively. In particular embodiments, the transcription termination sequence may comprise at least 40%, 50%, 55%, 60%, 65%, 70%, 75%, 80%, 85%, 90%, 95%, or 99% U residues. In bacteria, transcription termination may include Rho-independent termination or Rho-dependent termination. Rho-idependent termination involves a series of U residues preceded by a GC-rich self-complementary region with several intervening nucleotides in the transcribed RNA. The GC-rich self-complementary region may form a stem-loop structure.

In specific embodiments, a target sequence does not comprise a polyadenylation sequence, AAUAAA. In some embodiments, a target sequence does not comprise or is not a poly(A) tail. In certain embodiments, a target sequence is a ribozyme the cleaves the 3'end of a transcript, which may create consistent 3' ends. In particular embodiments, a target sequence is not a ribozyme the cleaves the 3'end of a transcript.

In other embodiments, a target sequence is not a UTR (*e.g.,* 5' UTR or 3' UTR), or a fragment thereof. In other embodiments, a target sequence is not translated. In some embodiments, a target sequence is not a coding region of a gene or an mRNA, or fragment thereof. In specific embodiments, a target sequence is not an siRNA or a miRNA, or a precursor thereof. In specific embodiments, a target sequence is an siRNA or a miRNA, or a precursor thereof.

Any fluorogenic oligonucleotide probes useful in the methods described herein can be used. Fluorogenic oligonucleotides can be useful for selection of plant cells, microorganisms, or eukaryotic cells with the desired features. By way of a non-limiting illustration, a fluorogenic oligonucleotide probe may comprise a fluorophore and a quencher positioned in the fluorogenic oligonucleotide so that the quencher and fluorophore are brought together in the absence of target sequence. For example, the fluorophore may be positioned at the terminus of an oligonucleotide probe and the quencher may be positioned at the other terminus of the oligonucleotide probe, wherein the oligonucleotide probe adopts one conformation or secondary structure, such as a stem-loop or hairpin loop, when not bound or hybridized to a target sequence, and adopts a different conformation or secondary structure when bound or hybridized to a target sequence. For example, upon binding between the fluorogenic oligonucleotide probe and the target sequence, the quencher and fluorophore separate, resulting in dequenching of the fluorescent signal. International PCT Patent Application Publication WO 2005/079462, for example, describes a number of signaling probes that may be, and are preferably, used in the methods described. The distance required for currently known fluorophore and quencher to interact is about 20-100 A. In specific embodiments, the distance between a fluorophore and a quencher of a fluorogenic oligonucleotide probe is about 10, 15, 20, 30, 40, 50, 60, 70, 80, 90, 100, 110, 105, 110, 115, or 120 A, or any value in between.

A fluorogenic oligonucleotide probe can comprise more than one interacting pair of fluorophore and quencher. For example, a wavelength-shifting fluorogenic oligonucleotide has a first fluorophore and a second fluorophore that both interact with the quencher, and the two fluorophores are FRET donor and acceptor pairs. The moieties of the interacting pair of fluorophore and quencher may be attached to the termini of the fluorogenic oligonucleotide probe or may be attached within the nucleic acid sequence. Examples of moieties that may be incorporated internally into the sequence of the fluorogenic oligonucleotide probe include the quenchers: dabcyl dT, BHQ2 dT, and BHQ1 dT, and the fluorophores: fluorescein dT, Alexa dT, and Tamra dT. Multiple quenchers can be used to decrease or eliminate signal in the absence of target sequence. Examples of quenchers include but are not limited to DABCYL, EDAC, Cesium, p-xylene-bis-pyridinium bromide, Thallium and Gold nanoparticles.

Fluorogenic oligonucleotide probes may be DNA or RNA oligonucleotides. Fluorogenic oligonucleotide probes may be chemically synthesized using techniques known in the art. Chemical modifications of fluorogenic oligonucleotide probes have been described in the art, *e.g.,* see U.S. Patent 6,692,965 and International PCT Patent Application Publication No. WO 2005/079462. Both of these documents are incorporated herein by reference in their entirety.

A target sequence and fluorogenic oligonucleotide probe may be designed to be fully complementary or comprise complementary regions and non-complementary regions. In one embodiment, the two separate target sequence and probe are designed to be fully complementary to each other. In one embodiment, the target sequence and probe are designed to not be fully complementary to each other. In one embodiment, a target sequence and fluorogenic oligonucleotide probe form a mutually complementary region of 4 to 9, 5 to 6, 2 to 10, 10 to 40, or 40 to 400 continuous bps at each end. A target sequence and fluorogenic oligonucleotide probe may each contain 5-7, 8-10, 11-15, 16-22, more than 30, 3-10, 11-80, 81-200, or more than 200 nucleotides or modified nucleotides. Target sequence and fluorogenic oligonucleotide probe may have the same or a different number of nucleotides. In one embodiment, the 5' end of one strand (*e.g.*, target sequence and fluorogenic oligonucleotide probe) is offset from the other strand, or the 3' end of that strand is offset from the other strand, or both, wherein the offset is up to 5, up to 10, up to 20, or up to 30 nucleotides or modified nucleotides.

The region that hybridizes to the target sequence may be in the complementary regions, non-complementary regions of one or both strands or a combination thereof. More than one target sequence may be targeted by the same fluorogenic oligonucleotide probe.

In one embodiment, the fluorogenic oligonucleotide probe comprises at least two separate strands. In one embodiment, one strand has at least a quencher moiety on one terminus, and a fluorophore on an adjacent terminus of the other strand. In one embodiment, each of the 5' and 3' terminus of one strand has the same or a different fluorophore, and each of the 5' and 3' terminus of the other strand has the same or a different quencher moiety. In one embodiment, the 5' terminus of one strand has a fluorophore and the 3' terminus has a quencher moiety, and the 3' terminus of the other strand has the same or a different quencher moiety and the 5' terminus has the same or a different fluorophore.

Whether the target sequences are the same or different, the fluorogenic oligonucleotides may comprise different signal emitters, such as different colored fluorophores and the like so that expression of more than one target RNA, *e.g.,* each target RNA detecting a different gene of interest, may be separately detected. By way of illustration, the fluorogenic nucleotide that specifically detects a first mRNA of interest can comprise a red fluorophore, the probe that detects a second mRNA of interest can comprise a green fluorophore, and the probe that detects a third mRNA of interest can comprise a blue fluorophore. Those of skill in the art will be aware of other means for differentially detecting the expression of the three subunits with a fluorogenic oligonucleotide in a triply transfected cell.

In one embodiment, the fluorogenic oligonucleotide probes are designed to be complementary to either a portion of the RNA encoding the protein of interest, *e.g.*, the reporter, or to portions of the 5' or 3' UTRs. Even if the fluorogenic oligonucleotide designed to recognize a messenger RNA of interest is able to detect spuriously endogenously expressed target sequences, the proportion of these in comparison to the proportion of the sequence of interest produced by transfected cells is such that the sorter is able to discriminate the two cell types.

One may use Fluorescence-activated cell sorting (FACS) or other cell sorting methods (*i.e.,* MACS) to evaluate expression levels. Protocols for FACS are well known to the skilled person. Additional rounds of introducing fluorescent oligonucleotide probes may be used, for example, to determine if and to what extent the cells remain positive over time for anyone or more of the RNAs for which they were originally isolated.

### 5.6 Genetic Variability

In certain aspects, the invention provides for methods for identifying, isolating, and enriching plants and plant cells that are naturally occurring, but that expresses one or more genes of interest that confer one or more desirable traits. Thus, the methods described herein rely on the genetic variability and diversity of plants, microorganisms, and animals that exists in nature. Natural genetic variability and diversity may also be increased using natural processes known to a person skilled in the art. Any suitable methods for creating or increasing genetic variability and/or diversity may be performed on plants, microoogansims, and/or animals for the methods described herein.

For example, out-breeding, *i.e.,* breeding of stocks or individuals that are not closely related genetically, may be carried out to increase genetic diversity or variability.

In other aspects, genetic variability may be achieved by exposing plants, microorganisms, and/or animals to UV light. In other aspects, genetic variability may be achieved by exposing plants, microorganisms, and/or animals to x-rays (e.g., gamma-rays).

In other aspects, genetic variability may be achieved by exposing plants, microorganisms, and/or animals to EMS. In some aspects, genetic variability may be achieved by exposing plants, microorganisms, and/or animals to mutagens, carcinogens, or chemical agents. Non-limiting examples of such agents include deaminating agents such as nitrous acid, intercalating agents, and alkylating agents. Other non-limiting examples of such agents include bromine, sodium azide, and benzene.

In specific aspects, genetic variability may be achieved by exposing plants, microorganisms, and/or animals to undesirable growth conditions, *e.g.*, low oxygen, low nutrients, oxidative stress, low nitrogen, *et al.*

The duration of exposure to certain conditions or agents depend on the conditions or agents used. In some aspects, seconds or minutes of exposure is sufficient. In other aspects, exposure for a period of hours, days or months are necessary.

In certain aspects, a method that increases gene variability produces a mutation or alteration in a promoter region of a gene that leads to a change in the transcriptional regulation of the gene, *e.g.,* gene activation wherein the gene is more highly expressed than a gene with an unaltered promoter region. Generally, a promoter region includes genomic DNA sequence upstream of a transcription start site that regulate gene transcription, and may include the minimal promoter and/or enhancers and/or repressor regions. A promoter region may range from about 20 basepairs (bps) to about 10,000 bps or more. In specific aspects, a method that increases gene variability produces a mutation or alteration in an intron of a gene of interest that leads to a change in the transcriptional regulation of the gene, *e.g.,* gene activation wherein the gene is more highly expressed than gene with an unaltered intron. In certain aspects, untranscribed genomic DNA is modified. For example, promoter, enhancer, modifier, or repressor regions can be added, deleted, or modified. In these cases, transcription of a transcript that is under control of the modified regulatory region can be used as read-out. For example, if a repressor is deleted, the transcript of the gene that is repressed by the repressor is tested for increased transcription levels.

In certain aspects, a cell of an organism (*e.g.*, plant, microorganism, or animal) can be engineered by introducing a transgene that encodes an RNA of interest. In certain aspects, a promoter is introduced into the genome to activate the expression of a gene of interest via gene activation. See, *e.g.*, International Patent Application Publication No. WO 94/012650. In certain aspects, the transgene encodes a protein that enhances the property for which the cell was selected by a method as described above. In certain aspects, the genome of a cell or an organism can be mutated by site-specific mutagenesis or homologous recombination. In certain aspects, oligonucleotide- or triplex-mediated recombination can be employed. See, *e.g.,* Faruqi et al., 2000, Molecular and Cellular Biology 20:990-1000 and Schleifman et al., 2008, Methods Molecular Biology 435:175-90. In the methods of the invention, fluorogenic oligonucleotide probes or molecular beacons are used to select cells in which the genetic modification was successful, *i.e.,* cells in which the transgene or the gene of interest is expressed. To identify cells in which a mutagenic or homologous recombination event was successful, a fluorogenic oligonucleotide that specifically hybridizes to the mutagenized or recombined transcript can be used.

### 5.7 Bioinformatics

### LANDMARK EXPRESSION PROFILES

The methods are practiced using a database comprising a group of gene expression profiles (called herein landmark RNA expression profiles). Each landmark RNA expression profile comprises of measured amounts of a plurality of different cellular constituents in a cell of an organism (*e.g.*, plant, animal or microorganism) that is associated with one or more known traits, which can be desired traits or undesired traits (including but not limited to the various traits discussed herein). A landmark RNA expression profile can be obtained by any method known in the art, including but not limited to the methods discussed herein. A landmark RNA expression profile can also be obtained from information in public databases or in published literature. A database of the landmark RNA expression profiles can be stored on a computer readable storage medium. In specific aspects, the database contains at least 10 landmark RNA expression profiles, at least 50 landmark RNA expression profiles, at least 100 landmark RNA expression profiles, at least 500 landmark RNA expression profiles, at least 1,000 landmark RNA expression profiles, at least 10,000 landmark RNA expression profiles, or at least 50,000 landmark RNA expression profiles, each landmark RNA expression profile containing measured amounts of at least 2, at least 10, at least 100, at least 200, at least 500, at least 1,000, at least 2000, at least 2500, at least 7500, at least 10,000, at least 20,000, at least 25,000, or at least 35,000 components.

## MEASURES OF SIMILARITY

The RNA expression profile of an organism (*e.g.*, plant, animal, or microorganism) of interest and a landmark RNA expression profile may be compared through computation of a correlation between these RNA expression profiles, such as but not limited to computing a measure of similarity between these RNA expression profiles. The RNA expression profile of the organism (*e.g.*, plant, animal, or microorganism) of interest can comprise measured amounts of a plurality of different cellular constituents in a cell of the organism (*e.g.*, plant, animal, or microorganism). In a specific aspect, each respective organism (*e.g.*, plant, animal, or microorganism) corresponding to a landmark RNA expression profile can be associated with a known trait (as discussed above). In the foregoing aspect, the RNA expression profile of the organism (*e.g.*, plant, animal, or microorganism) of interest can be compared to the plurality of landmark RNA expression profiles to determine the one or more landmark RNA expression profiles that correlate with (*e.g.,* are most similar to) the RNA expression profile of the organism (*e.g.*, plant, animal, or microorganism) of interest, and the organism of interest can be characterized as having the known trait(s) associated with the respective organism corresponding to these one or more landmark RNA expression profiles.

In another specific aspect, the organism (*e.g.*, plant, animal, or microorganism) of interest can be associated with a known trait. In the foregoing aspect, the RNA expression profile of the organism of interest can be compared to the plurality of landmark RNA expression profiles to determine the one or more landmark RNA expression profiles that correlate with (*e.g.,* are most similar to) the RNA expression profile of the organism (*e.g.,* plant, animal, or microorganism) of interest, and the respective organism corresponding to these one or more landmark RNA expression profiles can be characterized as having the known trait associated with the organism of interest.

In certain aspects, a correlation can be computed between the RNA expression profile of the organism (*e.g.*, plant, animal, or microorganism) of interest and each landmark RNA expression profile of a plurality of landmark RNA expression profiles stored in a database. The correlation can be computed by comparing a measured amount in the RNA expression profile of the organism (*e.g.*, plant, animal, or microorganism) of interest to the corresponding measured amount in a landmark RNA expression profile. The RNA expression profile of the organism (*e.g.*, plant, animal, or microorganism) of interest can be deemed to correlate with the landmark RNA expression profile if the measured amounts in the landmark RNA expression profile are within about 2%, about 5%, about 8%, about 10%, about 12%, about 15%, about 20%, about 25%, about 30%, or about 35% of the measured amounts in the RNA expression profile of the organism (e.g., plant, animal, or microorganism).

The RNA expression profile of an organism (*e.g.*, plant, animal, or microorganism) of interest also can be deemed to be most similar to a landmark RNA expression profile if a measure of similarity between the RNA expression profile of the organism of interest and the landmark RNA expression profile is above a predetermined threshold. In specific aspects, the predetermined threshold can be determined as the value of the measure of similarity which indicates that the measured amounts in a landmark RNA expression profile are within about 2%, about 5%, about 8%, about 10%, about 12%, about 15%, about 20%, about 25%, about 30%, or about 35% of the measured amounts in the RNA expression profile of the organism (*e.g.*, plant, animal, or microorganism).

In some aspects, the RNA expression profile of the organism (*e.g.*, plant, animal, or microorganism) of interest can be expressed as a vector $p$,

$$p = [\, p_1, \ldots p_i, \ldots p_n ]$$

where $p_i$ is the measured amount of the $i$'th component, for example, the measured amount of the $i$'th cellular constituent. In specific aspects, $n$ is more than 2, more than 10, more than 100, more than 200, more than 500, more than 1000, more than 2000, more than 2500, more than 7500, more than 10,000, more than 20,000, more than 25,000, or more than 35,000. Each landmark RNA expression profile also can be expressed as a vector $p$. In computing a correlation, the measured amount of the $i$'th component in the vector representing the RNA expression profile for the organism (*e.g.*,

plant, animal, or microorganism) of interest can be compared to the corresponding measured amount of the $i$'th component of the vector representing a landmark RNA expression profile, for each component $i$ = 1 ... $n$. However, there are many ways in which a correlation can be computed. Indeed, any statistical method in the art for determining the probability that two datasets are related may be used in accordance with the methods of the present disclosure in order to identify whether there is a correlation between the RNA expression profile of an organism (*e.g.*, plant, animal, or microorganism) of interest and a landmark RNA expression profile. For example, the correlation between the RNA expression profile ($p_{i_1}$) of the organism (*e.g.,* plant, animal, or microorganism) of interest and each landmark *RNA* expression profile $(p_{i_2})$ can be computed using a similarity metric $sim(p_{i_1}, p_{i_2})$. One way to compute the similarity metric $sim(p_{i_1}, p_{i_2})$ is to compute the negative square of the Euclidean distance. In alternative aspects, metrics other than Euclidean distance can be used to compute $sim(p_{i_1}, p_{i_2})$, such as a Manhattan distance, a Chebychev distance, an angle between vectors, a correlation distance, a standardized Euclidean distance, a Mahalanobis *distance,* a squared Pearson correlation coefficient, or a Minkowski distance. In some aspects a Pearson correlation coefficient, a squared Euclidean distance, a Euclidean sum of squares, or squared Pearson correlation coefficients is used to determine similarity. Such metrics can be computed, for example, using SAS (Statistics Analysis Systems Institute, Cary, North Carolina) or S-Plus (Statistical Sciences, Inc., Seattle, Washington). Use of such metrics are described in Draghici, 2003, Data Analysis Tools for DNA Microarrays, Chapman & Hall, CRC Press London, chapter 11.

The correlation can also be computed based on ranks, where $x_i$ and $y_i$ are the ranks of the values of the measured amounts in ascending or descending numerical order. See for example, Conover, Practical Nonparametric Statistics, 2nd ed., Wiley, (1971). Shannon mutual information also can be used as a measure of similarity. See for example, Pierce, An Introduction To Information Theory: Symbols, Signals, and Noise, Dover, (1980).

**CLASSIFIERS**

Various classifiers known in the art can be trained according to the methods described in this application, and used to classify an organism (*e.g.*, plant, animal, or microorganism) of interest as having a trait. Algorithms are used to produce classifiers capable of predicting a trait of an organism (*e.g.*, plant, animal, or microorganism) of interest using an RNA expression profile of the organism (*e.g.*, plant, animal, or microorganism) of interest.

In a specific aspect, the classifier can be trained to classify an organism (*e.g.*, plant, animal, or microorganism) as to a trait using the measured amounts in the landmark RNA expression profile of a previously characterized organism (*e.g.*, plant, animal, or microorganism) and the known trait associated with that previously characterized organism (*e.g.,* plant, animal, or microorganism). Each respective organism (*e.g.,* plant, animal, or microorganism) corresponding to a landmark RNA expression profile can be associated with a known trait. The classifier may be an algorithm used for classification by applying a non-supervised or supervised learning algorithm to evaluate the measured amounts in the landmark RNA expression profile of a previously characterized organism (*e.g.*, plant, animal, or microorganism) and the known trait associated with that previously characterized organism (*e.g.*, plant, animal, or microorganism). The RNA expression profile of the organism (*e.g.,* plant, animal, or microorganism) of interest can be processed using the classifier to classify the organism (*e.g.*, plant, animal, or microorganism) of interest as to a trait. That is, the classifier can be used to classify the organism (*e.g.*, plant, animal, or microorganism) of interest as having one or more of the known traits associated with the plurality of landmark RNA expression profiles used to train the classifier.

In another specific aspect, the organism (*e.g.*, plant, animal, or microorganism) of interest can be associated with a known trait. In the foregoing aspect, the classifier can be trained to identify the one or more landmark RNA expression profiles that can be associated with the known trait of the organism (*e.g.*, plant, animal, or microorganism) of interest based on the RNA expression profile of the organism (*e.g.*, plant, animal, or microorganism) of interest. The classifier may be an algorithm used for classification by applying a non-supervised or supervised learning algorithm to evaluate the measured amounts in the landmark RNA expression profile of a respective organism (*e.g.*, plant, animal, or microorganism), and to identify the one or more landmark RNA expression profiles that can be associated with the known trait of the organism (*e.g.*, plant, animal, or microorganism) of interest based on the RNA expression profile of the organism (*e.g.*, plant, animal, or microorganism) of interest.

Any standard non-supervised or supervised learning technique known in the art can be used to generate a classifier. Below are non-limiting examples of non-supervised and supervised algorithms known in the art. Given the disclosure in this application, one of skill in the art will appreciate that other pattern classification or regression techniques and algorithms may be used for the classifier and the present disclosure encompasses all such techniques.

*Neural networks.* In some aspects, the classifier is learned using a neural network. A neural network is a two-stage regression or classification decision rule. A neural network has a layered structure that includes a layer of input units (and the bias) connected by a layer of weights to a layer of output units. For regression, the layer of output units typically includes just one output unit. However, neural networks can handle multiple quantitative responses in a seamless fashion.

In multilayer neural networks, there are input units (input layer), hidden units (hidden layer), and output units (output layer). There is, furthermore, a single bias unit that is connected to each unit other than the input units. Neural networks are described in Duda et al., 2001, Pattern Classification, Second Edition, John Wiley & Sons, Inc., New York; and Hastie et al., 2001, The Elements of Statistical Learning, Springer-Verlag, New York. Neural networks are also described in Draghici, 2003, Data Analysis Tools for DNA Microarrays, Chapman & Hall/CRC; and Mount, 2001, Bioinformatics: sequence and genome analysis, Cold Spring Harbor Laboratory Press, Cold Spring Harbor, New York. What are discussed below are some exemplary forms of neural networks.

The basic approach to the use of neural networks is to start with an untrained network, present a training pattern to the input layer, and to pass signals through the net and determine the output at the output layer. These outputs are then compared to the target values; any difference corresponds to an error. For classification, this error can be either squared error or cross-entropy (deviation). *See,* for example, Hastie et al., 2001, The Elements of Statistical Learning, Springer-Verlag, New York.

Three commonly used training protocols are stochastic, batch, and on-line. In stochastic training, patterns are chosen randomly from the training set and network weights are updated for each pattern presentation. Multilayer nonlinear networks trained by gradient descent methods such as stochastic back-propagation perform a maximum-likelihood estimation of weight values in the classifier defined by the network topology. In batch training, all patterns are presented to the network before learning takes place. Typically, in batch training, several passes are made through the training data. In online training, each pattern is presented once and only once to the net.

A recurrent problem in the use of three-layer networks is the optimal number of hidden units to use in the network. The number of inputs and outputs of a three-layer network are determined by the problem to be solved. In the present disclosure, the number of inputs for a given neural network will equal the number of biomarkers selected from Y. The number of output for the neural network will typically be just one. If too many hidden units are used in a neural network, the network will have too many degrees of freedom and if trained too long, there is a danger that the network will overfit the data. If there are too few hidden units, the training set cannot be learned. Generally speaking, however, it is better to have too many hidden units than too few. With too few hidden units, the classifier might not have enough flexibility to capture the nonlinearities in the date; with too many hidden units, the extra weight can be shrunk towards zero if appropriate regularization or pruning, as described below, is used. In typical aspects, the number of hidden units is somewhere in the range of 5 to 100, with the number increasing with the number of inputs and number of training cases.

*Clustering.* In some aspects, the classifier is learned using clustering. In some aspects, select components $i$ of the vectors representing the landmark RNA expression profiles are used to cluster the RNA expression profiles. In some aspects, prior to clustering, the measured amounts are normalized to have a mean value of zero and unit variance.

Landmark RNA expression profiles that exhibit similar patterns of measured amounts across the training population will tend to cluster together. A particular combination of measured amounts of components i can be considered to be a good classifier in this aspect of the disclosure when the vectors form clusters for a particular known trait. Clustering is described on pages 211-256 of Duda and Hart, Pattern Classification and Scene Analysis, 1973, John Wiley & Sons, Inc., New York, (hereinafter "Duda 1973"). As described in Section 6.7 of Duda 1973, the clustering problem is described as one of finding natural groupings in a dataset. To identify natural groupings, two issues are addressed. First, a way to measure similarity (or dissimilarity) between two RNA expression profiles is determined. This metric (similarity measure) is used to ensure that the RNA expression profiles in one cluster are more like one another than they are to other RNA expression profiles. Second, a mechanism for partitioning the data into clusters using the similarity measure is determined.

Similarity measures are discussed in Section 6.7 of Duda 1973, where it is stated that one way to begin a clustering investigation is to define a distance function and to compute the matrix of distances between pairs of RNA expression profiles. If distance is a good measure of similarity, then the distance between RNA expression profiles in the same cluster will be significantly less than the distance between RNA expression profiles in different clusters. However, as stated on page 215 of Duda 1973, clustering does not require the use of a distance metric. For example, a nonmetric similarity function s(x, x') can be used to compare two vectors x and x'. Conventionally, s(x, x') is a symmetric function whose value is large when x and x' are somehow "similar". An example of a nonmetric similarity function s(x, x') is provided on page 216 of Duda 1973.

Once a method for measuring "similarity" or "dissimilarity" between points in a dataset has been selected, clustering requires a criterion function that measures the clustering quality of any partition of the data. Partitions of the data set that extremize the criterion function are used to cluster the data. See page 217 of Duda 1973. Criterion functions are discussed in Section 6.8 of Duda 1973. More recently, Duda et al., Pattern Classification, 2nd edition, John Wiley & Sons, Inc. New York, has been published. Pages 537-563 describe clustering in detail. More information on clustering techniques can be found in Kaufinan and Rousseeuw, 1990, Finding Groups in Data: An Introduction to Cluster Analysis, Wiley, New York, NY; Everitt, 1993, Cluster analysis (3d ed.), Wiley, New York, NY; and Backer, 1995, Computer-Assisted Reasoning in Cluster Analysis, Prentice Hall, Upper Saddle River, New Jersey. Particular exemplary clustering techniques that can be used in the present disclosure include, but are not limited to, hierarchical clustering (agglomerative clustering using nearest-neighbor algorithm, farthest-neighbor algorithm, the average linkage algorithm, the centroid algorithm, or the sum-of-squares algorithm), k-means clustering, fuzzy k-means clustering algorithm, and Jarvis-Patrick clustering.

*Principal* component *analysis.* In some aspects, the classifier is learned using principal component analysis. Principal component analysis is a classical technique to reduce the dimensionality of a data set by transforming the data to a new set of variable (principal components) that summarize the features of the data. See, for example, Jolliffe, 1986, Principal Component Analysis, Springer, New York. Principal component analysis is also described in Draghici, 2003, Data Analysis Tools for DNA Microarrays, Chapman & Hall/CRC. What follows is non-limiting examples of principal components analysis.

Principal components (PCs) are uncorrelated and are ordered such that the $k^{th}$ PC has the $k^{th}$ largest variance among PCs. The $k^{th}$ PC can be interpreted as the direction that maximizes the variation of the projections of the data points such that it is orthogonal to the first $k - 1$ PCs. The first few PCs capture most of the variation in the data set. In contrast, the last few PCs are often assumed to capture only the residual 'noise' in the data.

In one approach to using PCA to learn a classifier, vectors representing the landmark RNA expression profiles can be constructed in the same manner described for clustering above. In fact, the set of vectors, where each vector represents a landmark RNA expression profile, can be viewed as a matrix. In some aspects, this matrix is represented in a Free-Wilson method of qualitative binary description of monomers (Kubinyi, 1990, 3D QSAR in drug design theory methods and applications, Pergamon Press, Oxford, pp 589-638), and distributed in a maximally compressed space using PCA so that the first principal component (PC) captures the largest amount of variance information possible, the second principal component (PC) captures the second largest amount of all variance information, and so forth until all variance information in the matrix has been considered.

Then, each of the vectors, where each vector represents a member of the training population (such as the landmark RNA expression profiles), is plotted. Many different types of plots are possible. In some aspects, a one-dimensional plot is made. In this one-dimensional plot, the value for the first principal component from each of the members of the training population is plotted. In this form of plot, the expectation is that RNA expression profiles corresponding to a trait will cluster in one range of first principal component values and profiles corresponding to another trait will cluster in a second range of first principal component values.

In some aspects, the members of the training population are plotted against more than one principal component. For example, in some aspects, the members of the training population are plotted on a two-dimensional plot in which the first dimension is the first principal component and the second dimension is the second principal component.

*Nearest neighbor analysis.* In some aspects, the classifier is learned using nearest neighbor analysis. Nearest neighbor classifiers are memory-based and require no classifier to be fit. Given a query point $x_0$, the $k$ training points $x_{(r)}$, $r$, ..., $k$ closest in distance to $x_0$ are identified and then the point $x_0$ is classified using the $k$ nearest neighbors. Ties can be broken at random. In some aspects, Euclidean distance in feature space is used to determine distance as:

$$d_{(i)} = \left\| x_{(i)} - x_{\mathbf{o}} \right\|.$$

Typically, when the nearest neighbor algorithm is used, the abundance data from *Y* used to compute the linear discriminant is standardized to have mean zero and variance 1. In the present disclosure, the members of the training population are randomly divided into a training set and a test set. For example, in one aspect, two thirds of the members of the training population are placed in the training set and one third of the members of the training population are placed in the test set. A select combination of vector components *i* represents the feature space into which members of the test set are plotted. Next, the ability of the training set to correctly characterize the members of the test set is computed. In some aspects, nearest neighbor computation is performed several times for a given combination of vector components *i*. In each iteration of the computation, the members of the training population are randomly assigned to the training set and the test set.

The nearest neighbor rule can be refined to deal with issues of unequal class priors, differential misclassification costs, and feature selection. Many of these refinements involve some form of weighted voting for the neighbors. For more information on nearest neighbor analysis, see Duda, Pattern Classification, Second Edition, 2001, John Wiley & Sons, Inc; and Hastie, 2001, The Elements of Statistical Learning, Springer, New York.

*Linear discriminant* analysis. In some aspects, the classifier is learned using linear discriminant analysis. Linear discriminant analysis (LDA) attempts to classify a subject into one of two categories based on certain object properties. In other words, LDA tests whether object attributes measured in an experiment predict categorization of the objects. LDA typically requires continuous independent variables and a dichotomous categorical dependent variable. In the present disclosure, the abundance values for the select combinations of vector components *i* across a subset of the training population serve as the requisite continuous independent variables. The trait subgroup classification (a trait) of each of the members of the training population serves as the dichotomous categorical dependent variable.

LDA seeks the linear combination of variables that maximizes the ratio of between-group variance and within-group variance by using the grouping information. Implicitly, the linear weights used by LDA depend on how the measured amount of a vector component i across the training set separates in the groups of the trait. In some aspects, LDA is applied to the data matrix of the members in the training population. Then, the linear discriminant of each member of the training population is plotted. Ideally, those members of the training population representing a trait will cluster into one range of linear discriminant values (for example, negative) and those members of the training population representing another trait will cluster into a second range of linear discriminant values (for example, positive). The LDA is considered more successful when the separation between the clusters of discriminant values is larger. For more information on linear discriminant analysis, see Duda, Pattern Classification, Second Edition, 2001, John Wiley & Sons, Inc; and Hastie, 2001, The Elements of Statistical Learning, Springer, New York; and Venables & Ripley, 1997, Modern Applied Statistics with s-plus, Springer, New York.

*Quadratic* discriminant *analysis.* In some aspects, the classifier is learned using quadratic discriminant analysis. Quadratic discriminant analysis (QDA) takes the same input parameters and returns the same results as LDA. QDA uses quadratic equations, rather than linear equations, to produce results. LDA and QDA are interchangeable, and which to use is a matter of preference and/or availability of software to support the analysis. Logistic regression takes the same input parameters and returns the same results as LDA and QDA.

*Support vector* machine. In some aspects, the classifier is learned using a support vector machine. SVMs are described, for example, in Cristianini and Shawe-Taylor, 2000, An Introduction to Support Vector Machines, Cambridge University Press, Cambridge; Boser et al., 1992, "A training algorithm for optimal margin classifiers," in Proceedings of the 5th Annual ACM Workshop on Computational Learning Theory, ACM Press, Pittsburgh, PA, pp. 142-152; Vapnik, 1998, Statistical Learning Theory, Wiley, New York; Mount, 2001, Bioinformatics: sequence and genome analysis, Cold Spring Harbor Laboratory Press, Cold Spring Harbor, New York, Duda, Pattern Classification, Second Edition, 2001, John Wiley & Sons, Inc.; and Hastie, 2001, The Elements of Statistical Learning, Springer, New York; and Furey et al., 2000, Bioinformatics 16, 906-914. When used for classification, SVMs separate a given set of binary labeled data training data with a hyper-plane that is maximally distant from them. For cases in which no linear separation is possible, SVMs can work in combination with the technique of 'kernels', which automatically realizes a non-linear mapping to a feature space. The hyper-plane found by the SVM in feature space corresponds to a non-linear decision boundary in the input space. For more information on support vector machines see, for example, Furey et al., 2000, Bioinformatics 16, page 906-914.

*Decision tree.* In one aspect the classifier is a decision tree. Decision trees are described generally in Duda, 2001, Pattern Classification, John Wiley & Sons, Inc., New York, pp. 395-396, which is hereby incorporated herein by reference. One specific algorithm that can be used is a classification and regression tree (CART). Other specific algorithms for include, but are not limited to, ID3, C4.5, MART, and Random Forests. CART, ID3, and C4.5, each described in Duda, 2001, Pattern Classification, John Wiley & Sons, Inc., New York, pp. 396-408 and pp. 411-412. CART, MART, and C4.5 are also described in Hastie et al., 2001, The Elements of Statistical Learning, Springer-Verlag, New York, Chapter 9. The Random Forests technique is described in Breiman, 1999, "Random Forests - Random Features," Technical Report 567, Statistics Department, University of California at Berkeley, September 1999.

In addition to univariate decision trees in which each split is based on measured amounts for a corresponding vector component *i*, or the relative measured amounts of vector components *i*, the classifier can be a multivariate decision tree. In such a multivariate decision tree, some or all of the decisions actually comprise a linear combination of measured amounts for a plurality of vector components *i*. Multivariate decision trees are described in Duda, 2001, Pattern Classification, John Wiley & Sons, Inc., New York, pp. 408-409.

*Multivariate* adaptive *regression splines.* Another approach that can be used to learn a pairwise probability function $g_{pq}(X, W_{pq})$ uses multivariate adaptive regression splines (MARS). MARS is an adaptive procedure for regression, and is well suited for the high-dimensional problems addressed by the present disclosure. MARS can be viewed as a generalization of stepwise linear regression or a modification of the CART method to improve the performance of CART in the regression setting. MARS is described in Hastie et al., 2001, The Elements of Statistical Learning, Springer-Verlag, New York, pp. 283-295.

*Centroid* classifier *techniques.* In one aspect a nearest centroid classifier technique is used. Such a technique computes, for the different traits, a centroid given by the average measured amounts of vector components *i* in the training population (landmark RNA expression profiles), and then assigns vector representing the organism (*e.g.*, plant, animal, or microorganism) of interest to the class whose centroid is nearest. This approach is similar to k-means clustering except clusters are replaced by known classes. An example implementation of this approach is the Prediction Analysis of Microarray, or PAM. See, for example, Tibshirani et al., 2002, Proceedings of the National Academy of Science USA 99; 6567-6572.

*Regression.* In some aspects, the classifier is a regression classifier, such as a logistic regression classifier. Such a regression classifier includes a coefficient for each of the RNA expression profiles used to construct the classifier. In such aspects, the coefficients for the regression classifier are computed using, for example, a maximum likelihood approach. In such a computation, the measured amounts of vector components i are used.

*Other methods.* In some aspects, the classifier is learned using k-nearest neighbors (k-NN), an artificial neural network (ANN), a parametric linear equation, a parametric quadratic equation, a naive Bayes analysis, linear discriminant analysis, a decision tree, or a radial basis function.

## APPARATUS, COMPUTER AND COMPUTER PROGRAM PRODUCT IMPLEMENTATIONS

The present disclosure can be implemented as a computer program product that comprises a computer program mechanism embedded in a computer-readable storage medium. Further, any of the methods of the present disclosure can be implemented in one or more computers or other forms of apparatus. Examples of apparatus include but are not limited to, a computer, and a measuring device (for example, an assay reader or scanner). Further still, any of the methods of the present disclosure can be implemented in one or more computer program products. Some aspects of the present disclosure provide a computer program product that encodes any or all of the methods disclosed in this application. Such methods can be stored on a CD-ROM, DVD, magnetic disk storage product, or any other computer-readable data or program storage product. Such computer readable storage media are intended to be tangible, physical objects (as opposed to carrier waves). Such methods can also be embedded in permanent storage, such as ROM, one or more programmable chips, or one or more application specific integrated circuits (ASICs). Such permanent storage can be localized in a server, 802.11 access point, 802.11 wireless bridge/station, repeater, router, mobile phone, or other electronic devices. Such methods encoded in the computer program product can also be distributed electronically, via the Internet or otherwise, by transmission of a computer data signal (in which the software modules are embedded) either digitally or on a carrier wave (it will be clear that such use of carrier wave is for distribution, not storage).

Some aspects of the present disclosure provide a computer program product that contains any or all of the program modules shown in Fig. 1. These program modules can be stored on a CD-ROM, DVD, magnetic disk storage product, or any other computer-readable data or program storage product. The program modules can also be embedded in permanent storage, such as ROM, one or more programmable chips, or one or more application specific integrated circuits (ASICs). Such permanent storage can be localized in a server, 802.11 access point, 802.11 wireless bridge/station, repeater, router, mobile phone, or other electronic devices. The software modules in the computer program product can also be distributed electronically, via the Internet or otherwise, by transmission of a computer data signal (in which the software modules are embedded) either digitally or on a carrier wave.

In a specific aspect, the computer program provides for outputting a result of the claimed method to a user, a user interface device, a computer readable storage medium, a monitor, a local computer, or a computer that is part of a network. Such computer readable storage media are intended to be tangible, physical objects (as opposed to carrier waves).

### 5.8 Screening Assays

In certain aspects, provided herein are methods for identifying genes that are related to a particular phenotype or trait in an organism. In certain aspects, genetic variability of the organism is generated as discussed in section 5.6 "Genetic Variability;" gene expression is determined (see, *e.g.,* Section 5.5); the phenotype or trait is determined; the gene expression profile is correlated with the phenotype or trait. Methods for determining the phenotype or trait of a cell or organism or for correlating a gene expression profile with a phenotype or trait are known to a skilled person in the art. If the organism is a multicellular organism, such as a plant or animal, the method can include generation of a part of the organism or the organism from the cell (see Sections 5.2.3.2 or 5.4).

### 5.9 Cultivation of Cells

In certain aspects, a plurality of separate cell cultures is maintained under substantially identical conditions a discussed below. In certain aspects, separate cell cultures are maintained in parallel before and/or after identification and selection of cells as discussed above.

In certain aspects, a standardized maintenance schedule is used. Another advantageous feature of the method is that large numbers of individual cultures can be maintained simultaneously, so that a cell with a desired set of traits may be identified even if extremely rare. For those and other reasons, according to the disclosure, the plurality of separate cell cultures are cultured using automated cell culture methods so that the conditions are substantially identical for each well. Automated cell culture prevents the unavoidable variability inherent to manual cell culture.

Any automated cell culture system may be used in the method of the disclosure. A number of automated systems are commercially available and will be well-known to the skilled worker. In some aspects, these systems could be adapted for use to automate or standardize the culture of multiple separate cultures of cells or cell lines. In some aspects, these systems could be adapted for use to automate or standardize the culture of multiple separate cultures of cells or cell lines under substantially identical conditions. In some aspects, these systems could be adapted for use to automate or standardize the parallel culture of multiple separate cultures of cells or cell lines under substantially identical conditions. In some aspects, these systems could be adapted for use to automate or standardize the culture of multiple separate cultures of cells or cell lines such that the expression levels of at least 1, 2, 3, 4, 5, 6, 7, 8, 9, 10, 11, 12, 13, 14, 15, 16, 17, 18 , 19, 20, 21, 22, 23, 24, 25, 26, 27, 28, 29, 30, 35, 40, 45, 50, 100, 250, 500, 750, 1000, 2500, 5000, 7500, 10000, 15000 genes are maintained during culture. In some aspects, these systems could be adapted for use to automate or standardize the culture of multiple separate cultures of cells or cell lines such that a specific RNA or protein of interest is stably expressed by the cells or cell lines.

In some aspects, the automated system is a robotic system. Preferably, the system includes independently moving channels, a multichannel head (for instance a 5 96-tip head) and a gripper or cherry-picking arm and a HEPA filtration device to maintain sterility during the procedure. The number of channels in the pipettor should be suitable for the format of the culture. Convenient pipettors have, *e.g.,* 96 or 384 channels. Such systems are known and are commercially available. For example, a MICROLAB STAR™ instrument (Hamilton) may be used in the methods of the disclosure. The automated system should be able to perform a variety of desired cell culture tasks. Such tasks will be known by a person of skill in the art. They include but are not limited to: removing media, replacing media, adding reagents, cell washing, removing wash solution, adding a dispersing agent, removing cells from a culture vessel, adding cells to a culture vessel and the like.

The cultivation of a cell or cell line of the disclosure may include any number of separate cell cultures. However, the advantages provided by the method increase as the number of cells increases. In certain aspects, the number of separate cell cultures can be two or more but more advantageously is at least 3, 4, 5, 6, 7, 8, 9, 10 or more separate cell cultures, for example, at least 12, at least 15, at least 20, at least 24, at least 25, at least 30, at least 35, at least 40, at least 45, at least 48, at least 50, at least 75, at least 96, at least 100, at least 200, at least 300, at least 384, at 25 least 400, at least 500, at least 1000, at least 10,000, at least 100,000, at least 500,000 or more.

### 5.10 **Kits**

The present disclosure also provides any of the above-mentioned compositions in kits, optionally including instructions for use of the composition. The "kit" typically defines a package including one or more compositions described herein and the instructions. In certain aspects, provided herein are kits comprising a plurality of fluorogenic oligonucleotide probes, each one capable of detecting a gene of interest. In certain aspects, the gene of interest is a plant gene as set forth in Section **5**.**2**.**2** above. In certain aspects, the plurality of fluorogenic probes comprises at least 1, 2, 3, 4, 5, 6, 7, 8, 9, 10, 15, 20, 25, 30, 35, 40, 45, 50, 60, 70, 80, 90, 100, 150, 200, 250, 300, 400, 500, 600, 700, 800, 900, or at least 1000 fluorogenic oligonucleotides each capable of hybridizing to a gene of interest.

### Claims

1. A method for identifying a plant cell expressing an RNA of interest, wherein the method comprises:

   (a) introducing into a plant cell a fluorogenic oligonucleotide capable of
   detecting the RNA of interest, wherein the fluorogenic oligonucleotide is transferred into the plant cell by using polyethylene glycol (PEG); and
   (b) determining whether the RNA of interest is present in the plant cell;
   and optionally
   (c) quantifying the level of the RNA of interest,

   wherein the plant cell is a protoplast.

2. A method for identifying a plant cell with a desired RNA expression profile, wherein the method comprises:

   (a) introducing into a plant cell a plurality of fluorogenic oligonucleotides
   each capable of detecting an RNA of interest, wherein the plurality of fluorogenic oligonucleotides are transferred into the plant cell by using polyethylene glycol (PEG); and
   (b) quantifying the RNA levels detected by the plurality of fluorogenic
   oligonucleotides,

   wherein the plant cell is a protoplast.

3. The method of claim 1 or 2, wherein the method further comprises comparing the quantified RNA levels with the RNA levels in a reference cell.

4. The method of claim 1 or 2, wherein the method further comprises treating the plant cell with UV light, natural light, or a chemical agent.

5. The method of claim 2, wherein the plurality of fluorogenic probes comprises at least 2, 3, 4, 5, 6, 7, 8, 9, 10, 15, 20, 25, 30, 35, 40, 45, 50, 60, 70, 80, 90, 100, 125, 150, 175, 200, 250, 300, 350, 500, 600, 700, 800, 900, or at least 1000 fluorogenic probes.

6. The method of claim 1 or 2, wherein the RNA of interest is encoded by a gene selected from the group consisting of genes listed in Table 2.

7. The method of claim 1 or 2, wherein the plant cell has been derived from seedling.

8. The method of claim 7, wherein the seedling has been treated with UV light, natural light, or a chemical agent.

9. The method of claim 1 or 2, wherein the plant cell is a cell of a gymnosperm or an angiosperm.

10. The method of claim 1, 2 or 9, wherein the plant cell is a cell of a plant selected from the group consisting of oak, maple, pine, spruce, sequoia and cedar.

11. The method of claim 1, 2 or 9, wherein the plant cell is a cell of a plant selected from the group consisting of tobacco, tea, coffee, cocoa, wheat, barley, millet, corn, legumes, soybean, sugar cane, wild rice and rice.

12. The method of claim 1, 2 or 9, wherein the plant cell is a cell of a medicinal or horticultural plant.

13. A method for generating an improved plant, wherein the method comprises:

   (a) identifying a plant cell using the method of claim 1 or 2; and
   (b) generating an improved plant from the plant cell identified in step (a),

   wherein the plant cell is a protoplast.

14. The method of claim 1, wherein the determining of whether the RNA of interest is present in the plant cell is carried out using RT-PCR, real time PCR, immunoblotting, flow cytometry, or enzyme-linked immunosorbent assay (ELISA).

15. The method of claim 1 or 2, wherein the method further comprises isolation of the plant protoplast.

**Patentansprüche**

1. Verfahren für die Identifizierung einer Pflanzenzelle, die eine interessierende RNA exprimiert, wobei das Verfahren Folgendes umfasst:

   (a) Einführen eines fluorogenen Oligonukleotids, das die interessierende RNA nachweisen kann, in eine Pflanzenzelle, wobei das fluorogene Oligonukleotid in die Pflanzenzelle unter Verwendung von Polyethylenglycol (PEG) übertragen wird; und
   (b) Bestimmen, ob die interessierende RNA in der Pflanzenzelle vorliegt; und optional
   (c) Quantifizieren der Menge der interessierenden RNA,

   wobei die Pflanzenzelle ein Protoplast ist.

2. Verfahren für die Identifizierung einer Pflanzenzelle mit einem gewünschten RNA-Expressionsprofil, wobei das Verfahren Folgendes umfasst:

   (a) Einführen einer Vielzahl von fluorogenen Oligonukleotiden, die jeweils eine interessierende RNA nachweisen können, in eine Pflanzenzelle, wobei die Vielzahl von fluorogenen Oligonukleotiden in die Pflanzenzelle unter Verwendung von Polyethylenglycol (PEG) übertragen wird; und

(b) Quantifizieren der RNA-Mengen, die von der Vielzahl von fluorogenen Oligonukleotiden nachgewiesen werden,

wobei die Pflanzenzelle ein Protoplast ist.

3. Verfahren nach Anspruch 1 oder 2, wobei das Verfahren weiter das Vergleichen der quantifizierten RNA-Mengen mit den RNA-Mengen in einer Referenzzelle umfasst.

4. Verfahren nach Anspruch 1 oder 2, wobei das Verfahren weiter das Behandeln der Pflanzenzelle mit UV-Licht, natürlichem Licht oder einem chemischen Mittel umfasst.

5. Verfahren nach Anspruch 2, wobei die Vielzahl von fluorogenen Sonden mindestens 2, 3, 4, 5, 6, 7, 8, 9, 10, 15, 20, 25, 30, 35, 40, 45, 50, 60, 70, 80, 90, 100, 125, 150, 175, 200, 250, 300, 350, 500, 600, 700, 800, 900 oder mindestens 1000 fluorgene Sonden umfasst.

6. Verfahren nach Anspruch 1 oder 2, wobei die interessierende RNA von einem Gen kodiert wird, das aus der Gruppe ausgewählt ist, die aus Genen besteht, die in Tabelle 2 aufgelistet sind.

7. Verfahren nach Anspruch 1 oder 2, wobei die Pflanzenzelle von einem Keimling abstammt.

8. Verfahren nach Anspruch 7, wobei der Keimling mit UV-Licht, natürlichem Licht oder einem chemischen Mittel behandelt wurde.

9. Verfahren nach Anspruch 1 oder 2, wobei die Pflanzenzelle eine Zelle eines Nacktsamers oder eines Bedecktsamers ist.

10. Verfahren nach Anspruch 1, 2 oder 9, wobei die Pflanzenzelle eine Zelle einer Pflanze ist, die aus der Gruppe ausgewählt ist, die aus Eiche, Ahorn, Kiefer, Fichte, Sequoia und Zeder besteht.

11. Verfahren nach Anspruch 1, 2 oder 9, wobei die Pflanzenzelle eine Zelle einer Pflanze ist, die aus der Gruppe ausgewählt ist, die aus Folgenden besteht: Tabak, Tee, Kaffee, Kakao, Weizen, Gerste, Hirse, Mais, Hülsenfrüchten, Sojabohne, Zuckerrohr, Wildreis und Reis.

12. Verfahren nach Anspruch 1, 2 oder 9, wobei die Pflanzenzelle eine Zelle einer Heil- oder Gartenkulturpflanze ist.

13. Verfahren für die Erzeugung einer verbesserten Pflanze, wobei das Verfahren Folgendes umfasst:

(a) Identifizieren einer Pflanzenzelle unter Verwendung des Verfahrens nach Anspruch 1 oder 2; und
(b) Erzeugen einer verbesserten Pflanze aus der Pflanzenzelle, die im Schritt (a) identifiziert wurde,

wobei die Pflanzenzelle ein Protoplast ist.

14. Verfahren nach Anspruch 1, wobei das Bestimmen, ob die interessierende RNA in der Pflanzenzelle vorliegt, unter Verwendung von RT-PCR, Echtzeit-PCR, Immunoblotting, Durchflusszytometrie oder Enzyme-Linked Immunosorbent Assay (ELISA) durchgeführt wird.

15. Verfahren nach Anspruch 1 oder 2, wobei das Verfahren weiter die Isolierung des Pflanzenprotoplasten umfasst.

**Revendications**

1. Méthode d'identification d'une cellule végétale exprimant un ARN d'intérêt, où la méthode comprend :

(a) l'introduction dans une cellule végétale d'un oligonucléotide fluorogénique capable de détecter l'ARN d'intérêt, où l'oligonucléotide fluorogénique est transféré dans la cellule végétale par l'utilisation de polyéthylène glycol (PEG) ; et
(b) la détermination du fait que l'ARN d'intérêt est présent ou non dans la cellule végétale ; et éventuellement
(c) la quantification du taux de l'ARN d'intérêt,

où la cellule végétale est un protoplaste.

2. Méthode d'identification d'une cellule végétale présentant un profil d'expression d'ARN désiré, où la méthode comprend :

(a) l'introduction dans une cellule végétale d'une pluralité d'oligonucléotides fluorogéniques capables chacun de détecter un ARN d'intérêt, où la pluralité d'oligonucléotides fluorogéniques est transférée dans la cellule végétale par l'utilisation de polyéthylène glycol (PEG) ; et
(b) la quantification des taux d'ARN détectés par la pluralité d'oligonucléotides fluorogéniques,

où la cellule végétale est un protoplaste.

3. Méthode selon la revendication 1 ou 2, où la méthode comprend en outre la comparaison des taux d'ARN quantifiés avec les taux d'ARN dans une cellule de référence.

4. Méthode selon la revendication 1 ou 2, où la méthode comprend en outre le traitement de la cellule végétale par de la lumière UV, de la lumière naturelle ou un agent chimique.

5. Méthode selon la revendication 2, où la pluralité des sondes fluorogéniques comprend au moins, 2, 3, 4, 5, 6, 7, 8, 9, 10, 15, 20, 25, 30, 35, 40, 45, 50, 60, 70, 80, 90, 100, 125, 150, 175, 200, 250, 300, 350, 500, 600, 700, 800, 900 ou au moins 1000 sondes fluorogéniques.

6. Méthode selon la revendication 1 ou 2, où l'ARN d'intérêt est codé par un gène choisi dans le groupe constitué par les gènes énumérés dans le Tableau 2.

7. Méthode selon la revendication 1 ou 2, où la cellule végétale a été dérivée de plantules.

8. Méthode selon la revendication 7, où la plantule a été traitée par de la lumière UV, de la lumière naturelle ou un agent chimique.

9. Méthode selon la revendication 1 ou 2, où la cellule végétale est une cellule d'une gymnosperme ou d'une angiosperme.

10. Méthode selon la revendication 1, 2 ou 9, où la cellule végétale est une cellule d'une plante choisie dans le groupe constitué par le chêne, l'érable, le pin, l'épinette, le séquoia et le cèdre.

11. Méthode selon la revendication 1, 2 ou 9, où la cellule végétale est une cellule d'une plante choisie dans le groupe constitué par le tabac, le thé, le café, le cacao, le blé, l'orge, le millet, le maïs, les légumineuses, le soja, la canne à sucre, le riz sauvage et le riz.

12. Méthode selon la revendication 1, 2 ou 9, où la cellule végétale est une cellule d'une plante médicinale ou horticole.

13. Méthode de génération d'une plante améliorée, où la méthode comprend :

(a) l'identification d'une cellule végétale à l'aide de la méthode selon la revendication 1 ou 2 ; et
(b) la génération d'une plante améliorée à partir de la cellule végétale identifiée dans l'étape (a),

où la cellule végétale est un protoplaste.

14. Méthode selon la revendication 1, où la détermination du fait que l'ARN d'intérêt est présent ou non dans la cellule végétale est effectuée par RT-PCR, PCR en temps réel, immuno-buvardage, cytométrie en flux, ou une méthode immunoenzymatique (ELISA).

15. Méthode selon la revendication 1 ou 2, où la méthode comprend en outre l'isolement du protoplaste végétal.

Wide area network

Power Source

CPU

Communications Circuitry

Operating System

File system

Landmark RNA expression profiles data store

Landmark RNA expression profile 1

Plant or microorganism 1 having Trait 1

Measured amount 1

Measured amount 2

⋮

Measured amount N

Landmark RNA expression profile 2

⋮

Landmark RNA expression profile M

Plant or microorganism M having Trait M

Measured amount 1

Measured amount 2

⋮

Measured amount N

Data preprocessing module

Similarity computation module

Classifier training module

Trait determination module

Plant or microroganism RNA expression profile

Controller

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2005005080 W
- WO 2005079462 A
- US 6692965 B, Shekdar
- WO 08115487 A
- WO 08118394 A
- WO 9639805 A
- US 7566815 B
- US 20030215798 A1
- WO 9209696 A
- WO 9400583 A
- EP 331083 A
- EP 175966 A
- EP 290395 A
- WO 8706614 A
- DE 4005152
- WO 9012096 A
- US 4684611 A

- US 5100792 A
- EP 4448821 A
- EP 434616 A
- US 4945050 A, Sanford
- US 5240855 A, Tomes
- US 5322783 A, Buising
- US 5324646 A
- WO 9214828 A
- EP 270355 A
- EP 0116718 A
- US 5563055 A
- EP 486234 A
- EP 486233 A
- US 4407956 A
- WO 2009049274 A
- WO 2005079462A2 A
- WO 94012650 A

### Non-patent literature cited in the description

- **LODISH et al.** Molecular Cell Biology. W.H. Freeman and Company, 2000
- **VITOUSEK et al.** *Biogeochemistry,* 2004, 13
- **LOPEZ-BUCIO et al.** *Curr Opin Plant Biol,* 2003, vol. 6, 280-7
- **MALAMY et al.** *Plant Cell Environ,* 2005, vol. 28, 67-77
- **WALCH-LIU et al.** *Ann Bot (Lond),* 2006, vol. 97, 875-81
- **SHAISTA NAQVI et al.** Transgenic multivitamin corn through biofortificatio n of endosperm with three vitamins representing three distinct metabolic pathways. *PNAS,* 27 April 2009
- U.S. Food and Drug Administratio n Center for Food Safety and Applied Nutrition. Biotechnology of Food. FDA, 18 May 1994
- **JOHN M. RILEY.** *CRFG J.,* 1987, vol. 19, 10-12
- **SEO et al.** *Plant Mol Biol.,* 2009, vol. 69 (4), 463-72
- **SUNKAR et al.** *Plant J.,* 2003, 452-64
- **MEYERS.** *The Plant Journal,* 1999, vol. 3, 317-332
- **SUNKAR.** *The Plant Journal,* 2003, vol. 4, 452-464
- **RODRIGUES.** *Journal of Experimental Botany,* 2006, vol. 9, 1909-1918

- **KIRCH.** *The Plant Journal,* 2001, vol. 5, 555-567
- **HUANG.** *Plant Mol Biol.,* 2008, vol. 4 (5), 451-463
- **KWAK.** *Journal of Experimental Botany,* 2005, vol. 56, 3007-3016
- **LEE.** *Journal of Plant Physiol.,* 2007, vol. 12, 1626-1638
- **PENG.** *Planta,* 2007, vol. 3, 729-740
- **KOTCHONI.** *Plant Cell Environ.,* 2006, vol. 6, 1033-1048
- **SAKAMOTO.** *The Plant Journal,* 2008, vol. 3, 411-422
- **ALIA.** *Plant Mol Biol.,* 1999, vol. 2, 279-288
- **JUNG.** *Plant Physiology,* 2008, vol. 146, 623-635
- **JAIN.** *Plant Cell Rep.,* 2008, vol. 4, 767-778
- **TANG.** *Transgenic Res.,* 2008, vol. 4, 705-715
- **NARANJO.** *Plant Cell Environ.,* 2006, vol. 10, 1890-1900
- **PIAO.** *The Plant Journal,* 2001, vol. 4, 305-314
- **YANG.** *Plant Mol Biol.,* 2008, vol. 66, 329-343
- **MOON.** *PNAS,* 2003, vol. 100, 358-363
- **GUO.** *Journal of Experimental Botany,* 2009
- **KWAK.** *Plant Cell Physiol.,* 2007, vol. 2, 221-231
- **LILDBALLE.** *J Mol Biol.,* 2008, vol. 1, 9-12

- **SANJAYA.** *Plant Cell Environ.,* 2008, vol. 8, 1074-1085
- **KASUGA.** *Nature Biotechnology,* 1999, vol. 17, 287-291
- **JIA.** *Journal of Experimental Botany,* 2007, vol. 59, 739-751
- **LI.** *Plant Biotechnol. Journal,* 2008, vol. 2, 146-159
- **LIAO.** *J Integr Plant Biol.,* 2008, vol. 2, 221-230
- **WU.** *Plant Cell Rep.,* 2009, vol. 1, 21-30
- **WANG.** *Plant Mol Biol.,* 2008, vol. 6, 589-602
- **ORABY.** *Crop Science,* 2005, vol. 45, 2218-2227
- **MIFLIN.** *Journal of Experimental Botany,* 2002, vol. 53, 979-987
- **WINICOV.** *Annals of Botany,* 1998, vol. 82, 703-710
- **FLOWERS.** *Journal of Experimental Botany,* 2004, vol. 55, 307-319
- **HU.** *PNAS,* 2006, vol. 103, 12987-12992
- **FUKAO.** *PNAS,* 2008, vol. 105, 16814-16819
- **KOZIK.** *J. Amer. Soc. Hort. Sci.,* 2008, vol. 2, 225-227
- **HUANG.** *Plant Cell,* 2009, vol. 21 (2), 655-667
- **DOI.** *Genes & Development,* 2004, vol. 18, 926-936
- **ZHANG.** *PNAS,* 2008, vol. 105 (52), 21028-21033
- **WILHELM.** *Theor Appl Genet.,* 2009, vol. 118 (2), 285-294
- **WILHWLM.** *Theor Appl Genet.,* 2009, vol. 118 (2), 285-294
- **LIU.** *J Plant Physiol.,* vol. 166 (3), 278-289
- **LOCASCIO.** *New Phytol.,* 2009, vol. 182 (3), 630-643
- **YAO.** *J Integr Plant Biol.,* 2009, vol. 51 (1), 35-44
- **KIM.** *Planta,* 2008, vol. 228 (2), 355-365
- **NEMOTO.** *Plant J.,* 2003, vol. 36 (1), 82-93
- **KOJIMA.** *Plant Cell Physiol.,* 2002, vol. 43 (10), 1096-1105
- **SHEORAN.** *Journal of Experimental Botany,* 2007, vol. 58 (13), 3525-3535
- **WANG.** *Genetics,* 2006, vol. 173, 965-974
- **LU.** *Acta Biochim Biophys Sin (Shanghai),* 2006, vol. 38 (7), 492-499
- **VERNON.** *EMBO J.,* 1992, vol. 11 (6), 2077-2085
- **DOEBLEY et al.** *Cell,* 29 December 2006, vol. 127 (7), 1309-1321
- **MEINKE et al.** *Plant Physiol.,* February 2003, vol. 131 (2), 409-418
- **KOMARI et al.** *Curr. Opin. Plant Biol.,* 1998, vol. 1, 161
- **FINER et al.** *Curr. Top. Microbiol. Immunol.,* 1999, vol. 240, 59
- **BATES.** *Methods Mol. Biol.,* 1999, vol. 111, 359
- **PORTA ; LOMONOSSOFF.** *Mol. Biotechnol.,* 1996, vol. 5, 209
- **SAMBROOK et al.** Molecular Cloning: A Laboratory Manual. Cold Spring Harbor Laboratory Press, 1989
- Methods of Enzymology. Academic Press, 1987, vol. 153
- **GREEN et al.** Plant Tissue and Cell Culture. Academic Press, 1987
- **CROSSWAY et al.** *Biotechniques,* 1986, vol. 4, 320-334
- **RIGGS et al.** *Proc. Natl. Acad. Sci. USA,* 1986, vol. 83, 5602-5606
- **D'HALLUIN et al.** *Plant Cell,* 1992, vol. 4, 1495-1505
- **PASZKOWSKI et al.** *EMBO J.,* 1984, vol. 3, 2717-2722
- **KLEIN et al.** *Nature,* 1987, vol. 327, 70
- **SANFORD et al.** *Techniques,* 1991, vol. 3, 3-16
- **KLEIN et al.** *Bio/Techniques,* 1992, vol. 10, 286
- Direct DNA Transfer into Intact Plant Cells via Microprojectile Bombardment. **TOMES et al.** Plant Cell, Tissue, and Organ Culture: Fundamental Methods. Springer-Verlag, 1995
- **MCCABE et al.** *Biotechnology,* 1988, vol. 6, 923-926
- **DONG et al.** *Plant Cell Rep.,* 2006, vol. 25, 457-465
- **FRALEY et al.** *Trends Biochem. Sci.,* 1981, vol. 6, 77
- **GREGORIADIS, G.** *Trends in Biotechnology,* 1985, vol. 3, 235-241
- **FREEMAN et al.** *Plant Cell Physiol.,* 1984, vol. 29, 1353
- **OARD.** *Biotech. Adv.,* 1991, vol. 9, 1-11
- **WEISSINGER et al.** *sAnn. Rev. Genet.,* 1988, vol. 22, 421-477
- **SANFORD et al.** *Particulate Science and Technology,* 1987, vol. 5, 27-37
- **CHRISTOU et al.** *Plant Physiol.,* 1988, vol. 87, 671-674
- **MCCABE et al.** *Bio/Technology,* 1988, vol. 6, 923-926
- **FINER ; MCMULLEN.** *In vitro Cell Dev. Biol.,* 1991, vol. 27P, 175-182
- **SINGH et al.** *Theor. Appl. Genet.,* 1998, vol. 96, 319-324
- **DATTA et al.** *Biotechnology,* 1990, vol. 8, 736-740
- **KLEIN et al.** *Proc. Natl. Acad. Sci. USA,* 1988, vol. 85, 4305-4309
- **KLEIN et al.** *Biotechnology,* 1988, vol. 6, 559-563

- **KLEIN et al.** *Plant Physiol.,* 1988, vol. 91, 440-444
- **FROMM et al.** *Biotechnology,* 1990, vol. 8, 833-839
- **HOOYKAAS-VAN SLOGTEREN et al.** *Nature (London),* 1984, vol. 311, 763-764
- **BYTEBIER et al.** *Proc. Natl. Acad. Sci. USA,* 1987, vol. 84, 5345-5349
- **DE W et al.** The Experimental Manipulation of Ovule Tissues. Longman, 1985, 197-209
- **KAEPPLER et al.** *Plant Cell Reports,* 1990, vol. 9, 415-418
- **KAEPPLER et al.** *Theor. Appl. Genet.,* 1992, vol. 84, 560-566
- **LI et al.** *Plant Cell Reports,* 1993, vol. 12, 250-255
- **CHRISTOU ; FORD.** *Annals of Botany,* 1995, vol. 75, 407-413
- **OSJODA et al.** *Nature Biotechnology,* 1996, vol. 14, 745-750
- **TORIYARNA et al.** *Bio/Technology,* 1988, vol. 6, 1072-1074
- **ZHANG et al.** *Plant Cell Rep.,* 1988, vol. 7, 379-384
- **ZHANG et al.** *Theor. Appl. Genet.,* 1988, vol. 76, 835-840
- **SHIMAMOTO et al.** *Nature,* 1989, vol. 338, 274-276
- **DATTA et al.** *Bio/Technology,* 1990, vol. 8, 736-740
- **CHRISTOU et al.** *Bio/Technology,* 1991, vol. 9, 957-962
- **PENG et al.** *International Rice Research Institute,* 1991, 563-574
- **CAO et al.** *Plant Cell Rep.,* 1992, vol. 11, 585-591
- **LI et al.** *Plant Cell Rep.,* 1993, vol. 12, 250-255
- **RATHORE et al.** *Plant Mol. Biol.,* 1993, vol. 21, 871-884
- **FROMM et al.** *Bio/Technology,* 1990, vol. 8, 833-839
- **WALTERS et al.** *Plant Mol. Biol.,* 1992, vol. 18, 189-200
- **KOZIEL et al.** *Biotechnology,* 1993, vol. 11, 194-200
- **VASIL, I. K.** *Plant Mol. Biol.,* 1994, vol. 25, 925-937
- **WEEKS et al.** *Plant Physiol.,* 1993, vol. 102, 1077-1084
- **SOMERS et al.** *Bio/Technology,* 1992, vol. 10, 1589-1594
- **HIEI et al.** *The Plant Journal,* 1994, vol. 6, 271-282
- **SHIMAMOTO, K.** *Current Opinion in Biotechnology,* 1994, vol. 5, 158-162
- **VASIL et al.** *Bio/Technology,* 1992, vol. 10, 667-674
- **VAIN et al.** *Biotechnology Advances,* 1995, vol. 13 (4), 653-671
- **VASIL et al.** *Nature Biotechnology,* 1996, vol. 14, 702
- **WEISSBACH ; WEISSBACH.** Methods for Plant Molecular Biology. Academic Press, 1988, 421-463
- **GRIERSON ; COREY.** Plant Molecular Biology. Blackie, 1988
- **HORSCH et al.** *Science,* 1985, vol. 227, 1229
- **DE FRAMOND.** *Biotechnology,* 1983, vol. 1, 262
- **HOEKEMA et al.** *Nature,* 1983, vol. 303, 179
- **TOWNSEND et al.** *NAR,* 1984, vol. 12, 8711
- **BECHTOLD et al.** *C.R. Acad. Sci. Paris,* 1993, vol. 316, 1194
- **BENT, A.F.** *Plant Physiol.,* 2000, vol. 124, 1540-1547
- Cell Culture and Somatic Cell Genetics of Plants. **VASIL et al.** Laboratory Procedures and Their Applications. Academic Press, 1984, vol. I, II, and III
- **WEISSBACH.** Methods for Plant Molecular Biology. Academic Press, 1989
- Methods for Plant Molecular Biology. Academic Press, Inc, 1988
- The Maize Handbook. Springer, 1994
- Corn and Corn Improvement. American Society of Agronomy, 1988
- **PRIOLI et al.** *Bio/Technology,* 1989, vol. 7, 589-594
- *Methods in Enzymology,* vol. 118
- **KLEE et al.** *Annual Review of Plant Physiology,* 1987, vol. 38, 467
- **GORDON-KAMM et al.** *The Plant Cell,* 1990, vol. 2, 603-618
- Handbook of Plant Cell Culture. **EVANS et al.** Protoplasts Isolation and Culture. Macmillan Publishing Company, 1983, 124-176
- **BINDING.** Regeneration of Plants, Plant Protoplasts. CRC Press, 1985, 21-73
- **ALCOZ et al.** *Agronomy Journal,* 1993, vol. 85, 1198-1203
- **RAO ; DAO.** *J. Am. Soc. Agronomy,* 1992, vol. 84, 1028-1032
- **HOWARD ; LESSMAN.** *Agronomy Journal,* 1991, vol. 83, 208-211
- **TOLLENEAR et al.** *Agronomy Journal,* 1993, vol. 85, 251-255
- **STRAW et al.** Tennessee Farm and Home Science: Progress Report. Spring, 1993, vol. 166, 20-24
- **MILES, S. R.** *J. Am. Soc. Agronomy,* 1934, vol. 26, 129-137
- **DARA et al.** *J. Am. Soc. Agronomy,* 1992, vol. 84, 1006-1010
- **BINFORD et al.** *Agronomy Journal,* 1992, vol. 84, 53-59

- **CHEN et al.** *Canadian Journal of Plant Science,* 1992, vol. 72, 1049-1056
- **WALLACE et al.** *Journal of Plant Nutrition,* 1990, vol. 13, 1523-1537
- **ORITANI ; YOSHIDA.** *Japanese Journal of Crop Science,* 1984, vol. 53, 204-212
- **DIEPENBROCK ; PORKSEN.** *Industrial Crops and Products,* 1992, vol. 1, 165-173
- **GRUBINGER et al.** *Journal of the American Society for Horticultural Science,* 1993, vol. 118, 212-216
- **CERNE, M.** *Acta Horticulture,* 1990, vol. 277, 179-182
- **MAGISTAD et al.** *J. Am. Soc. Agronomy,* 1932, vol. 24, 610-622
- **ASOEGWU, S. N.** *Fertilizer Research,* 1988, vol. 15, 203-210
- **ASOEGWU, S. N.** *Fruits,* 1987, vol. 42, 505-509
- **RICHARDSON ; HARDGRAVE.** *Journal of the Science of Food and Agriculture,* 1992, vol. 59, 345-349
- **MUNSI, P. S.** *Acta Horticulturae,* 1992, vol. 306, 436-443
- **LETCHAMO, W.** *Acta Horticulturae,* 1992, vol. 306, 375-384
- **SISSON et al.** *Crop Science,* 1991, vol. 31, 1615-1620
- **PORTER ; SISSON.** *American Potato Journal,* 1991, vol. 68, 493-505
- **RAHN et al.** Conference "Proceedings, second congress of the European Society for Agronomy. Warwick Univ, 1992, 424-425
- **HEGDE ; SRINIVAS.** *Tropical Agriculture,* 1991, vol. 68, 331-334
- **LANGENEGGER ; SMITH.** *Fruits,* 1988, vol. 43, 639-643
- **HUMAN ; KOTZE.** *Communications in Soil Science and Plant Analysis,* 1990, vol. 21, 771-782
- **MAHALLE ; SETH.** *Indian Journal of Agricultural Sciences,* 1989, vol. 59, 395-397
- **ANJORIN ; OBIGBESAN.** Conference "International Cooperation for Effective Plantain and Banana Research. *Proceedings of the third meeting,* 1985, 115-117
- **YADAV, R. L.** *Fertiliser News,* 1986, vol. 31, 17-22
- **YADAV ; SHARMA.** *Indian Journal of Agricultural Sciences,* 1983, vol. 53, 38-43
- **DRAYCOTT et al.** *Conference "Symposium Nitrogen and Sugar Beet" International Institute for Sugar Beet Research-Brussels Belgium,* 1983, 293-303
- Nitrogen and Agronomic Practice. **GOH ; HAYNES.** Mineral Nitrogen in the Plant-Soil System. Academic Press, Inc, 1986, 379-468
- Soil Science Society of America. **ENGELSTAD, O. P.** Fertilizer Technology and Use. 1985, 633
- **YADAV ; SHARMNA.** *Indian Journal of Agricultural Sciences,* 1983, vol. 53, 3-43
- **MUTO.** *BMC Biotechnology,* 2009, vol. 9, 26
- **SOMANCHI.** *Plant J.,* 2005, vol. 42 (3), 341-352
- **BELIGNI.** *The Plant Cell,* 2004, vol. 16, 3357-3369
- **MAYFIELD.** *Plant J. Vol.,* 2004, vol. 37 (3), 449-458
- **HU.** *Plant J.,* 2008, vol. 54 (4), 621-639
- **BEER.** *Curr Opin Biotechnol.,* 2009, vol. 20 (3), 264-271
- **GODMAN.** *Genetics,* 2008, vol. 179, 59-68
- **CHARRON.** *Plant Physiol.,* 2005, vol. 139, 2017-2028
- **BONENTE.** *Photochem Photobiol.,* 2008, vol. 84 (6), 1359-1370
- **RUMPHO.** *PNAS,* 2008, vol. 105 (46), 17867-17871
- **NGUYEN.** *Eukaryot Cell,* 2008, vol. 7 (11), 1965-1979
- **UNIACKE.** *PNAS,* 2009, vol. 106 (5), 1439-1444
- **CHUNG.** *Appl Environ Microbiol.,* 2008, vol. 74 921, 6521-6527
- **BANG.** *Plant Cell Physiol.,* 2008, vol. 49 (9), 1350-1363
- **PUTHIYAVEETIL.** *PNAS,* 2008, vol. 105 (29), 10061-10066
- **HERNSCHEMEIER.** *Eukaryot Cell,* 2008, vol. 7 (3), 518-526
- **LI.** *J Plant Physiol.,* 2008, vol. 165 (17), 1783-1797
- **KOHINATA.** *Plant Cell Physiol.,* 2008, vol. 49 (2), 273-283
- **LOSI.** *PNAS,* 2008, vol. 105 (1), 7-8
- **JUNG.** *J Microbiol Biotechnol.,* 2007, vol. 17 (8), 1330-1337
- **CALDWELL.** *Microbiology,* 2007, vol. 153, 2231-3340
- **CHIDA.** *Plant Cell Physiol.,* 2007, vol. 48 (7), 948-957
- **ROBBENS.** *J Mol Evol.,* 2007, vol. 64 (5), 601-604
- **LUIS.** *Plant Cell Environ.,* 2006, vol. 29 (11), 2043-2054
- **WOLFE-SIMON.** *Plant Physiol.,* 2006, vol. 142, 1701-1709
- **FOERSTER.** *Proteomics,* 2006, vol. 6 (15), 4309-4320
- **JOHN.** *ISME J.,* 2007, vol. 1 (6), 517-531
- **CHENG.** *J Photochem Photobiol B.,* 2007, vol. 87 (2), 137-143
- **FISHER.** *J Biol Chem.,* 1997, vol. 272 (3), 1565-1570
- **SERRANO.** *Curr Biol.,* 2009, vol. 19 (5), 359-368

- **RAMOS.** *Appl Microbiol Biotechnol.,* 2008, vol. 79 (5), 819-828
- **YANG.** *J Basic Microbiol.,* 2007, vol. 47 (3), 266-274
- **TANAKA.** *FEMS Microbiol Lett.,* 2007, vol. 271 (1), 48-52
- **QUINGHUA.** *DNA Seq.,* 2005, vol. 16 (3), 202-206
- **TANAKA.** *FEMS Microbiol Lett.,* 2004, vol. 236 (1), 41-45
- **ZHANG.** *DNA Seq.,* 2002, vol. 13 (4), 195-202
- **ZHANG.** *DNA Seq.,* 2002, vol. 13 (3), 173-177
- **TANAKA.** *Curr Microbiol.,* 2001, vol. 42 (3), 173-177
- **RICHARD.** *Plant Mol Biol.,* 2000, vol. 42 (2), 303-316
- **NI.** *PNAS,* 1999, vol. 96, 3611-3615
- **VARDI.** *Curr Biol.,* 2008, vol. 18 (12), 895-899
- **SCHULZ-RAFFELT.** *Plant J.,* 2007, vol. 52 (2), 286-295
- **SIEZEN.** *Curr Opin Biotechnol.,* 2004, vol. 15 (2), 105-115
- **PFEILER.** *Trends Microbiol.,* 2007, vol. 15 (12), 546-553
- **ZHU.** *Appl. Michrobiol Biotechn.,* 2009, vol. 83 (4), 597-610
- **NES.** *Curr Opin Biotechnol.,* 2004, vol. 15 (2), 100-104
- **DE VOS.** *Curr Opin Biotechnol.,* 2004, vol. 15 (2), 86-93
- **LORCA.** *Biochim Biophys Acta,* 2004, vol. 1768 (6), 1342-1366
- **DE VUYST.** *J Mol Microbiol Biotechnol.,* 2007, vol. 13 (4), 194-199
- **KLAENHAMMER.** *Microbiol Rev.,* 2005, vol. 29 (3), 393-409
- **MAKAROVA.** *PNAS,* 2006, vol. 103, 15611-15616
- **RODIONOV.** *Nucleic Acids Research,* 2004, vol. 32 (11), 3340-3353
- **COGAN.** *J Dairy Science,* 2007, vol. 90 (9), 4005-4021
- **SCHROETER.** *FEMS Microbiol Lett.,* 2009, vol. 292 (1), 1-6
- **GOH.** *Front Biosci,* 2009, vol. 14, 1362-1386
- **LEPAGE.** *J Bacteriol.,* 2006, vol. 188 (19), 6858-6868
- **JURKOVIC.** *Lett Appl Microbiol.,* 2006, vol. 42 (6), 553-559
- **O'SULLIVAN.** *MBC Microbiology,* 2009, vol. 9, 50
- **TESTA.** *Biochim Biophys Acta,* 2006, vol. 1764 (1), 85-96
- **LI.** *Toxicon.,* 2009, vol. 53 (6), 595-601
- **ILMEN.** *Appl Environ Microbiol,* 1997, vol. 63 (3), 1298-1306
- **BAUTISTA.** *Appl Environ Microbiol.,* 2000, vol. 66 (10), 4579-4581
- **HOFFMANN.** *Mol Biol Cell,* 2001, vol. 12, 2846-2857
- **SHIMA.** *Biotechnol Appl Biochem.,* 2009, vol. 53, 155-164
- **HONG.** *Plant Physiol.,* 2002, vol. 129, 354-362
- **ZUBER.** *Genetics,* 2003, vol. 164, 487-499
- **GOVENDER.** *Appl Environ Microbiol.,* 2008, vol. 74 (19), 6041-6052
- **ESPINAZO-ROMEU.** *FEMS Yeast Res.,* 2008, vol. 8 (7), 1127-1136
- **WOLTJEN et al.** *Nature,* 2009, vol. 458, 766-770
- **ZHAO et al.** iPS cells produce viable mice through tetraploid complementation. *Nature,* 23 July 2009
- **AMIT et al.** *Semin. Reprod. Med.,* 2006, vol. 24 (5), 298-303
- **PANAGIOTIS A. TSONIS.** Stem Cells from Differentiated Cells. *Molecular Interventions,* 2004, vol. 4, 81-83
- **OHTA et al.** *Biol Reprod.,* 2008, vol. 79 (3), 486-92
- **FARUQI et al.** *Molecular and Cellular Biology,* 2000, vol. 20, 990-1000
- **SCHLEIFMAN et al.** *Methods Molecular Biology,* 2008, vol. 435, 175-90
- **DRAGHICI.** Data Analysis Tools for DNA Microarrays. Chapman & Hall, CRC Press, 2003
- **CONOVER.** Practical Nonparametric Statistics. Wiley, 1971
- **PIERCE.** An Introduction To Information Theory: Symbols, Signals, and Noise. Dover, 1980
- **DUDA et al.** Pattern Classification. John Wiley & Sons, Inc, 2001
- **HASTIE et al.** The Elements of Statistical Learning. Springer-Verlag, 2001
- **DRAGHICI.** Data Analysis Tools for DNA Microarrays. Chapman & Hall/CRC, 2003
- **MOUNT.** Bioinformatics: sequence and genome analysis. Cold Spring Harbor Laboratory Press, 2001
- **DUDA ; HART.** Pattern Classification and Scene Analysis. John Wiley & Sons, Inc, 1973, 211-256
- **DUDA et al.** Pattern Classification. John Wiley & Sons, Inc,
- **KAUFINAN ; ROUSSEEUW.** Finding Groups in Data: An Introduction to Cluster Analysis. Wiley, 1990
- **EVERITT.** Cluster analysis. Wiley, 1993
- **BACKER.** Computer-Assisted Reasoning in Cluster Analysis. Prentice Hall, 1995
- **JOLLIFFE.** Principal Component Analysis. Springer, 1986
- **KUBINYI.** 3D QSAR in drug design theory methods and applications. Pergamon Press, 1990, 589-638

- **DUDA.** Pattern Classification. John Wiley & Sons, Inc, 2001
- **HASTIE.** The Elements of Statistical Learning. Springer, 2001
- **VENABLES ; RIPLEY.** Modern Applied Statistics with s-plus. Springer, 1997
- **CRISTIANINI ; SHAWE-TAYLOR.** An Introduction to Support Vector Machines. Cambridge University Press, 2000
- A training algorithm for optimal margin classifiers. **BOSER et al.** Proceedings of the 5th Annual ACM Workshop on Computational Learning Theory. ACM Press, 1992, 142-152
- **VAPNIK.** Statistical Learning Theory. Wiley, 1998
- **DUDA.** Pattern Classification. John Wiley & Sons, Inc, 2001
- **FUREY et al.** *Bioinformatics,* 2000, vol. 16, 906-914
- **DUDA.** Pattern Classification. John Wiley & Sons, Inc, 2001, 395-396
- **DUDA.** Pattern Classification. John Wiley & Sons, Inc, 2001, 396-408, 411-412
- Technical Report 567, Statistics Department. **BREIMAN.** Random Forests - Random Features. University of California at Berkeley, September 1999
- **DUDA.** Pattern Classification. John Wiley & Sons, Inc, 2001, 408-409
- **HASTIE et al.** The Elements of Statistical Learning. Springer-Verlag, 2001, 283-295
- **TIBSHIRANI et al.** *Proceedings of the National Academy of Science USA,* 2002, vol. 99, 6567-6572